(19) Europäisches Patentamt
European Patent Office
Office européen des brevets

(11) **EP 4 026 905 B1**

(12) **EUROPEAN PATENT SPECIFICATION**

(45) Date of publication and mention of the grant of the patent:
**17.04.2024 Bulletin 2024/16**

(21) Application number: **21214927.2**

(22) Date of filing: **18.10.2017**

(51) International Patent Classification (IPC):
*C12N 15/10* (2006.01)    *C12Q 1/6869* (2018.01)

(52) Cooperative Patent Classification (CPC):
(C-Sets available)
**C12N 15/1065; C12Q 1/6869**    (Cont.)

(54) **METHODS FOR BARCODING NUCLEIC ACID MOLECULES FROM INDIVIDUAL CELLS OR CELL POPULATIONS**

VERFAHREN ZUM BARCODING VON NUKLEINSÄUREMOLEKÜLEN AUS EINZELNEN ZELLEN ODER ZELLPOPULATIONEN

PROCÉDÉS DE CODAGE À BARRES DE MOLÉCULES D'ACIDE NUCLÉIQUE À PARTIR DE CELLULES INDIVIDUELLES OU DE POPULATIONS DE CELLULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.10.2016 US 201662410326 P**
**26.04.2017 US 201762490546 P**

(43) Date of publication of application:
**13.07.2022 Bulletin 2022/28**

(62) Document number(s) of the earlier application(s) in accordance with Art. 76 EPC:
**17862611.5 / 3 529 357**

(73) Proprietor: **10X Genomics, Inc.**
**Pleasanton, CA 94588 (US)**

(72) Inventors:
• **MIKKELSEN, Tarjei, Sigurd**
**Pleasanton, 94588-3260 (US)**
• **BELGRADER, Phillip**
**Pleasanton, 94588-3260 (US)**
• **ZHENG, Xinying**
**Pleasanton, 94588-3260 (US)**

(74) Representative: **J A Kemp LLP**
**80 Turnmill Street**
**London EC1M 5QU (GB)**

(56) References cited:
WO-A1-2014/144495    WO-A1-2015/103339
US-A1- 2015 376 609    US-A1- 2016 244 825

• N. Rohland ET AL: "Partial uracil-DNA-glycosylase treatment for screening of ancient DNA", Proceedings of the Royal Society B: Biological Sciences, vol. 279, no. 1748, 8 December 2014 (2014-12-08), pages 4724-20130624, XP055215058, ISSN: 0962-8452, DOI: 10.1098/rstb.2013.0624

(52) Cooperative Patent Classification (CPC): (Cont.)

C-Sets
**C12Q 1/6869, C12Q 2521/107, C12Q 2563/149,
C12Q 2563/159, C12Q 2563/179**

**Description**

**BACKGROUND**

Significant advances in analyzing and characterizing biological and biochemical materials and systems have led to unprecedented advances in understanding the mechanisms of life, health, disease and treatment. Among these advances, technologies that target and characterize the genomic make up of biological systems have yielded some of the most groundbreaking results, including advances in the use and exploitation of genetic amplification technologies, and nucleic acid sequencing technologies.

Nucleic acid sequencing can be used to obtain information in a wide variety of biomedical contexts, including diagnostics, prognostics, biotechnology, and forensic biology. Sequencing may involve methods including Maxam-Gilbert sequencing and chain-termination methods, or de novo sequencing methods including shotgun sequencing and bridge PCR, or next-generation methods including polony sequencing, 454 pyrosequencing, Illumina sequencing, SOLiD sequencing, Ion Torrent semiconductor sequencing, HeliScope single molecule sequencing, SMRT® sequencing, and others. Nucleic acid sequencing technologies, including next-generation DNA sequencing, have been useful for genomic and proteomic analysis of cell populations. WO 2015/103339, WO 2014/144495, US 2016/244825 and US 2015/376609 all relate to single cell sequencing.

**SUMMARY**

Recognized herein is the need for methods, compositions and systems for analyzing genomic and proteomic information from individual cells or a small population of cells. Such cells include, but are not limited to, cancer cells, fetal cells, and immune cells involved in immune responses. The present invention is defined by the scope of the appended claims. Disclosed herein but not part of the invention are further methods, compositions and systems for analyzing individual cells or a small population of cells, including the analysis and attribution of nucleic acids from and to these individual cells or cell populations.

In particular, the invention provides a method for nucleic acid sequencing, comprising:

(a) providing a plurality of partitions, wherein a partition of said plurality of partitions comprises (i) a ribonucleic acid (RNA) molecule from an immune cell, wherein the RNA molecule comprises a nucleic acid sequence of a T cell receptor or a B cell receptor, and (ii) a bead comprising a nucleic acid barcode molecule coupled thereto, wherein said nucleic acid barcode molecule comprises a barcode sequence and a primer sequence;

(b) using said RNA molecule and said nucleic acid barcode molecule to generate a barcoded nucleic acid molecule comprising, from a 5' end to a 3' end, said barcode sequence and a sequence corresponding to said nucleic acid sequence of said RNA molecule, wherein the generating comprises (i) hybridizing said nucleic acid barcode molecule to said RNA molecule, (ii) extending said nucleic acid barcode molecule using a reverse transcriptase having terminal transferase activity to generate a nucleic acid product comprising a reverse complement of a sequence of said RNA molecule and a sequence at its 3' end that is complementary to a portion of a template switching oligonucleotide, (iii) hybridizing said nucleic acid product to said template switching oligonucleotide, and (iv) extending said nucleic acid product using said template switching oligonucleotide as a template;

(c) using said barcoded nucleic acid molecules, or a derivative thereof, and a pair of primers to generate a subset of nucleic acids having a TCR or BCR target nucleic acid sequence to provide an enriched subset of barcoded nucleic acid molecules or derivatives thereof; and

(d) sequencing said enriched subset of barcoded nucleic acid molecules, or derivatives thereof, and an unenriched subset of barcoded nucleic acid molecules, or derivatives thereof.

In some embodiments, the partition comprises the cell. In some embodiments, the method further comprises releasing the RNA molecule from the cell prior to (b).

In some embodiments, the bead comprises a plurality of nucleic acid molecules coupled thereto, wherein the plurality of nucleic acid molecules comprises the nucleic acid barcode molecule.

In some embodiments, each of the plurality of nucleic acid molecules comprises the barcode sequence. In some embodiments, each of the plurality of nucleic acid molecules comprises an additional barcode sequence that varies across the plurality of nucleic acid molecules.

In some embodiments, the method further comprises, prior to (d), subjecting the barcoded nucleic acid molecule or derivative thereof to nucleic acid amplification. In some embodiments, the nucleic acid amplification is performed subsequent to releasing the barcoded nucleic acid molecule or derivative thereof from the partition. In some embodiments, the nucleic acid amplification is polymerase chain reaction. In some embodiments, the RNA molecule is a messenger ribonucleic acid (mRNA) molecule.

In some embodiments, in (a) the partition comprises (i) an additional nucleic acid molecule comprising an additional nucleic acid sequence, and (ii) an additional nucleic acid barcode molecule comprising an additional barcode sequence, and wherein in (b) the additional nucleic acid molecule and the additional nucleic acid barcode molecule are used to generate an additional barcoded nucleic acid molecule comprising, from a 5'end to a 3'end, the additional barcode sequence and an additional sequence corresponding to the additional nucleic acid sequence. In some embodiments, the additional nucleic acid barcode molecule is coupled to the bead. In some embodiments, the additional nucleic acid barcode molecule is coupled to an additional bead.

In some embodiments, (b) is performed in the partition.

In some embodiments, the method further comprises releasing the barcoded nucleic acid molecule or a derivative thereof from the partition.

In some embodiments, the barcoded nucleic acid molecule further comprises, towards a 3' end, a functional sequence for permitting the barcoded nucleic acid molecule or a derivative thereof to couple to a flow cell of a sequencer.

In some embodiments, the sequence is a reverse complement of the nucleic acid sequence.

In some embodiments, the target nucleic acid sequence comprises a T cell receptor variable region sequence or a B cell receptor variable region sequence. In some embodiments, the at least one of the pair of primers hybridizes to a constant region of a T cell receptor nucleic acid sequence or a constant region of a B cell receptor nucleic acid sequence. The subset of nucleic acids or derivatives thereof are sequenced in (d).

In some embodiments, the method further comprises releasing the nucleic acid barcode molecule from the bead. In some embodiments, the nucleic acid barcode molecule is released from the bead before the barcoded nucleic acid molecule is generated. In some embodiments, the nucleic acid barcode molecule is released from the bead while the barcoded nucleic acid molecule is generated. In some embodiments, the nucleic acid barcode molecule is released from the bead after the barcoded nucleic acid molecule is generated. In some embodiments, the bead is a gel bead.

In some embodiments, the barcode sequence is a combinatorial assembly of a plurality of barcode segments. In some embodiments, the plurality of barcode segments comprises at least three segments.

In an aspect, the present disclosure provides a method for generating a labeled polynucleotide. The method comprises (a) subjecting a reaction mixture to a first amplification reaction under conditions sufficient to generate a first amplification product, wherein the reaction mixture comprises a template polynucleotide and (i) a primer having a sequence towards a 3' end that hybridizes to the template polynucleotide, and (ii) a template switching oligonucleotide that comprises a first predefined sequence towards a 5' end; and (b) subjecting the first amplification product to a second amplification reaction in the presence of a barcoded oligonucleotide under conditions sufficient to generate a second amplification product, wherein the barcoded oligonucleotide comprises a sequence of at least a segment of the template switching oligonucleotide and at least a second predefined sequence, wherein (i) the second amplification reaction uses the first amplification product as a template and the barcoded oligonucleotide as a primer, or (ii) the second amplification reaction uses the barcoded oligonucleotide as a template and at least a portion of the first amplification product as a primer, to generate the second amplification product, wherein the first amplification reaction and the second amplification reaction are performed within a same reaction volume. In some embodiments, the second amplification reaction uses the first amplification product as a template and the barcoded oligonucleotide as a primer. In some embodiments, the second amplification reaction uses the barcoded oligonucleotide as a template and at least a portion of the first amplification product as a primer.

In an aspect, the present disclosure provides a method for generating a labeled polynucleotide comprising (a) providing a reaction mixture in a reaction volume, wherein the reaction mixture comprises (i) a template polynucleotide, (ii) a primer comprising a sequence towards a 3' end of the primer that hybridizes to the template polynucleotide, and (iii) a template switching oligonucleotide; (b) in the reaction volume, subjecting the reaction mixture to a first reaction under conditions sufficient to generate a first nucleic acid product comprising the primer, a reverse complement of a sequence of the template polynucleotide, and a sequence complementary to at least a portion of the template switch oligonucleotide; and (c) subjecting the first nucleic acid product to a second reaction in the reaction volume, which second reaction comprises using (i) the first nucleic acid product as a template and a barcoded oligonucleotide as a primer, which barcoded oligonucleotide comprises a sequence of at least a segment of the template switching oligonucleotide, or (ii) the barcoded oligonucleotide as a template and at least a portion of the first nucleic acid as a primer, to generate a second nucleic acid product.

The template polynucleotide is obtained from a single cell, wherein the single cell is an immune cell. In some embodiments, the immune cell is a T-cell. In some embodiments, the immune cell is a B-cell. In some embodiments, the method further comprises lysing the single cell in the same reaction volume to obtain the template polynucleotide prior to generating the first amplification product in the first amplification reaction.

In some embodiments, the template polynucleotide comprises a T-cell receptor gene or gene product. In some embodiments, the template polynucleotide comprises a B-cell receptor gene or gene product. In some embodiments, the template polynucleotide is among a plurality of template polynucleotides.

In some embodiments, a concentration of the template switching oligonucleotide in the same reaction volume is at least two times that of a concentration of the barcoded oligonucleotide in the same reaction volume. In some embodiments, a concentration of the template switching oligonucleotide in the same reaction volume is at least five times that of a concentration of the barcoded oligonucleotide in the same reaction volume. In some embodiments, a concentration of the template switching oligonucleotide in the same reaction volume is at least ten times that of a concentration of the barcoded oligonucleotide in the same reaction volume. In some embodiments, a concentration of the template switching oligonucleotide in the same reaction volume is at least twenty times that of a concentration of the barcoded oligonucleotide in the same reaction volume. In some embodiments, a concentration of the template switching oligonucleotide in the same reaction volume is at least fifty times that of a concentration of the barcoded oligonucleotide in the same reaction volume. In some embodiments, a concentration of the template switching oligonucleotide in the same reaction volume is at least one hundred times that of a concentration of the barcoded oligonucleotide in the same reaction volume. In some embodiments, a concentration of the template switching oligonucleotide in the same reaction volume is at least two hundred times that of a concentration of the barcoded oligonucleotide in the same reaction volume.

In some embodiments, the primer comprises a sequence towards a 5' end that does not specifically hybridize to the template polynucleotide.

In some embodiments, the first amplification reaction is facilitated using an enzyme comprising polymerase activity. In some embodiments, the enzyme is a DNA-dependent polymerase. In some embodiments, the enzyme is a reverse transcriptase.

In some embodiments, the second amplification reaction is facilitated using an enzyme comprising polymerase activity. In some embodiments, the enzyme is a DNA-dependent polymerase.

In some embodiments, the first amplification reaction comprises polymerase chain reaction. In some embodiments, the first amplification reaction comprises reverse transcription. In some embodiments, the second amplification reaction comprises polymerase chain reaction.

In some embodiments, the first amplification reaction and the second amplification reaction are performed sequentially in the absence of an intervening purification step.

In some embodiments, the template switching oligonucleotide is not available for primer extension during the second amplification reaction.

In some embodiments, the method further comprises degrading the template switching oligonucleotide prior to the second amplification reaction. In some embodiments, the template switching oligonucleotide comprises ribonucleic acids (RNA). In some embodiments, the template switching oligonucleotide comprises at least 10% ribonucleic acids (RNA).

In some embodiments, the method further comprises degrading the template switching oligonucleotide during the second amplification reaction. In some embodiments, the template switching oligonucleotide comprises ribonucleic acids (RNA). In some embodiments, the template switching oligonucleotide comprises at least 10% ribonucleic acids (RNA).

In some embodiments, a first reaction rate of the second amplification reaction using the barcoded oligonucleotide is greater than a second reaction rate of the second amplification using the template switching oligonucleotide.

In some embodiments, the first amplification product and the barcoded oligonucleotide has a higher melting temperature as compared to a melting temperature of the first amplification product and the template switching oligonucleotide. In some embodiments, a primer annealing temperature of the second amplification reaction is at least 0.5 °C greater than a primer annealing temperature of the first amplification reaction.

In some embodiments, the template switching oligonucleotide comprises modified nucleotides. In some embodiments, the template switching oligonucleotide comprises at least 10% modified nucleotides. In some embodiments, the template switching oligonucleotide comprises modified nucleotides selected from unlocked nucleic acids (UNAs), locked nucleic acids (LNAs), and 5-hydroxybutynl-2'-deoxyuridine.

In some embodiments, the barcoded oligonucleotide comprises modified nucleotides. In some embodiments, the barcoded oligonucleotide comprises at least 10% modified nucleotides. In some embodiments, the barcoded oligonucleotide comprises modified nucleotides selected from locked nucleic acids (LNAs), unlocked nucleic acids (UNAs), and 5-hydroxybutynl-2'-deoxyuridine.

In some embodiments, the same reaction volume comprises an emulsion, a droplet, or a microwell.

In some embodiments, the first defined sequence comprises at least one of an adaptor sequence, a barcode sequence, a unique molecular identifier sequence, a primer binding site, and a sequencing primer binding site. In some embodiments, the second defined sequence comprises at least one of an adaptor sequence, a barcode sequence, a unique molecular identifier sequence, a primer binding site, and a sequencing primer binding site.

In some embodiments, the primer is among a plurality of primers. In some embodiments, the sequence towards the 3' end of the primer comprises a random sequence. In some embodiments, the sequence towards the 3' end of the primer comprises a gene specific sequence. In some embodiments, the sequence towards the 3' end of the primer comprises a polyA sequence.

In some embodiments, the template switching oligonucleotide is among a plurality of template switching oligonucleotides. In some embodiments, the barcoded oligonucleotide is among a plurality of barcoded oligonucleotides.

In some embodiments, the method further comprises subjecting the second amplification product to sequencing.

In some embodiments, the barcoded oligonucleotide is releasably coupled to a microcapsule. In some embodiments, the method further comprises releasing the barcoded oligonucleotide from the microcapsule. In some embodiments, the barcoded oligonucleotide is released from the microcapsule upon application of a stimulus. In some embodiments, the stimulus is at least one of a biological stimulus, a chemical stimulus, a thermal stimulus, an electrical stimulus, a magnetic stimulus, a photo stimulus, or any combination thereof. In some embodiments, the microcapsule is a degradable microcapsule and releasing the barcoded oligonucleotide comprises degrading the microcapsule. In some embodiments, the microcapsule comprises a polymer gel. In some embodiments, the polymer gel is a polyacrylamide. In some embodiments, the microcapsule comprises a bead. In some embodiments, the bead is a gel bead. In some embodiments, the microcapsule comprises a chemical cross-linker. In some embodiments, the chemical cross-linker is a disulfide bond.

In an aspect, the present disclosure provides a method comprising (a) providing a reaction volume comprising (i) a cell or cell derivative, and (ii) a bead comprising a barcoded oligonucleotide releasably coupled thereto, wherein the barcoded oligonucleotide is a template switching oligonucleotide; and (b) releasing the barcoded oligonucleotide from the bead to provide the barcoded oligonucleotide in the reaction volume at a concentration of at least about 0.20 $\mu$M; and (c) subjecting the reaction volume to an amplification reaction to generate an amplification product, wherein during the amplification reaction, the reaction volume comprises a template polynucleotide from the cell or cell derivative, the barcoded oligonucleotide and a primer having a sequence towards a 3' end that hybridizes to the template polynucleotide, and wherein the amplification product has sequence complementarity with the template polynucleotide and the barcoded oligonucleotide.

In an aspect, the present disclosure provides a method comprising (a) providing a reaction volume comprising a cell and a microcapsule comprising a barcoded oligonucleotide releasably coupled thereto, wherein the barcoded oligonucleotide is a template switching oligonucleotide; and (b) subjecting the reaction volume to dissociation conditions sufficient to release the barcoded oligonucleotide from the microcapsule, thereby providing the barcoded oligonucleotide in the reaction volume at a concentration of at least about 0.20 uM; and (c) subjecting the reaction volume to an amplification reaction to generate an amplification product, wherein during the amplification reaction, the reaction volume comprises a template polynucleotide from the cell, the barcoded oligonucleotide and a primer having a sequence towards a 3' end that hybridizes to the template polynucleotide, and wherein the amplification product has sequence complementarity with the template polynucleotide and the barcoded oligonucleotide.

In some embodiments, the method further comprises subjecting the amplification product to sequencing. In some embodiments, the barcoded oligonucleotide does not hybridize to the template polynucleotide. In some embodiments, the template polynucleotide is an mRNA molecule. In some embodiments, the method further comprises subjecting the reaction volume to a second amplification reaction to generate an additional amplification product using the amplification product as a template.

In some embodiments, the method further comprises subjecting the additional amplification product to sequencing.

In some embodiments, the cell is a mammalian cell. In some embodiments, the cell is an immune cell. In some embodiments, the immune cell is a B-cell. In some embodiments, the immune cell is a T-cell. In some embodiments, the cell is cancer cell. In some embodiments, the cancer cell is obtained from a tissue sample. In some embodiments, the cancer cell is obtained from a biological fluid. In some embodiments, the biological fluid comprises blood. In some embodiments, the biological fluid comprises lymph fluid.

The template polynucleotide comprises a T cell receptor gene sequence or a B cell receptor gene sequence, wherein the template polynucleotide is a T cell receptor mRNA molecule or a B cell receptor mRNA molecule.

In some embodiments, the reaction volume further comprises an enzyme. In some embodiments, the enzyme is a DNA polymerase. In some embodiments, the enzyme is a reverse transcriptase.

In some embodiments, the reaction volume further comprises at least one reagent for nucleic acid amplification. In some embodiments, the at least one reagent comprises dNTPs. In some embodiments, the at least one reagent comprises oligonucleotide primers.

In some embodiments, the microcapsule comprises a polymer gel. In some embodiments, the polymer gel is a polyacrylamide. In some embodiments, the microcapsule comprises a bead. In some embodiments, the bead is a gel bead. In some embodiments, the microcapsule comprises a chemical cross-linker. In some embodiments, the chemical cross-linker is a disulfide bond. In some embodiments, the dissociation condition is at least one of a biological stimulus, a chemical stimulus, a thermal stimulus, an electrical stimulus, a magnetic stimulus, a photo stimulus, or any combination thereof.

In some embodiments, the barcoded oligonucleotide comprises at least one of an adaptor sequence, a barcode sequence, unique molecular identifier sequence, a primer binding site, and a sequencing primer binding site.

In some embodiments, the same reaction volume comprises an emulsion, a droplet, or a microwell.

In some embodiments, the method further comprises performing a third reaction, wherein the third reaction specifically amplifies variable region cDNAs, wherein the variable region cDNA are derived from a T cell receptor cDNA or a B cell receptor cDNA. In some embodiments, the third reaction comprises use of a primer that specifically binds in the constant region of the T cell receptor cDNA or B cell receptor cDNA, and extends through the variable region of the T cell receptor cDNA or B cell receptor cDNA. In some embodiments, the third reaction results in an enrichment product that comprises at (a) least one of a T cell receptor variable region sequence or a B cell receptor variable region sequence, and (b) at least one of an adaptor sequence, a barcode sequence, a unique molecular identifier sequence, a primer binding site, and a sequencing primer binding site. In some embodiments, greater than about 25% of reads in a subsequent short-read sequencing reaction map to a T cell receptor or a B cell receptor.

In an aspect, the present disclosure provides a non-transitory computer-readable medium comprising machine-executable code that, upon execution by one of more computer processors, implements a method for nucleic acid sequencing, comprising (a) providing a plurality of droplets, wherein a droplet of the plurality of droplets comprises (i) a ribonucleic acid (RNA) molecule comprising a nucleic acid sequence, and (ii) a bead comprising a nucleic acid barcode molecule coupled thereto, wherein the nucleic acid barcode molecule comprises a barcode sequence; (b) using the RNA molecule and the nucleic acid barcode molecule to generate a barcoded nucleic acid molecule comprising, from a 5'end to a 3'end, a sequence corresponding to the nucleic acid sequence of the RNA molecule and a complement of the barcode sequence; and (c) sequencing the barcoded nucleic acid molecule or a derivative thereof.

In an aspect, the present disclosure provides a non-transitory computer-readable medium comprising machine-executable code that, upon execution by one of more computer processors, implements a method for generating a labeled polynucleotide, the method comprising (a) subjecting a reaction mixture to a first reaction under conditions sufficient to generate a first nucleic acid product, wherein the reaction mixture comprises (i) a template polynucleotide, (ii) a primer having a sequence towards a 3' end that hybridizes to the template polynucleotide, and (iii) a template switching oligonucleotide, wherein the first nucleic acid product comprises the primer, a reverse complement of a sequence of the template polynucleotide, and a sequence complementary to at least a portion of the template switch oligonucleotide; and (b) subjecting the first nucleic acid product to a second reaction in the presence of a barcoded oligonucleotide under conditions sufficient to generate a second nucleic acid product, wherein the barcoded oligonucleotide comprises a sequence of at least a segment of the template switching oligonucleotide, wherein (i) the second reaction uses the first nucleic acid as a template and the barcoded oligonucleotide as a primer, or (ii) the second reaction uses the barcoded oligonucleotide as a template and at least a portion of the first nucleic acid as a primer, to generate the second nucleic acid product, wherein the first reaction and the second reaction are performed within a same reaction volume.

In an aspect, the present disclosure provides a non-transitory computer-readable medium comprising machine-executable code that, upon execution by one of more computer processors, implements a method for generating a labeled polynucleotide. The method comprises (a) subjecting a reaction mixture to a first amplification reaction under conditions sufficient to generate a first amplification product, wherein the reaction mixture comprises a template polynucleotide and (i) a primer having a sequence towards a 3' end that hybridizes to the template polynucleotide, and (ii) a template switching oligonucleotide that comprises a first predefined sequence towards a 5' end; and (b) subjecting the first amplification product to a second amplification reaction in the presence of a barcoded oligonucleotide under conditions sufficient to generate a second amplification product, wherein the barcoded oligonucleotide comprises a sequence of at least a segment of the template switching oligonucleotide and at least a second predefined sequence, wherein (i) the second amplification reaction uses the first amplification product as a template and the barcoded oligonucleotide as a primer, or (ii) the second amplification reaction uses the barcoded oligonucleotide as a template and at least a portion of the first amplification product as a primer, to generate the second amplification product, wherein the first amplification reaction and the second amplification reaction are performed within a same reaction volume.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

## BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "Figure" and "FIG." herein), of which:

Figure 1 schematically illustrates a microfluidic channel structure for partitioning individual or small groups of cells.

Figure 2 schematically illustrates a microfluidic channel structure for co-partitioning cells and microcapsules (e.g., beads) comprising additional reagents.

Figure 3 schematically illustrates an example process for amplification and barcoding of cell's nucleic acids.

Figure 4 provides a schematic illustration of use of barcoding of cell's nucleic acids in attributing sequence data to individual cells or groups of cells for use in their characterization.

Figure 5 provides a schematic illustration of cells associated with labeled cell-binding ligands.

Figure 6 provides a schematic illustration of an example workflow for performing RNA analysis using the methods described herein.

Figure 7 provides a schematic illustration of an example barcoded oligonucleotide structure for use in analysis of ribonucleic (RNA) using the methods described herein.

Figure 8 provides an image of individual cells co-partitioned along with individual barcode bearing beads.

Figures 9A-9E provide schematic illustration of example barcoded oligonucleotide structures for use in analysis of RNA and example operations for performing RNA analysis.

Figure 10 provides schematic illustration of example barcoded oligonucleotide structure for use in example analysis of RNA and use of a sequence for in vitro transcription.

Figure 11 provides schematic illustration of an example barcoded oligonucleotide structure for use in analysis of RNA and example operations for performing RNA analysis.

Figures 12A-12B provide schematic illustrations of example barcoded oligonucleotide structure for use in analysis of RNA.

Figures 13A-13C provide illustrations of example yields from template switch reverse transcription and PCR in partitions,

Figures 14A-14B provide illustrations of example yields from reverse transcription and complementary deoxyribonucleic acid (cDNA) amplification in partitions with various cell numbers.

Figure 15 provides an illustration of example yields from cDNA synthesis and realtime quantitative PCR at various input cell concentrations and also the effect of varying primer concentration on yield at a fixed cell input concentration.

Figure 16 provides an illustration of example yields from in vitro transcription.

Figure 17 shows an example computer control system that is programmed or otherwise configured to implement methods provided herein.

Figure 18 provides a schematic illustration of an example barcoded oligonucleotide structure.

Figures 19A and 19B show example operations for performing RNA analysis.

Figure 20 shows a schematic for enriching VDJ sequences from immune molecules such as TCRs and BCRs.

Figures 21A-21C show enrichment of target sequences (A) after cDNA amplification, (B) after enrichment, and (C) after sequencing library preparation.

Figure 22 shows cDNA yields from 12,000; 6,000; or 3,000 cells using gel-beads in an emulsion-reverse transcription reaction (GEM-RT).

Figure 23 shows sequencing results from cDNA that has been enriched using constant region primers compared to unenriched cDNA.

Figure 24 shows cDNA yields using differing concentrations of a template switch oligo (TSO) were tested.

Figures 25A and 25B show cDNA yields from TSO immobilized to gel beads (GB-TSO) using either 6,000 primary T cells (A) or 2,200 Jurkat cells (B).

Figures 26A and 26B show cDNA yields from an enrichment using an in solution RT reaction (A) or a GEM RT reaction (B) using nested enrichment primers,

Figures 27A-27C show enrichment of TCR cDNA using p7 primers only (A), variable region primers with TCR beta chain constant region primers (B), and variable region primers with TCR alpha chain constant region primers (C),

Figures 28A-28D show a comparison of enriched product generated with either 8 $\mu$M or 200 $\mu$M TSO gel beads using P7 primers with TCR alpha chain constant region primers (A), variable region primers with TCR beta chain constant region primers (B), variable region primers with TCR alpha chain constant region primers (C), and variable region primers with TCR beta chain constant region primers (D).

Figures 29A and 29B show variations of a schematic for generating labeled polynucleotides.

## DETAILED DESCRIPTION

While various embodiments of the invention have been shown and described herein, it will be obvious to those skilled in the art that such embodiments are provided by way of example only. Numerous variations, changes, and substitutions may occur to those skilled in the art without departing from the invention. It should be understood that various alternatives to the embodiments of the invention described herein may be employed.

Where values are described as ranges, it will be understood that such disclosure includes the disclosure of all possible sub-ranges within such ranges, as well as specific numerical values that fall within such ranges irrespective of whether a specific numerical value or specific sub-range is expressly stated.

The term "barcode," as used herein, generally refers to a label, or identifier, that can be part of an analyte to convey information about the analyte. A barcode can be a tag attached to an analyte (e.g., nucleic acid molecule) or a combination of the tag in addition to an endogenous characteristic of the analyte (e.g., size of the analyte or end sequence(s)). The barcode may be unique. Barcodes can have a variety of different formats, for example, barcodes can include: polynucleotide barcodes; random nucleic acid and/or amino acid sequences; and synthetic nucleic acid and/or amino acid sequences. A barcode can be attached to an analyte in a reversible or irreversible manner. The barcode can be added to, for example, a fragment of a deoxyribonucleic acid (DNA) or ribonucleic acid (RNA) sample before, during, and/or after sequencing of the sample. Barcodes can allow for identification and/or quantification of individual sequencing-reads in real time. In some examples, the barcode is generated in a combinatorial manner. Barcodes that may be used with methods, devices and systems of the present disclosure, including methods for forming such barcodes, are described in, for example, U.S. Patent Pub. No. 2014/0378350.

The term "subject," as used herein, generally refers to an animal, such as a mammalian species (e.g., human) or avian (e.g., bird) species, or other organism, such as a plant. The subject can be a vertebrate, a mammal, a mouse, a primate, a simian or a human. Animals may include, but are not limited to, farm animals, sport animals, and pets. A subject can be a healthy individual, an individual that has or is suspected of having a disease or a pre-disposition to the disease, or an individual that is in need of therapy or suspected of needing therapy. A subject can be a patient.

The term "genome," as used herein, generally refers to an entirety of a subject's hereditary information. A genome can be encoded either in DNA or in RNA. A genome can comprise coding regions that code for proteins as well as non-coding regions. A genome can include the sequence of all chromosomes together in an organism. For example, the human genome has a total of 46 chromosomes. The sequence of all of these together may constitute a human genome.

The terms "adaptor(s)", "adapter(s)" and "tag(s)" may be used synonymously. An adaptor or tag can be coupled to a polynucleotide sequence to be "tagged" by any approach including ligation, hybridization, or other approaches.

The term "sequencing," as used herein, generally refers to methods and technologies for determining the sequence of nucleotide bases in one or more polynucleotides. The polynucleotides can be, for example, deoxyribonucleic acid (DNA) or ribonucleic acid (RNA), including variants or derivatives thereof (e.g., single stranded DNA). Sequencing can be performed by various systems currently available, such as, with limitation, a sequencing system by Illumina, Pacific Biosciences, Oxford Nanopore, or Life Technologies (Ion Torrent). Such devices may provide a plurality of raw genetic data corresponding to the genetic information of a subject (e.g., human), as generated by the device from a sample provided by the subject. In some situations, systems and methods provided herein may be used with proteomic information.

The term "variant," as used herein, generally refers to a genetic variant, such as a nucleic acid molecule comprising a polymorphism. A variant can be a structural variant or copy number variant, which can be genomic variants that are larger than single nucleotide variants or short indels. A variant can be an alteration or polymorphism in a nucleic acid sample or genome of a subject. Single nucleotide polymorphisms (SNPs) are a form of polymorphisms. Polymorphisms can include single nucleotide variations (SNVs), insertions, deletions, repeats, small insertions, small deletions, small repeats, structural variant junctions, variable length tandem repeats, and/or flanking sequences. Copy number variants (CNVs), transversions and other rearrangements are also forms of genetic variation. A genomic alternation may be a base change, insertion, deletion, repeat, copy number variation, or transversion.

The term "bead," as used herein, generally refers to a particle. The bead may be a solid or semi-solid particle. The bead may be a gel. The bead may be formed of a polymeric material. The bead may be magnetic or non-magnetic.

The term "sample," as used herein, generally refers to a biological sample of a subject. The sample may be a tissue sample, such as a biopsy, core biopsy, needle aspirate, or fine needle aspirate. The sample may be a fluid sample, such as a blood sample, urine sample, or saliva sample. The sample may be a skin sample. The sample may be a cheek swap. The sample may be a plasma or serum sample. The sample may be a cell-free or cell free sample. A cell-free sample may include extracellular polynucleotides. Extracellular polynucleotides may be isolated from a bodily sample that may be selected from a group consisting of blood, plasma, serum, urine, saliva, mucosal excretions, sputum, stool and tears.

The term "primer," as used herein generally refers to a strand of RNA or DNA that serves as a starting point for nucleic acid (e.g., DNA) synthesis. A primer may be used in a primer extension reaction, which may be a nucleic acid amplification reaction, such as, for example, polymerase chain reaction (PCR) or reverse transcription PCR (RT-PCR). The primer may have a sequence that is capable of coupling to a nucleic acid molecule. Such sequence may be complementary to the nucleic acid molecule, such as a poly-T sequence or a predetermined sequence, or a sequence that is otherwise capable of coupling (e.g., hybridizing) to the nucleic acid molecule, such as a universal primer.

Nucleic acid sequencing technologies have yielded substantial results in sequencing biological materials, including providing substantial sequence information on individual organisms, and relatively pure biological samples. However, these systems have traditionally not been effective at being able to identify and characterize cells at the single cell level.

Many nucleic acid sequencing technologies derive the nucleic acids that they sequence from collections of cells obtained from tissue or other samples, such as biological fluids (e.g., blood, plasma, etc). The cells can be processed (e.g., all together) to extract the genetic material that represents an average of the population of cells, which can then be processed into sequencing ready DNA libraries that are configured for a given sequencing technology. Although often discussed in terms of DNA or nucleic acids, the nucleic acids derived from the cells may include DNA, or RNA, including, e.g., mRNA, total RNA, or the like, that may be processed to produce complementary DNA (cDNA) for sequencing. Following processing, absent a cell specific marker, attribution of genetic material as being contributed by a subset of cells or an individual cell may not be possible in such an ensemble approach.

In addition to the inability to attribute characteristics to particular subsets of cells or individual cells, such ensemble sample preparation methods can be, from the outset, predisposed to primarily identifying and characterizing the majority constituents in the sample of cells, and may not be designed to pick out the minority constituents, e.g., genetic material contributed by one cell, a few cells, or a small percentage of total cells in the sample. Likewise, where analyzing expression levels, e.g., of mRNA, an ensemble approach can be predisposed to presenting potentially inaccurate data from cell populations that are non-homogeneous in terms of expression levels. In some cases, where expression is high in a small minority of the cells in an analyzed population, and absent in the majority of the cells of the population, an ensemble method may indicate low level expression for the entire population.

These inaccuracies can be further magnified through processing operations used in generating the sequencing libraries from these samples. In particular, many next generation sequencing technologies (e.g., massively parallel sequencing) may rely upon the geometric amplification of nucleic acid fragments, such as via polymerase chain reaction, in order to produce sufficient DNA for the sequencing library. However, such amplification can be biased toward amplification of majority constituents in a sample, and may not preserve the starting ratios of such minority and majority components. While some of these difficulties may be addressed by utilizing different sequencing systems, such as single molecule systems that do not require amplification, the single molecule systems, as well as the ensemble sequencing methods of other next generation sequencing systems, can also have large input DNA requirements. Some single molecule sequencing systems, for example, can have sample input DNA requirements of from 500 nanograms (ng) to upwards of 10 micrograms ($\mu$g), which may not be obtainable from individual cells or even small subpopulations of cells. Likewise, other NGS systems can be optimized for starting amounts of sample DNA in the sample of from approximately 50 ng to about 1 $\mu$g.

Disclosed herein are methods and systems for characterizing nucleic acids from small populations of cells, and in some cases, for characterizing nucleic acids from individual cells. The methods described herein may compartmentalize the analysis of individual cells or small populations of cells, including e.g., nucleic acids from individual cells or small groups of cells, and then allow that analysis to be attributed back to the individual cell or small group of cells from which the nucleic acids were derived. This can be accomplished regardless of whether the cell population represents a 50/50 mix of cell types, a 90/10 mix of cell types, or virtually any ratio of cell types, as well as a complete heterogeneous mix of different cell types, or any mixture between these. Differing cell types may include cells from different tissue types of an individual or the same tissue type from different individuals, or biological organisms such as microorganisms from differing genera, species, strains, variants, or any combination of any or all of the foregoing. For example, differing cell types may include normal and tumor tissue from an individual, various cell types obtained from a human subject such as a variety of immune cells (e.g., B cells, T cells, and the like), multiple different bacterial species, strains and/or variants from environmental, forensic, microbiome or other samples, or any of a variety of other mixtures of cell types.

Methods and systems described herein may provide for the compartmentalization, depositing or partitioning of the nucleic acid contents of individual cells from a sample material containing cells, into discrete compartments or partitions (referred to interchangeably herein as partitions), where each partition maintains separation of its own contents from the contents of other partitions. In some examples, a partition is a droplet or well. Unique identifiers, e.g., barcodes, may be previously, subsequently or concurrently delivered to the partitions that hold the compartmentalized or partitioned cells or cellular derivatives, in order to allow for the later attribution of the characteristics of the individual cells to the particular compartment. Barcodes may be delivered, for example in an oligonucleotide to a partition via any suitable mechanism, such as using beads (e.g., gel beads). In some examples, cellular derivatives, such as cells or constituents of such cells in matrix (e.g., gel or polymeric matrix), are compartmentalized or partitioned in partitions (e.g., droplets or wells).

In some embodiments, barcoded oligonucleotides are delivered to a partition via a microcapsule. In some cases, barcoded oligonucleotides are initially associated with the microcapsule and then released from the microcapsule upon application of a stimulus which allows the oligonucleotides to dissociate or to be released from the microcapsule.

A microcapsule, in some embodiments, comprises a bead. In some embodiments, a bead may be porous, non-porous, solid, semi-solid, semi-fluidic, or fluidic. In some embodiments, a bead may be dissolvable, disruptable, or degradable. In some cases, a bead may not be degradable. In some embodiments, the bead may be a gel bead. A gel bead may be a hydrogel bead. A gel bead may be formed from molecular precursors, such as a polymeric or monomeric species. A semi-solid bead may be a liposomal bead. Solid beads may comprise metals including iron oxide, gold, and silver. In some cases, the beads are silica beads. In some cases, the beads are rigid. In some cases, the beads may be flexible and/or compressible.

In some embodiments, the bead may contain molecular precursors (e.g., monomers or polymers), which may form a polymer network via polymerization of the precursors. In some cases, a precursor may be an already polymerized species capable of undergoing further polymerization via, for example, a chemical cross-linkage. In some cases, a precursor comprises one or more of an acrylamide or a methacrylamide monomer, oligomer, or polymer. In some cases, the bead may comprise prepolymers, which are oligomers capable of further polymerization. For example, polyurethane beads may be prepared using prepolymers. In some cases, the bead may contain individual polymers that may be further polymerized together. In some cases, beads may be generated via polymerization of different precursors, such that they comprise mixed polymers, co-polymers, and/or block co-polymers.

A bead may comprise natural and/or synthetic materials. For example, a polymer can be a natural polymer or a synthetic polymer. In some cases, a bead comprises both natural and synthetic polymers. Examples of natural polymers include proteins and sugars such as deoxyribonucleic acid, rubber, cellulose, starch (e.g., amylose, amylopectin), proteins, enzymes, polysaccharides, silks, polyhydroxyalkanoates, chitosan, dextran, collagen, carrageenan, ispaghula, acacia, agar, gelatin, shellac, sterculia gum, xanthan gum, Corn sugar gum, guar gum, gum karaya, agarose, alginic acid, alginate, or natural polymers thereof. Examples of synthetic polymers include acrylics, nylons, silicones, spandex, viscose rayon, polycarboxylic acids, polyvinyl acetate, polyacrylamide, polyacrylate, polyethylene glycol, polyurethanes, polylactic acid, silica, polystyrene, polyacrylonitrile, polybutadiene, polycarbonate, polyethylene, polyethylene terephthalate, poly(chlorotrifluoroethylene), poly(ethylene oxide), poly(ethylene terephthalate), polyethylene, polyisobutylene, poly(methyl methacrylate), poly(oxymethylene), polyformaldehyde, polypropylene, polystyrene, poly(tetrafluoroethylene), poly(vinyl acetate), poly(vinyl alcohol), poly(vinyl chloride), poly(vinylidene dichloride), poly(vinylidene difluoride), poly(vinyl fluoride) and combinations (e.g., co-polymers) thereof. Beads may also be formed from materials other than polymers, including lipids, micelles, ceramics, glassceramics, material composites, metals, other inorganic materials, and others.

In some cases, a chemical cross-linker may be a precursor used to cross-link monomers during polymerization of the monomers and/or may be used to attach oligonucleotides (e.g., barcoded oligonucleotides) to the bead. In some cases, polymers may be further polymerized with a cross-linker species or other type of monomer to generate a further polymeric network. Non-limiting examples of chemical cross-linkers (also referred to as a "crosslinker" or a "crosslinker agent" herein) include cystamine, gluteraldehyde, dimethyl suberimidate, N-Hydroxysuccinimide crosslinker BS3, formaldehyde, carbodiimide (EDC), SMCC, Sulfo-SMCC, vinylsilane, N,N'diallyltartardiamide (DATD), N,N'-Bis(acryloyl)cystamine (BAC), or homologs thereof. In some cases, the crosslinker used in the present disclosure contains cystamine.

Crosslinking may be permanent or reversible, depending upon the particular crosslinker used. Reversible crosslinking may allow for the polymer to linearize or dissociate under appropriate conditions. In some cases, reversible cross-linking may also allow for reversible attachment of a material bound to the surface of a bead. In some cases, a cross-linker may form disulfide linkages. In some cases, the chemical cross-linker forming disulfide linkages may be cystamine or a modified cystamine.

In some embodiments, disulfide linkages can be formed between molecular precursor units (e.g., monomers, oligomers, or linear polymers) or precursors incorporated into a bead and oligonucleotides. Cystamine (including modified cystamines), for example, is an organic agent comprising a disulfide bond that may be used as a crosslinker agent between individual monomeric or polymeric precursors of a bead. Polyacrylamide may be polymerized in the presence of cystamine or a species comprising cystamine (e.g., a modified cystamine) to generate polyacrylamide gel beads comprising disulfide linkages (e.g., chemically degradable beads comprising chemically-reducible cross-linkers). The disulfide linkages may permit the bead to be degraded (or dissolved) upon exposure of the bead to a reducing agent.

In some embodiments, chitosan, a linear polysaccharide polymer, may be crosslinked with glutaraldehyde via hydrophilic chains to form a bead. Crosslinking of chitosan polymers may be achieved by chemical reactions that are initiated by heat, pressure, change in pH, and/or radiation.

In some embodiments, the bead may comprise covalent or ionic bonds between polymeric precursors (e.g., monomers, oligomers, linear polymers), oligonucleotides, primers, and other entities. In some cases, the covalent bonds comprise carbon-carbon bonds or thioether bonds.

In some cases, a bead may comprise an acrydite moiety, which in certain aspects may be used to attach one or more oligonucleotides (e.g., barcode sequence, barcoded oligonucleotide, primer, or other oligonucleotide) to the bead. In some cases, an acrydite moiety can refer to an acrydite analogue generated from the reaction of acrydite with one or more species, such as, the reaction of acrydite with other monomers and cross-linkers during a polymerization reaction. Acrydite moieties may be modified to form chemical bonds with a species to be attached, such as an oligonucleotide (e.g., barcode sequence, barcoded oligonucleotide, primer, or other oligonucleotide). Acrydite moieties may be modified with thiol groups capable of forming a disulfide bond or may be modified with groups already comprising a disulfide bond. The thiol or disulfide (via disulfide exchange) may be used as an anchor point for a species to be attached or another part of the acrydite moiety may be used for attachment. In some cases, attachment is reversible, such that when the disulfide bond is broken (e.g., in the presence of a reducing agent), the attached species is released from the bead. In other cases, an acrydite moiety comprises a reactive hydroxyl group that may be used for attachment.

Functionalization of beads for attachment of oligonucleotides may be achieved through a wide range of different approaches, including activation of chemical groups within a polymer, incorporation of active or activatable functional groups in the polymer structure, or attachment at the pre-polymer or monomer stage in bead production.

For example, precursors (e.g., monomers, cross-linkers) that are polymerized to form a bead may comprise acrydite moieties, such that when a bead is generated, the bead also comprises acrydite moieties. The acrydite moieties can be attached to an oligonucleotide, such as a primer (e.g., a primer for amplifying target nucleic acids, barcoded oligonucleotide, etc) that is desired to be incorporated into the bead. In some cases, the primer comprises a P5 sequence for attachment to a sequencing flow cell for Illumina sequencing. In some cases, the primer comprises a P7 sequence for attachment to a sequencing flow cell for Illumina sequencing. In some cases, the primer comprises a barcode sequence. In some cases, the primer further comprises a unique molecular identifier (UMI). In some cases, the primer comprises an R1 primer sequence for Illumina sequencing. In some cases, the primer comprises an R2 primer sequence for Illumina sequencing.

In some cases, precursors comprising a functional group that is reactive or capable of being activated such that it becomes reactive can be polymerized with other precursors to generate gel beads comprising the activated or activatable functional group. The functional group may then be used to attach additional species (e.g., disulfide linkers, primers, other oligonucleotides, etc.) to the gel beads. For example, some precursors comprising a carboxylic acid (COOH) group can co-polymerize with other precursors to form a gel bead that also comprises a COOH functional group. In some cases, acrylic acid (a species comprising free COOH groups), acrylamide, and bis(acryloyl)cystamine can be co-polymerized together to generate a gel bead comprising free COOH groups. The COOH groups of the gel bead can be activated (e.g., via 1-Ethyl-3-(3-dimethylaminopropyl)carbodiimide (EDC) and N-Hydroxysuccinimide (NHS) or 4-(4,6-Dimethoxy-1,3,5-triazin-2-yl)-4-methylmorpholinium chloride (DMTMM)) such that they are reactive (e.g., reactive to amine functional groups where EDC/NHS or DMTMM are used for activation). The activated COOH groups can then react with an appropriate species (e.g., a species comprising an amine functional group where the carboxylic acid groups are activated to be reactive with an amine functional group) comprising a moiety to be linked to the bead.

Beads comprising disulfide linkages in their polymeric network may be functionalized with additional species via reduction of some of the disulfide linkages to free thiols. The disulfide linkages may be reduced via, for example, the action of a reducing agent (e.g., DTT, TCEP, etc.) to generate free thiol groups, without dissolution of the bead. Free thiols of the beads can then react with free thiols of a species or a species comprising another disulfide bond (e.g., via thiol-disulfide exchange) such that the species can be linked to the beads (e.g., via a generated disulfide bond). In some cases, free thiols of the beads may react with any other suitable group. For example, free thiols of the beads may react with species comprising an acrydite moiety. The free thiol groups of the beads can react with the acrydite via Michael addition chemistry, such that the species comprising the acrydite is linked to the bead. In some cases, uncontrolled reactions can be prevented by inclusion of a thiol capping agent such as N-ethylmalieamide or iodoacetate.

Activation of disulfide linkages within a bead can be controlled such that only a small number of disulfide linkages are activated. Control may be exerted, for example, by controlling the concentration of a reducing agent used to generate free thiol groups and/or concentration of reagents used to form disulfide bonds in bead polymerization. In some cases, a low concentration (e.g., molecules of reducing agent: gel bead ratios of less than about 10,000; 100,000; 1,000,000; 10,000,000; 100,000,000; 1,000,000,000; 10,000,000,000; or 100,000,000,000) of reducing agent may be used for reduction. Controlling the number of disulfide linkages that are reduced to free thiols may be useful in ensuring bead structural integrity during functionalization. In some cases, optically-active agents, such as fluorescent dyes may be may be coupled to beads via free thiol groups of the beads and used to quantify the number of free thiols present in a bead and/or track a bead.

In some cases, addition of moieties to a gel bead after gel bead formation may be advantageous. For example, addition of an oligonucleotide (e.g., barcoded oligonucleotide) after gel bead formation may avoid loss of the species during chain transfer termination that can occur during polymerization. Moreover, smaller precursors (e.g., monomers or cross linkers that do not comprise side chain groups and linked moieties) may be used for polymerization and can be minimally hindered from growing chain ends due to viscous effects. In some cases, functionalization after gel bead synthesis can minimize exposure of species (e.g., oligonucleotides) to be loaded with potentially damaging agents (e.g., free radicals) and/or chemical environments. In some cases, the generated gel may possess an upper critical solution temperature (UCST) that can permit temperature driven swelling and collapse of a bead. Such functionality may aid in oligonucleotide (e.g., a primer) infiltration into the bead during subsequent functionalization of the bead with the oligonucleotide. Post-production functionalization may also be useful in controlling loading ratios of species in beads, such that, for example, the variability in loading ratio is minimized. Species loading may also be performed in a batch process such that a plurality of beads can be functionalized with the species in a single batch.

In some cases, an acrydite moiety linked to precursor, another species linked to a precursor, or a precursor itself comprises a labile bond, such as chemically, thermally, or photosensitive bonds e.g., disulfide bonds, UV sensitive bonds, or the like. Once acrydite moieties or other moieties comprising a labile bond are incorporated into a bead, the bead may also comprise the labile bond. The labile bond may be, for example, useful in reversibly linking (e.g., covalently linking) species (e.g., barcodes, primers, etc.) to a bead. In some cases, a thermally labile bond may include a nucleic acid hybridization based attachment, e.g., where an oligonucleotide is hybridized to a complementary sequence that is attached to the bead, such that thermal melting of the hybrid releases the oligonucleotide, e.g., a barcode containing sequence, from the bead or microcapsule.

The addition of multiple types of labile bonds to a gel bead may result in the generation of a bead capable of responding to varied stimuli. Each type of labile bond may be sensitive to an associated stimulus (e.g., chemical stimulus, light, temperature, etc.) such that release of species attached to a bead via each labile bond may be controlled by the application of the appropriate stimulus. Such functionality may be useful in controlled release of species from a gel bead. In some cases, another species comprising a labile bond may be linked to a gel bead after gel bead formation via, for example, an activated functional group of the gel bead as described above. As will be appreciated, barcodes that are releasably, cleavably or reversibly attached to the beads described herein include barcodes that are released or releasable through cleavage of a linkage between the barcode molecule and the bead, or that are released through degradation of the underlying bead itself, allowing the barcodes to be accessed or accessible by other reagents, or both.

The barcodes that are releasable as described herein may sometimes be referred to as being activatable, in that they are available for reaction once released. Thus, for example, an activatable barcode may be activated by releasing the barcode from a bead (or other suitable type of partition described herein). Other activatable configurations are also envisioned in the context of the described methods and systems.

In addition to thermally cleavable bonds, disulfide bonds and UV sensitive bonds, other non-limiting examples of labile bonds that may be coupled to a precursor or bead include an ester linkage (e.g., cleavable with an acid, a base, or hydroxylamine), a vicinal diol linkage (e.g., cleavable via sodium periodate), a Diels-Alder linkage (e.g., cleavable via heat), a sulfone linkage (e.g., cleavable via a base), a silyl ether linkage (e.g., cleavable via an acid), a glycosidic linkage (e.g., cleavable via an amylase), a peptide linkage (e.g., cleavable via a protease), or a phosphodiester linkage (e.g., cleavable via a nuclease (e.g., DNAase)).

Species that do not participate in polymerization may also be encapsulated in beads during bead generation (e.g., during polymerization of precursors). Such species may be entered into polymerization reaction mixtures such that generated beads comprise the species upon bead formation. In some cases, such species may be added to the gel beads after formation. Such species may include, for example, oligonucleotides, reagents for a nucleic acid amplification reaction (e.g., primers, polymerases, dNTPs, co-factors (e.g., ionic co-factors)) including those described herein, reagents for enzymatic reactions (e.g., enzymes, co-factors, substrates), or reagents for a nucleic acid modification reactions such as polymerization, ligation, or digestion. Trapping of such species may be controlled by the polymer network density generated during polymerization of precursors, control of ionic charge within the gel bead (e.g., via ionic species linked to polymerized species), or by the release of other species. Encapsulated species may be released from a bead upon bead degradation and/or by application of a stimulus capable of releasing the species from the bead.

Beads may be of uniform size or heterogeneous size. In some cases, the diameter of a bead may be about $1\mu m$, $5\mu m$, $10\mu m$, $20\mu m$, $30\mu m$, $40\mu m$, $50\mu m$, $60\mu m$, $70\mu m$, $80\mu m$, $90\mu m$, $100\mu m$, $250\mu m$, $500\mu m$, or 1mm. In some cases, a bead may have a diameter of at least about $1\mu m$, $5\mu m$, $10\mu m$, $20\mu m$, $30\mu m$, $40\mu m$, $50\mu m$, $60\mu m$, $70\mu m$, $80\mu m$, $90\mu m$, $100\mu m$, $250\mu m$, $500\mu m$, 1mm, or more. In some cases, a bead may have a diameter of less than about $1\mu m$, $5\mu m$, $10\mu m$, $20\mu m$, $30\mu m$, $40\mu m$, $50\mu m$, $60\mu m$, $70\mu m$, $80\mu m$, $90\mu m$, $100\mu m$, $250\mu m$, $500\mu m$, or 1mm. In some cases, a bead may have a diameter in the range of about $40\text{-}75\mu m$, $30\text{-}75\mu m$, $20\text{-}75\mu m$, $40\text{-}85\mu m$, $40\text{-}95\mu m$, $20\text{-}100\mu m$, $10\text{-}100\mu m$, $1\text{-}100\mu m$, $20\text{-}250\mu m$, or $20\text{-}500\mu m$.

In certain aspects, beads are provided as a population or plurality of beads having a relatively monodisperse size distribution. Where it may be desirable to provide relatively consistent amounts of reagents within partitions, maintaining relatively consistent bead characteristics, such as size, can contribute to the overall consistency. In particular, the beads described herein may have size distributions that have a coefficient of variation in their crosssectional dimensions of less than 50%, less than 40%, less than 30%, less than 20%, and in some cases less than 15%, less than 10%, or even less than 5%.

Beads may be of any suitable shape. Examples of bead shapes include, but are not limited to, spherical, non-spherical, oval, oblong, amorphous, circular, cylindrical, and variations thereof.

In addition to, or as an alternative to the cleavable linkages between the beads and the associated molecules, e.g., barcode containing oligonucleotides, described above, the beads may be degradable, disruptable, or dissolvable spontaneously or upon exposure to one or more stimuli (e.g., temperature changes, pH changes, exposure to particular chemical species or phase, exposure to light, reducing agent, etc.). In some cases, a bead may be dissolvable, such that material components of the beads are solubilized when exposed to a particular chemical species or an environmental change, such as a change temperature or a change in pH. In some cases, a gel bead is degraded or dissolved at elevated temperature and/or in basic conditions. In some cases, a bead may be thermally degradable such that when the bead is exposed to an appropriate change in temperature (e.g., heat), the bead degrades. Degradation or dissolution of a bead bound to a species (e.g., an oligonucleotide, e.g., barcoded oligonucleotide) may result in release of the species from the bead.

A degradable bead may comprise one or more species with a labile bond such that, when the bead/species is exposed to the appropriate stimuli, the bond is broken and the bead degrades. The labile bond may be a chemical bond (e.g., covalent bond, ionic bond) or may be another type of physical interaction (e.g., van der Waals interactions, dipole-dipole interactions, etc.). In some cases, a crosslinker used to generate a bead may comprise a labile bond. Upon exposure to the appropriate conditions, the labile bond can be broken and the bead degraded. For example, upon exposure of a polyacrylamide gel bead comprising cystamine crosslinkers to a reducing agent, the disulfide bonds of the cystamine can be broken and the bead degraded.

A degradable bead may be useful in more quickly releasing an attached species (e.g., an oligonucleotide, a barcode sequence, a primer, etc) from the bead when the appropriate stimulus is applied to the bead as compared to a bead that does not degrade. For example, for a species bound to an inner surface of a porous bead or in the case of an encapsulated species, the species may have greater mobility and accessibility to other species in solution upon degradation of the bead. In some cases, a species may also be attached to a degradable bead via a degradable linker (e.g., disulfide linker). The degradable linker may respond to the same stimuli as the degradable bead or the two degradable species may respond to different stimuli. For example, a barcode sequence may be attached, via a disulfide bond, to a polyacrylamide bead comprising cystamine. Upon exposure of the barcoded-bead to a reducing agent, the bead degrades and the barcode sequence is released upon breakage of both the disulfide linkage between the barcode sequence and the bead and the disulfide linkages of the cystamine in the bead.

A degradable bead may be introduced into a partition, such as a droplet of an emulsion or a well, such that the bead degrades within the partition and any associated species (e.g., oligonucleotides) are released within the droplet when the appropriate stimulus is applied. The free species (e.g., oligonucleotides) may interact with other reagents contained in the partition. For example, a polyacrylamide bead comprising cystamine and linked, via a disulfide bond, to a barcode sequence, may be combined with a reducing agent within a droplet of a water-in-oil emulsion. Within the droplet, the reducing agent breaks the various disulfide bonds resulting in bead degradation and release of the barcode sequence into the aqueous, inner environment of the droplet. In another example, heating of a droplet comprising a bead-bound barcode sequence in basic solution may also result in bead degradation and release of the attached barcode sequence into the aqueous, inner environment of the droplet.

As will be appreciated from the above disclosure, while referred to as degradation of a bead, in many instances as noted above, that degradation may refer to the disassociation of a bound or entrained species from a bead, both with and without structurally degrading the physical bead itself. For example, entrained species may be released from beads through osmotic pressure differences due to, for example, changing chemical environments. By way of example, alteration of bead pore sizes due to osmotic pressure differences can generally occur without structural degradation of the bead itself. In some cases, an increase in pore size due to osmotic swelling of a bead can permit the release of entrained species within the bead. In other cases, osmotic shrinking of a bead may cause a bead to better retain an entrained species due to pore size contraction.

Where degradable beads are provided, it may be desirable to avoid exposing such beads to the stimulus or stimuli that cause such degradation prior to the desired time, in order to avoid premature bead degradation and issues that arise from such degradation, including for example poor flow characteristics and aggregation. By way of example, where beads comprise reducible cross-linking groups, such as disulfide groups, it will be desirable to avoid contacting such beads with reducing agents, e.g., DTT or other disulfide cleaving reagents. In such cases, treatment to the beads described herein will, in some cases be provided free of reducing agents, such as DTT. Because reducing agents are often provided in commercial enzyme preparations, it may be desirable to provide reducing agent free (or DTT free) enzyme preparations in treating the beads described herein. Examples of such enzymes include, e.g., polymerase enzyme preparations, reverse transcriptase enzyme preparations, ligase enzyme preparations, as well as many other enzyme preparations that may be used to treat the beads described herein. The terms "reducing agent free" or "DTT free" preparations can refer to a preparation having less than 1/10th, less than 1/50th, and even less than 1/100th of the lower ranges for such materials used in degrading the beads. For example, for DTT, the reducing agent free preparation will typically have less than 0.01 mM, 0.005 mM, 0.001 mM DTT, 0.0005 mM DTT, or even less than 0.0001 mM DTT. In many cases, the amount of DTT will be undetectable.

In some cases, a stimulus may be used to trigger degradation of the bead, which may result in the release of contents from the bead. Generally, a stimulus may cause degradation of the bead structure, such as degradation of the covalent bonds or other types of physical interaction. These stimuli may be useful in inducing a bead to degrade and/or to release its contents. Examples of stimuli that may be used include chemical stimuli, thermal stimuli, optical stimuli (e.g., light) and any combination thereof, as described more fully below.

Numerous chemical triggers may be used to trigger the degradation of beads. Examples of these chemical changes may include, but are not limited to pH-mediated changes to the integrity of a component within the bead, degradation of a component of a bead via cleavage of cross-linked bonds, and depolymerization of a component of a bead.

In some embodiments, a bead may be formed from materials that comprise degradable chemical crosslinkers, such as BAC or cystamine. Degradation of such degradable crosslinkers may be accomplished through a number of mechanisms. In some examples, a bead may be contacted with a chemical degrading agent that may induce oxidation, reduction or other chemical changes. For example, a chemical degrading agent may be a reducing agent, such as dithiothreitol (DTT). Additional examples of reducing agents may include β-mercaptoethanol, (2S)-2-amino-1,4-dimercaptobutane (dithiobutylamine or DTBA), tris(2-carboxyethyl) phosphine (TCEP), or combinations thereof. A reducing agent may degrade the disulfide bonds formed between gel precursors forming the bead, and thus, degrade the bead. In other cases, a change in pH of a solution, such as an increase in pH, may trigger degradation of a bead. In other cases, exposure to an aqueous solution, such as water, may trigger hydrolytic degradation, and thus degradation of the bead.

Beads may also be induced to release their contents upon the application of a thermal stimulus. A change in temperature can cause a variety of changes to a bead. For example, heat can cause a solid bead to liquefy. A change in heat may cause melting of a bead such that a portion of the bead degrades. In other cases, heat may increase the internal pressure of the bead components such that the bead ruptures or explodes. Heat may also act upon heat-sensitive polymers used as materials to construct beads.

The methods, compositions, devices, and kits of this disclosure may be used with any suitable agent to degrade beads. In some embodiments, changes in temperature or pH may be used to degrade thermo-sensitive or pH-sensitive bonds within beads. In some embodiments, chemical degrading agents may be used to degrade chemical bonds within beads by oxidation, reduction or other chemical changes. For example, a chemical degrading agent may be a reducing agent, such as DTT, wherein DTT may degrade the disulfide bonds formed between a crosslinker and gel precursors, thus degrading the bead. In some embodiments, a reducing agent may be added to degrade the bead, which may or may not cause the bead to release its contents. Examples of reducing agents may include dithiothreitol (DTT), β-mercaptoethanol, (2S)-2-amino-1,4-dimercaptobutane (dithiobutylamine or DTBA), tris(2-carboxyethyl) phosphine (TCEP), or combinations thereof. The reducing agent may be present at a concentration of about 0.1mM, 0.5mM, 1mM, 5mM, or 10mM. The reducing agent may be present at a concentration of at least about 0.1mM, 0.5mM, 1mM, 5mM, 10mM, or greater. The reducing agent may be present at concentration of at most about 0.1mM, 0.5mM, 1mM, 5mM, or 10mM,

Any suitable number of nucleic acid molecules (e.g., primer, e.g., barcoded oligonucleotide) can be associated with a bead such that, upon release from the bead, the nucleic acid molecules (e.g., primer, e.g., barcoded oligonucleotide) are present in the partition at a predefined concentration. Such pre-defined concentration may be selected to facilitate certain reactions for generating a sequencing library, e.g., amplification, within the partition. In some cases, the pre-defined concentration of the primer is limited by the process of producing oligonucleotide bearing beads.

In some aspects, the partitions refer to containers or vessels (such as wells, microwells, tubes, vials, through ports in nanoarray substrates, e.g., BioTrove nanoarrays, or other containers). In some aspects, the compartments or partitions comprise partitions that are flowable within fluid streams. These partitions may comprise, e.g., micro-vesicles that have an outer barrier surrounding an inner fluid center or core, or, in some cases, they may comprise a porous matrix that is capable of entraining and/or retaining materials within its matrix. In some aspects, partitions comprise droplets of aqueous fluid within a non-aqueous continuous phase, e.g., an oil phase. A variety of different vessels are described in, for example, U.S. Patent Application Publication No. 20140155295. Emulsion systems for creating stable droplets in non-aqueous or oil continuous phases are described in detail in, e.g., U.S. Patent Application Publication No. 20100105112.

In the case of droplets in an emulsion, allocating individual cells to discrete partitions may generally be accomplished by introducing a flowing stream of cells in an aqueous fluid into a flowing stream of a non-aqueous fluid, such that droplets are generated at the junction of the two streams. By providing the aqueous cell-containing stream at a certain concentration of cells, the occupancy of the resulting partitions (e.g., number of cells per partition) can be controlled. Where single cell partitions are desired, the relative flow rates of the fluids can be selected such that, on average, the partitions contain less than one cell per partition, in order to ensure that those partitions that are occupied, are primarily singly occupied. In some embodiments, the relative flow rates of the fluids can be selected such that a majority of partitions are occupied, e.g., allowing for only a small percentage of unoccupied partitions. In some aspects, the flows and channel architectures are controlled as to ensure a desired number of singly occupied partitions, less than a certain level of unoccupied partitions and less than a certain level of multiply occupied partitions.

The systems and methods described herein can be operated such that a majority of occupied partitions include no more than one cell per occupied partition. In some cases, the partitioning process is conducted such that fewer than 25% of the occupied partitions contain more than one cell, and in many cases, fewer than 20% of the occupied partitions have more than one cell. In some cases, fewer than 10% or even fewer than 5% of the occupied partitions include more than one cell per partition.

In some cases, it is desirable to avoid the creation of excessive numbers of empty partitions. For example, from a cost perspective and/or efficiency perspective, it may desirable to minimize the number of empty partitions. While this may be accomplished by providing sufficient numbers of cells into the partitioning zone, the Poissonian distribution may expectedly increase the number of partitions that may include multiple cells. As such, in accordance with aspects described herein, the flow of one or more of the cells, or other fluids directed into the partitioning zone are conducted such that, in many cases, no more than 50% of the generated partitions, no more than 25% of the generated partitions, or no more than 10% of the generated partitions are unoccupied. Further, in some aspects, these flows are controlled so as to present non-Poissonian distribution of single occupied partitions while providing lower levels of unoccupied partitions, Restated, in some aspects, the above noted ranges of unoccupied partitions can be achieved while still providing any of the single occupancy rates described above. For example, in many cases, the use of the systems and methods described herein creates resulting partitions that have multiple occupancy rates of less than 25%, less than 20%, less than 15%, less than 10%, and in many cases, less than 5%, while having unoccupied partitions of less than 50%, less than 40%, less than 30%, less than 20%, less than 10%, and in some cases, less than 5%.

As will be appreciated, the above-described occupancy rates are also applicable to partitions that include both cells and additional reagents, including, but not limited to, microcapsules carrying barcoded oligonucleotides. In some aspects, a substantial percentage of the overall occupied partitions can include both a microcapsule (e.g., bead) comprising barcoded oligonucleotides and a cell.

Although described in terms of providing substantially singly occupied partitions, above, in certain cases, it is desirable to provide multiply occupied partitions, e.g., containing two, three, four or more cells and/or microcapsules (e.g., beads) comprising barcoded oligonucleotides within a single partition. Accordingly, as noted above, the flow characteristics of the cell and/or bead containing fluids and partitioning fluids may be controlled to provide for such multiply occupied partitions. In particular, the flow parameters may be controlled to provide a desired occupancy rate at greater than 50% of the partitions, greater than 75%, and in some cases greater than 80%, 90%, 95%, or higher.

In some cases, additional microcapsules are used to deliver additional reagents to a partition. In such cases, it may be advantageous to introduce different beads into a common channel or droplet generation junction, from different bead sources, i.e., containing different associated reagents, through different channel inlets into such common channel or droplet generation junction. In such cases, the flow and frequency of the different beads into the channel or junction may be controlled to provide for the desired ratio of microcapsules from each source, while ensuring the desired pairing or combination of such beads into a partition with the desired number of cells.

The partitions described herein may comprise small volumes, e.g., less than 10 $\mu$L, less than 5$\mu$L, less than 1$\mu$L, less than 900 picoliters (pL), less than 800 pL, less than 700 pL, less than 600 pL, less than 500 pL, less than 400pL, less than 300 pL, less than 200 pL, less than 100pL, less than 50 pL, less than 20 pL, less than 10 pL, less than 1 pL, less than 500 nanoliters (nL), or even less than 100 nL, 50 nL, or even less.

For example, in the case of droplet based partitions, the droplets may have overall volumes that are less than 1000 pL, less than 900 pL, less than 800 pL, less than 700 pL, less than 600 pL, less than 500 pL, less than 400pL, less than 300 pL, less than 200 pL, less than 100pL, less than 50 pL, less than 20 pL, less than 10 pL, or even less than 1 pL. Where co-partitioned with microcapsules, it will be appreciated that the sample fluid volume, e.g., including co-partitioned cells, within the partitions may be less than 90% of the above described volumes, less than 80%, less than 70%, less than 60%, less than 50%, less than 40%, less than 30%, less than 20%, or even less than 10% the above described volumes.

As is described elsewhere herein, partitioning species may generate a population or plurality of partitions. In such cases, any suitable number of partitions can be generated to generate the plurality of partitions. For example, in a method described herein, a plurality of partitions may be generated that comprises at least about 1,000 partitions, at least about 5,000 partitions, at least about 10,000 partitions, at least about 50,000 partitions, at least about 100,000 partitions, at least about 500,000 partitions, at least about 1,000,000 partitions, at least about 5,000,000 partitions at least about 10,000,000 partitions, at least about 50,000,000 partitions, at least about 100,000,000 partitions, at least about 500,000,000 partitions or at least about 1,000,000,000 partitions. Moreover, the plurality of partitions may comprise both unoccupied partitions (e.g., empty partitions) and occupied partitions

Microfluidic channel networks can be utilized to generate partitions as described herein. Alternative mechanisms may also be employed in the partitioning of individual cells, including porous membranes through which aqueous mixtures of cells are extruded into non-aqueous fluids.

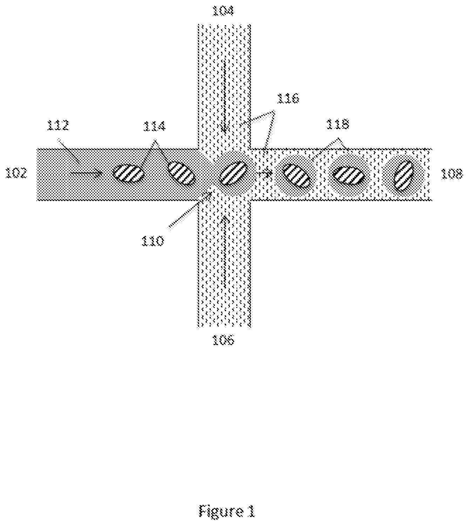

An example of a simplified microfluidic channel structure for partitioning individual cells is illustrated in Figure 1. As described elsewhere herein, in some cases, the majority of occupied partitions include no more than one cell per occupied partition and, in some cases, some of the generated partitions are unoccupied. In some cases, though, some of the occupied partitions may include more than one cell. In some cases, the partitioning process may be controlled such that fewer than 25% of the occupied partitions contain more than one cell, and in many cases, fewer than 20% of the occupied partitions have more than one cell, while in some cases, fewer than 10% or even fewer than 5% of the occupied partitions include more than one cell per partition. As shown, the channel structure can include channel segments 102, 104, 106 and 108 communicating at a channel junction 110. In operation, a first aqueous fluid 112 that includes suspended cells 114, may be transported along channel segment 102 into junction 110, while a second fluid 116 that is immiscible with the aqueous fluid 112 is delivered to the junction 110 from channel segments 104 and 106 to create discrete droplets 118 of the aqueous fluid including individual cells 114, flowing into channel segment 108.

In some aspects, this second fluid 116 comprises an oil, such as a fluorinated oil, that includes a fluorosurfactant for stabilizing the resulting droplets, e.g., inhibiting subsequent coalescence of the resulting droplets. Examples of particularly useful partitioning fluids and fluorosurfactants are described for example, in U.S. Patent Application Publication No. 20100105112.

In other aspects, in addition to or as an alternative to droplet based partitioning, cells may be encapsulated within a microcapsule that comprises an outer shell or layer or porous matrix in which is entrained one or more individual cells or small groups of cells, and may include other reagents. Encapsulation of cells may be carried out by a variety of processes. Such processes combine an aqueous fluid containing the cells to be analyzed with a polymeric precursor material that may be capable of being formed into a gel or other solid or semi-solid matrix upon application of a particular stimulus to the polymer precursor. Such stimuli include, e.g., thermal stimuli (either heating or cooling), photo-stimuli (e.g., through photo-curing), chemical stimuli (e.g., through crosslinking, polymerization initiation of the precursor (e.g., through added initiators), or the like.

Preparation of microcapsules comprising cells may be carried out by a variety of methods. For example, air knife droplet or aerosol generators may be used to dispense droplets of precursor fluids into gelling solutions in order to form microcapsules that include individual cells or small groups of cells. Likewise, membrane based encapsulation systems may be used to generate microcapsules comprising encapsulated cells as described herein. In some aspects, microfluidic systems like that shown in Figure 1 may be readily used in encapsulating cells as described herein. In particular, and with reference to Figure 1, the aqueous fluid comprising the cells and the polymer precursor material is flowed into channel junction 110, where it is partitioned into droplets 118 comprising the individual cells 114, through the flow of non-aqueous fluid 116. In the case of encapsulation methods, non-aqueous fluid 116 may also include an initiator to cause polymerization and/or crosslinking of the polymer precursor to form the microcapsule that includes the entrained cells. Examples of polymer precursor/initiator pairs include those described in U.S. Patent Application Publication No. 20140378345.

For example, in the case where the polymer precursor material comprises a linear polymer material, e.g., a linear polyacrylamide, PEG, or other linear polymeric material, the activation agent may comprise a cross-linking agent, or a chemical that activates a cross-linking agent within the formed droplets. Likewise, for polymer precursors that comprise polymerizable monomers, the activation agent may comprise a polymerization initiator. For example, in certain cases, where the polymer precursor comprises a mixture of acrylamide monomer with a N,N'-bis-(acryloyl)cystamine (BAC) comonomer, an agent such as tetraethylmethylenediamine (TEMED) may be provided within the second fluid streams in channel segments 104 and 106, which initiates the copolymerization of the acrylamide and BAC into a cross-linked polymer network or, hydrogel.

Upon contact of the second fluid stream 116 with the first fluid stream 112 at junction 110 in the formation of droplets, the TEMED may diffuse from the second fluid 116 into the aqueous first fluid 112 comprising the linear polyacrylamide, which will activate the crosslinking of the polyacrylamide within the droplets, resulting in the formation of the gel, e.g., hydrogel, microcapsules 118, as solid or semi-solid beads or particles entraining the cells 114. Although described in terms of polyacrylamide encapsulation, other 'activatable' encapsulation compositions may also be employed in the context of the methods and compositions described herein. For example, formation of alginate droplets followed by exposure to divalent metal ions, e.g., Ca2+, can be used as an encapsulation process using the described processes. Likewise, agarose droplets may also be transformed into capsules through temperature based gelling, e.g., upon cooling, or the like. In some cases, encapsulated cells can be selectively releasable from the microcapsule, e.g., through passage of time, or upon application of a particular stimulus, that degrades the microcapsule sufficiently to allow the cell, or its contents to be released from the microcapsule, e.g., into a partition, such as a droplet. For example, in the case of the polyacrylamide polymer described above, degradation of the microcapsule may be accomplished through the introduction of an appropriate reducing agent, such as DTT or the like, to cleave disulfide bonds that cross link the polymer matrix (See, e.g., U.S. Patent Application Publication No. 20140378345.

Encapsulated cells or cell populations provide certain potential advantages of being storable, and more portable than droplet based partitioned cells. Furthermore, in some cases, it may be desirable to allow cells to be analyzed to incubate for a select period of time, in order to characterize changes in such cells over time, either in the presence or absence of different stimuli. In such cases, encapsulation of individual cells may allow for longer incubation than partitioning in emulsion droplets, although in some cases, droplet partitioned cells may also be incubated for different periods of time, e.g., at least 10 seconds, at least 30 seconds, at least 1 minute, at least 5 minutes, at least 10 minutes, at least 30 minutes, at least 1 hour, at least 2 hours, at least 5 hours, or at least 10 hours or more. The encapsulation of cells may constitute the partitioning of the cells into which other reagents are co-partitioned. Alternatively, encapsulated cells may be readily deposited into other partitions, e.g., droplets, as described above.

In accordance with certain aspects, the cells may be partitioned along with lysis reagents in order to release the contents of the cells within the partition. In such cases, the lysis agents can be contacted with the cell suspension concurrently with, or immediately prior to the introduction of the cells into the partitioning junction/droplet generation zone, e.g., through an additional channel or channels upstream of channel junction 110. Examples of lysis agents include bioactive reagents, such as lysis enzymes that are used for lysis of different cell types, e.g., gram positive or negative bacteria, plants, yeast, mammalian, etc., such as lysozymes, achromopeptidase, lysostaphin, labiase, kitalase, lyticase, and a variety of other lysis enzymes available from, e.g., Sigma-Aldrich, Inc. (St Louis, MO), as well as other commercially available lysis enzymes. Other lysis agents may additionally or alternatively be co-partitioned with the cells to cause the release of the cell's contents into the partitions. For example, in some cases, surfactant based lysis solutions may be used to lyse cells, although these may be less desirable for emulsion based systems where the surfactants can interfere with stable emulsions. In some cases, lysis solutions may include non-ionic surfactants such as, for example, TritonX-100 and Tween 20. In some cases, lysis solutions may include ionic surfactants such as, for example, sarcosyl and sodium dodecyl sulfate (SDS). Electroporation, thermal, acoustic or mechanical cellular disruption may also be used in certain cases, e.g., non-emulsion based partitioning such as encapsulation of cells that may be in addition to or in place of droplet partitioning, where any pore size of the encapsulate is sufficiently small to retain nucleic acid fragments of a desired size, following cellular disruption.

In addition to the lysis agents co-partitioned with the cells described above, other reagents can also be co-partitioned with the cells, including, for example, DNase and RNase inactivating agents or inhibitors, such as proteinase K, chelating agents, such as EDTA, and other reagents employed in removing or otherwise reducing negative activity or impact of different cell lysate components on subsequent processing of nucleic acids. In addition, in the case of encapsulated cells, the cells may be exposed to an appropriate stimulus to release the cells or their contents from a co-partitioned microcapsule. For example, in some cases, a chemical stimulus may be co-partitioned along with an encapsulated cell to allow for the degradation of the microcapsule and release of the cell or its contents into the larger partition. In some cases, this stimulus may be the same as the stimulus described elsewhere herein for release of oligonucleotides from their respective microcapsule (e.g., bead). In alternative aspects, this may be a different and non-overlapping stimulus, in order to allow an encapsulated cell to be released into a partition at a different time from the release of oligonucleotides into the same partition.

Additional reagents may also be co-partitioned with the cells, such as endonucleases to fragment the cell's DNA, DNA polymerase enzymes and dNTPs used to amplify the cell's nucleic acid fragments and to attach the barcode oligonucleotides to the amplified fragments. Additional reagents may also include reverse transcriptase enzymes, including enzymes with terminal transferase activity, primers and oligonucleotides, and switch oligonucleotides (also referred to herein as "switch oligos" or "template switching oligonucleotides") which can be used for template switching. In some cases, template switching can be used to increase the length of a cDNA. In some cases, template switching can be used to append a predefined nucleic acid sequence to the cDNA. In one example of template switching, cDNA can be generated from reverse transcription of a template, e.g., cellular mRNA, where a reverse transcriptase with terminal transferase activity can add additional nucleotides, e.g., polyC, to the cDNA in a template independent manner. Switch oligos can include sequences complementary to the additional nucleotides, e.g., polyG. The additional nucleotides (e.g., polyC) on the cDNA can hybridize to the additional nucleotides (e.g., polyG) on the switch oligo, whereby the switch oligo can be used by the reverse transcriptase as template to further extend the cDNA. Template switching oligonucleotides may comprise a hybridization region and a template region. The hybridization region can comprise any sequence capable of hybridizing to the target. In some cases, as previously described, the hybridization region comprises a series of G bases to complement the overhanging C bases at the 3' end of a cDNA molecule. The series of G bases may comprise 1 G base, 2 G bases, 3 G bases, 4 G bases, 5 G bases or more than 5 G bases. The template sequence can comprise any sequence to be incorporated into the cDNA. In some cases, the template region comprises at least 1 (e.g., at least 2, 3, 4, 5 or more) tag sequences and/or functional sequences. Switch oligos may comprise deoxyribonucleic acids; ribonucleic acids; modified nucleic acids including 2-Aminopurine, 2,6-Diaminopurine (2-Amino-dA), inverted dT, 5-Methyl dC, 2'-deoxyInosine, Super T (5-hydroxybutynl-2'-deoxyuridine), Super G (8-aza-7-deazaguanosine), locked nucleic acids (LNAs), unlocked nucleic acids (UNAs, e.g., UNA-A, UNA-U, UNA-C, UNA-G), Iso-dG, Iso-dC, 2' Fluoro bases (e.g., Fluoro C, Fluoro U, Fluoro A, and Fluoro G), or any combination.

In some cases, the length of a switch oligo may be 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250 nucleotides or longer.

In some cases, the length of a switch oligo may be at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249 or 250 nucleotides or longer.

In some cases, the length of a switch oligo may be at most 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249 or 250 nucleotides.

Once the contents of the cells are released into their respective partitions, the nucleic acids contained therein may be further processed within the partitions. In accordance with the methods and systems described herein, the nucleic acid contents of individual cells can be provided with unique identifiers such that, upon characterization of those nucleic acids they may be attributed as having been derived from the same cell or cells. The ability to attribute characteristics to individual cells or groups of cells is provided by the assignment of unique identifiers specifically to an individual cell or groups of cells. Unique identifiers, e.g., in the form of nucleic acid barcodes can be assigned or associated with individual cells or populations of cells, in order to tag or label the cell's components (and as a result, its characteristics) with the unique identifiers. These unique identifiers can then be used to attribute the cell's components and characteristics to an individual cell or group of cells. In some aspects, this is carried out by co-partitioning the individual cells or groups of cells with the unique identifiers. In some aspects, the unique identifiers are provided in the form of oligonucleotides that comprise nucleic acid barcode sequences that may be attached to or otherwise associated with the nucleic acid contents of individual cells, or to other components of the cells, and particularly to fragments of those nucleic acids. The oligonucleotides are partitioned such that as between oligonucleotides in a given partition, the nucleic acid barcode sequences contained therein are the same, but as between different partitions, the oligonucleotides can, and do have differing barcode sequences, or at least represent a large number of different barcode sequences across all of the partitions in a given analysis. In some aspects, only one nucleic acid barcode sequence can be associated with a given partition, although in some cases, two or more different barcode sequences may be present.

The nucleic acid barcode sequences can include from 6 to about 20 or more nucleotides within the sequence of the oligonucleotides. In some cases, the length of a barcode sequence may be 6, 7, 8, 9, 10, 11, 12, 13, 14, 15, 16, 17, 18, 19, 20 nucleotides or longer. In some cases, the length of a barcode sequence may be at least 6, 7, 8, 9, 10, 11, 12, 13, 14, 15, 16, 17, 18, 19, 20 nucleotides or longer. In some cases, the length of a barcode sequence may be at most 6, 7, 8, 9, 10, 11, 12, 13, 14, 15, 16, 17, 18, 19, 20 nucleotides or shorter. These nucleotides may be completely contiguous, i.e., in a single stretch of adjacent nucleotides, or they may be separated into two or more separate subsequences that are separated by 1 or more nucleotides. In some cases, separated barcode subsequences can be from about 4 to about 16 nucleotides in length. In some cases, the barcode subsequence may be 4, 5, 6, 7, 8, 9, 10, 11, 12, 13, 14, 15, 16 nucleotides or longer. In some cases, the barcode subsequence may be at least 4, 5, 6, 7, 8, 9, 10, 11, 12, 13, 14, 15, 16 nucleotides or longer. In some cases, the barcode subsequence may be at most 4, 5, 6, 7, 8, 9, 10, 11, 12, 13, 14, 15, 16 nucleotides or shorter.

The co-partitioned oligonucleotides can also comprise other functional sequences useful in the processing of the nucleic acids from the co-partitioned cells. These sequences include, e.g., targeted or random/universal amplification primer sequences for amplifying the genomic DNA from the individual cells within the partitions while attaching the associated barcode sequences, sequencing primers or primer recognition sites, hybridization or probing sequences, e.g., for identification of presence of the sequences or for pulling down barcoded nucleic acids, or any of a number of other potential functional sequences. Other mechanisms of co-partitioning oligonucleotides may also be employed, including, e.g., coalescence of two or more droplets, where one droplet contains oligonucleotides, or microdispensing of oligonucleotides into partitions, e.g., droplets within microfluidic systems.

Briefly, in one example, microcapsules, such as beads, are provided that each include large numbers of the above described barcoded oligonucleotides releasably attached to the beads, where all of the oligonucleotides attached to a particular bead will include the same nucleic acid barcode sequence, but where a large number of diverse barcode sequences are represented across the population of beads used. In some embodiments, hydrogel beads, e.g., comprising polyacrylamide polymer matrices, are used as a solid support and delivery vehicle for the oligonucleotides into the partitions, as they are capable of carrying large numbers of oligonucleotide molecules, and may be configured to release those oligonucleotides upon exposure to a particular stimulus, as described elsewhere herein. In some cases, the population of beads will provide a diverse barcode sequence library that includes at least 1,000 different barcode sequences, at least 5,000 different barcode sequences, at least 10,000 different barcode sequences, at least at least 50,000 different barcode sequences, at least 100,000 different barcode sequences, at least 1,000,000 different barcode sequences, at least 5,000,000 different barcode sequences, or at least 10,000,000 different barcode sequences. Additionally, each bead can be provided with large numbers of oligonucleotide molecules attached. In particular, the number of molecules of oligonucleotides including the barcode sequence on an individual bead can be at least 1,000 oligonucleotide molecules, at least 5,000 oligonucleotide molecules, at least 10,000 oligonucleotide molecules, at least 50,000 oligonucleotide molecules, at least 100,000 oligonucleotide molecules, at least 500,000 oligonucleotides, at least 1,000,000 oligonucleotide molecules, at least 5,000,000 oligonucleotide molecules, at least 10,000,000 oligonucleotide molecules, at least 50,000,000 oligonucleotide molecules, at least 100,000,000 oligonucleotide molecules, and in some cases at least 1 billion oligonucleotide molecules.

Moreover, when the population of beads is partitioned, the resulting population of partitions can also include a diverse barcode library that includes at least 1,000 different barcode sequences, at least 5,000 different barcode sequences, at least 10,000 different barcode sequences, at least at least 50,000 different barcode sequences, at least 100,000 different barcode sequences, at least 1,000,000 different barcode sequences, at least 5,000,000 different barcode sequences, or at least 10,000,000 different barcode sequences. Additionally, each partition of the population can include at least 1,000 oligonucleotide molecules, at least 5,000 oligonucleotide molecules, at least 10,000 oligonucleotide molecules, at least 50,000 oligonucleotide molecules, at least 100,000 oligonucleotide molecules, at least 500,000 oligonucleotides, at least 1,000,000 oligonucleotide molecules, at least 5,000,000 oligonucleotide molecules, at least 10,000,000 oligonucleotide molecules, at least 50,000,000 oligonucleotide molecules, at least 100,000,000 oligonucleotide molecules, and in some cases at least 1 billion oligonucleotide molecules.

In some cases, it may be desirable to incorporate multiple different barcodes within a given partition, either attached to a single or multiple beads within the partition. For example, in some cases, a mixed, but known barcode sequences set may provide greater assurance of identification in the subsequent processing, e.g., by providing a stronger address or attribution of the barcodes to a given partition, as a duplicate or independent confirmation of the output from a given partition.

The oligonucleotides are releasable from the beads upon the application of a particular stimulus to the beads. In some cases, the stimulus may be a photo-stimulus, e.g., through cleavage of a photo-labile linkage that releases the oligonucleotides. In other cases, a thermal stimulus may be used, where elevation of the temperature of the beads environment will result in cleavage of a linkage or other release of the oligonucleotides form the beads. In still other cases, a chemical stimulus is used that cleaves a linkage of the oligonucleotides to the beads, or otherwise results in release of the oligonucleotides from the beads. In one case, such compositions include the polyacrylamide matrices described above for encapsulation of cells, and may be degraded for release of the attached oligonucleotides through exposure to a reducing agent, such as DTT.

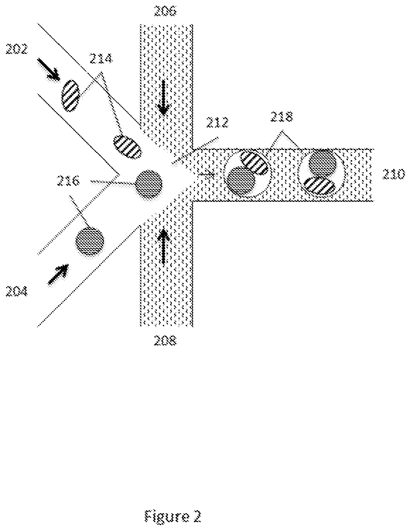

In accordance with the methods and systems described herein, the beads including the attached oligonucleotides are co-partitioned with the individual cells, such that a single bead and a single cell are contained within an individual partition. As noted above, while single cell/single bead occupancy is the most desired state, it will be appreciated that multiply occupied partitions (either in terms of cells, beads or both), or unoccupied partitions (either in terms of cells, beads or both) will often be present. An example of a microfluidic channel structure for co-partitioning cells and beads comprising barcode oligonucleotides is schematically illustrated in Figure 2. As described elsewhere herein, in some aspects, a substantial percentage of the overall occupied partitions will include both a bead and a cell and, in some cases, some of the partitions that are generated will be unoccupied. In some cases, some of the partitions may have beads and cells that are not partitioned 1:1. In some cases, it may be desirable to provide multiply occupied partitions, e.g., containing two, three, four or more cells and/or beads within a single partition. As shown, channel segments 202, 204, 206, 208 and 210 are provided in fluid communication at channel junction 212. An aqueous stream comprising the individual cells 214, is flowed through channel segment 202 toward channel junction 212. As described above, these cells may be suspended within an aqueous fluid, or may have been pre-encapsulated, prior to the partitioning process.

Concurrently, an aqueous stream comprising the barcode carrying beads 216, is flowed through channel segment 204 toward channel junction 212. A non-aqueous partitioning fluid 216 is introduced into channel junction 212 from each of side channels 206 and 208, and the combined streams are flowed into outlet channel 210. Within channel junction 212, the two combined aqueous streams from channel segments 202 and 204 are combined, and partitioned into droplets 218, that include co-partitioned cells 214 and beads 216. As noted previously, by controlling the flow characteristics of each of the fluids combining at channel junction 212, as well as controlling the geometry of the channel junction, partitioning can be optimized to achieve a desired occupancy level of beads, cells or both, within the partitions 218 that are generated.

In some cases, lysis agents, e.g., cell lysis enzymes, may be introduced into the partition with the bead stream, e.g., flowing through channel segment 204, such that lysis of the cell only commences at or after the time of partitioning. Additional reagents may also be added to the partition in this configuration, such as endonucleases to fragment the cell's DNA, DNA polymerase enzyme and dNTPs used to amplify the cell's nucleic acid fragments and to attach the barcode oligonucleotides to the amplified fragments. As noted above, in many cases, a chemical stimulus, such as DTT, may be used to release the barcodes from their respective beads into the partition. In such cases, it may be particularly desirable to provide the chemical stimulus along with the cell-containing stream in channel segment 202, such that release of the barcodes only occurs after the two streams have been combined, e.g., within the partitions 218. Where the cells are encapsulated, however, introduction of a common chemical stimulus, e.g., that both releases the oligonucleotides form their beads, and releases cells from their microcapsules may generally be provided from a separate additional side channel (not shown) upstream of or connected to channel junction 212.

A number of other reagents may be co-partitioned along with the cells, beads, lysis agents and chemical stimuli, including, for example, protective reagents, like proteinase K, chelators, nucleic acid extension, replication, transcription or amplification reagents such as polymerases, reverse transcriptases, transposases which can be used for transposon based methods (e.g., Nextera), nucleoside triphosphates or NTP analogues, primer sequences and additional cofactors such as divalent metal ions used in such reactions, ligation reaction reagents, such as ligase enzymes and ligation sequences, dyes, labels, or other tagging reagents.

The channel networks, e.g., as described herein, can be fluidly coupled to appropriate fluidic components. For example, the inlet channel segments, e.g., channel segments 202, 204, 206 and 208 are fluidly coupled to appropriate sources of the materials they are to deliver to channel junction 212. For example, channel segment 202 will be fluidly coupled to a source of an aqueous suspension of cells 214 to be analyzed, while channel segment 204 may be fluidly coupled to a source of an aqueous suspension of beads 216. Channel segments 206 and 208 may then be fluidly connected to one or more sources of the non-aqueous fluid. These sources may include any of a variety of different fluidic components, from simple reservoirs defined in or connected to a body structure of a microfluidic device, to fluid conduits that deliver fluids from off-device sources, manifolds, or the like. Likewise, the outlet channel segment 210 may be fluidly coupled to a receiving vessel or conduit for the partitioned cells. Again, this may be a reservoir defined in the body of a microfluidic device, or it may be a fluidic conduit for delivering the partitioned cells to a subsequent process operation, instrument or component.

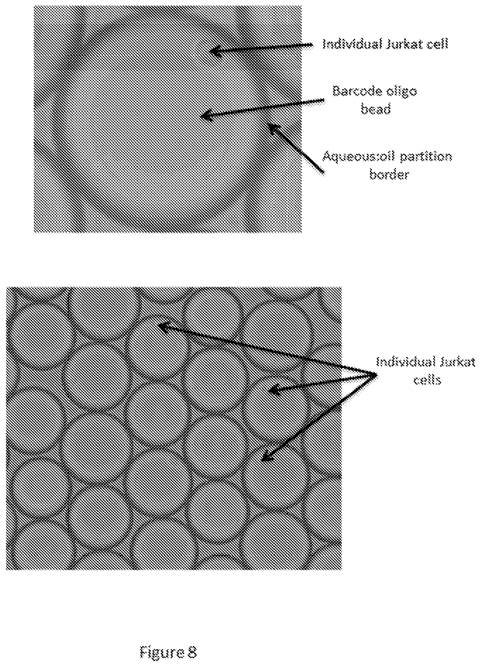

Figure 8 shows images of individual Jurkat cells co-partitioned along with barcode oligonucleotide containing beads in aqueous droplets in an aqueous in oil emulsion. As illustrated, individual cells may be readily co-partitioned with individual beads. As will be appreciated, optimization of individual cell loading may be carried out by a number of methods, including by providing dilutions of cell populations into the microfluidic system in order to achieve the desired cell loading per partition as described elsewhere herein.

In operation, once lysed, the nucleic acid contents of the individual cells are then available for further processing within the partitions, including, e.g., fragmentation, amplification and barcoding, as well as attachment of other functional sequences. As noted above, fragmentation may be accomplished through the co-partitioning of shearing enzymes, such as endonucleases, in order to fragment the nucleic acids into smaller fragments. These endonucleases may include restriction endonucleases, including type II and type IIs restriction endonucleases as well as other nucleic acid cleaving enzymes, such as nicking endonucleases, and the like. In some cases, fragmentation may not be desired, and full length nucleic acids may be retained within the partitions, or in the case of encapsulated cells or cell contents, fragmentation may be carried out prior to partitioning, e.g., through enzymatic methods, e.g., those described herein, or through mechanical methods, e.g., mechanical, acoustic or other shearing.

Once co-partitioned, and the cells are lysed to release their nucleic acids, the oligonucleotides disposed upon the bead may be used to barcode and amplify fragments of those nucleic acids. Briefly, in one aspect, the oligonucleotides present on the beads that are co-partitioned with the cells, are released from their beads into the partition with the cell's nucleic acids. The oligonucleotides can include, along with the barcode sequence, a primer sequence at its 5'end. This primer sequence may be a random oligonucleotide sequence intended to randomly prime numerous different regions on the cell's nucleic acids, or it may be a specific primer sequence targeted to prime upstream of a specific targeted region of the cell's genome.

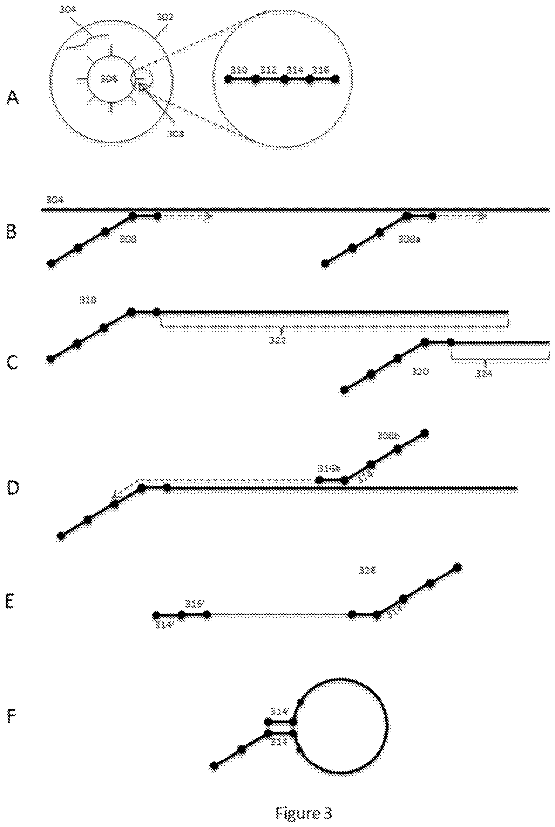

Once released, the primer portion of the oligonucleotide can anneal to a complementary region of the cell's nucleic acid. Extension reaction reagents, e.g., DNA polymerase, nucleoside triphosphates, co-factors (e.g., $Mg^{2+}$ or $Mn^{2+}$), that are also co-partitioned with the cells and beads, then extend the primer sequence using the cell's nucleic acid as a template, to produce a complementary fragment to the strand of the cell's nucleic acid to which the primer annealed, which complementary fragment includes the oligonucleotide and its associated barcode sequence. Annealing and extension of multiple primers to different portions of the cell's nucleic acids will result in a large pool of overlapping complementary fragments of the nucleic acid, each possessing its own barcode sequence indicative of the partition in which it was created. In some cases, these complementary fragments may themselves be used as a template primed by the oligonucleotides present in the partition to produce a complement of the complement that again, includes the barcode sequence. In some cases, this replication process is configured such that when the first complement is duplicated, it produces two complementary sequences at or near its termini, to allow formation of a hairpin structure or partial hairpin structure, the reduces the ability of the molecule to be the basis for producing further iterative copies. As described herein, the cell's nucleic acids may include any desired nucleic acids within the cell including, for example, the cell's DNA, e.g., genomic DNA, RNA, e.g., messenger RNA, and the like. For example, in some cases, the methods and systems described herein are used in characterizing expressed mRNA, including, e.g., the presence and quantification of such mRNA, and may include RNA sequencing processes as the characterization process. Alternatively or additionally, the reagents partitioned along with the cells may include reagents for the conversion of mRNA into cDNA, e.g., reverse transcriptase enzymes and reagents, to facilitate sequencing processes where DNA sequencing is employed. In some cases, where the nucleic acids to be characterized comprise RNA, e.g., mRNA, schematic illustration of one example of this is shown in Figure 3.

As shown, oligonucleotides that include a barcode sequence are co-partitioned in, e.g., a droplet 302 in an emulsion, along with a sample nucleic acid 304. As noted elsewhere herein, the oligonucleotides 308 may be provided on a bead 306 that is co-partitioned with the sample nucleic acid 304, which oligonucleotides are releasable from the bead 306, as shown in panel A. The oligonucleotides 308 include a barcode sequence 312, in addition to one or more functional sequences, e.g., sequences 310, 314 and 316. For example, oligonucleotide 308 is shown as comprising barcode sequence 312, as well as sequence 310 that may function as an attachment or immobilization sequence for a given sequencing system, e.g., a P5 sequence used for attachment in flow cells of an Illumina Hiseq® or Miseq® system. As shown, the oligonucleotides also include a primer sequence 316, which may include a random or targeted N-mer for priming replication of portions of the sample nucleic acid 304. Also included within oligonucleotide 308 is a sequence 314 which may provide a sequencing priming region, such as a "read1" or R1 priming region, that is used to prime polymerase mediated, template directed sequencing by synthesis reactions in sequencing systems. As will be appreciated, the functional sequences may be selected to be compatible with a variety of different sequencing systems, e.g., 454 Sequencing, Ion Torrent Proton or PGM, Illumina X10, etc., and the requirements thereof. In many cases, the barcode sequence 312, immobilization sequence 310 and R1 sequence 314 may be common to all of the oligonucleotides attached to a given bead. The primer sequence 316 may vary for random N-mer primers, or may be common to the oligonucleotides on a given bead for certain targeted applications.

As will be appreciated, in some cases, the functional sequences may include primer sequences useful for RNA-seq applications. For example, in some cases, the oligonucleotides may include poly-T primers for priming reverse transcription of RNA for RNA-seq. In still other cases, oligonucleotides in a given partition, e.g., included on an individual bead, may include multiple types of primer sequences in addition to the common barcode sequences, such as both DNA-sequencing and RNA sequencing primers, e.g., poly-T primer sequences included within the oligonucleotides coupled to the bead. In such cases, a single partitioned cell may be both subjected to DNA and RNA sequencing processes.

Based upon the presence of primer sequence 316, the oligonucleotides can prime the sample nucleic acid as shown in panel B, which allows for extension of the oligonucleotides 308 and 308a using polymerase enzymes and other extension reagents also co-partitioned with the bead 306 and sample nucleic acid 304. As shown in panel C, following extension of the oligonucleotides that, for random N-mer primers, may anneal to multiple different regions of the sample nucleic acid 304; multiple overlapping complements or fragments of the nucleic acid are created, e.g., fragments 318 and 320. Although including sequence portions that are complementary to portions of sample nucleic acid, e.g., sequences 322 and 324, these constructs are generally referred to herein as comprising fragments of the sample nucleic acid 304, having the attached barcode sequences.

The barcoded nucleic acid fragments may then be subjected to characterization, e.g., through sequence analysis, or they may be further amplified in the process, as shown in panel D. For example, additional oligonucleotides, e.g., oligonucleotide 308b, also released from bead 306, may prime the fragments 318 and 320. This shown for fragment 318. In particular, again, based upon the presence of the random N-mer primer 316b in oligonucleotide 308b (which in many cases can be different from other random N-mers in a given partition, e.g., primer sequence 316), the oligonucleotide anneals with the fragment 318, and is extended to create a complement 316 to at least a portion of fragment 318 which includes sequence 328, that comprises a duplicate of a portion of the sample nucleic acid sequence. Extension of the oligonucleotide 308b continues until it has replicated through the oligonucleotide portion 308 of fragment 318. As noted elsewhere herein, and as illustrated in panel D, the oligonucleotides may be configured to prompt a stop in the replication by the polymerase at a desired point, e.g., after replicating through sequences 316 and 314 of oligonucleotide 308 that is included within fragment 318. As described herein, this may be accomplished by different methods, including, for example, the incorporation of different nucleotides and/or nucleotide analogues that are not capable of being processed by the polymerase enzyme used. For example, this may include the inclusion of uracil containing nucleotides within the sequence region 312 to prevent a non-uracil tolerant polymerase to cease replication of that region. As a result a fragment 316 is created that includes the full-length oligonucleotide 308b at one end, including the barcode sequence 312, the attachment sequence 310, the R1 primer region 314, and the random N-mer sequence 316b. At the other end of the sequence may be included the complement 316' to the random N-mer of the first oligonucleotide 308, as well as a complement to all or a portion of the R1 sequence, shown as sequence 314'. The R1 sequence 314 and its complement 314' are then able to hybridize together to form a partial hairpin structure 328. As will be appreciated because the random N-mers differ among different oligonucleotides, these sequences and their complements may not be expected to participate in hairpin formation, e.g., sequence 316', which is the complement to random N-mer 316, may not be expected to be complementary to random N-mer sequence 316b. This may not be the case for other applications, e.g., targeted primers, where the N-mers may be common among oligonucleotides within a given partition.

By forming these partial hairpin structures, it allows for the removal of first level duplicates of the sample sequence from further replication, e.g., preventing iterative copying of copies. The partial hairpin structure also provides a useful structure for subsequent processing of the created fragments, e.g., fragment 316,

In general, the amplification of the cell's nucleic acids is carried out until the barcoded overlapping fragments within the partition constitute at least 1X coverage of the particular portion or all of the cell's genome, at least 2X, at least 3X, at least 4X, at least 5X, at least 10X, at least 20X, at least 40X or more coverage of the genome or its relevant portion of interest. Once the barcoded fragments are produced, they may be directly sequenced on an appropriate sequencing system, e.g., an Illumina Hiseq®, Miseq® or X10 system, or they may be subjected to additional processing, such as further amplification, attachment of other functional sequences, e.g., second sequencing primers, for reverse reads, sample index sequences, and the like.

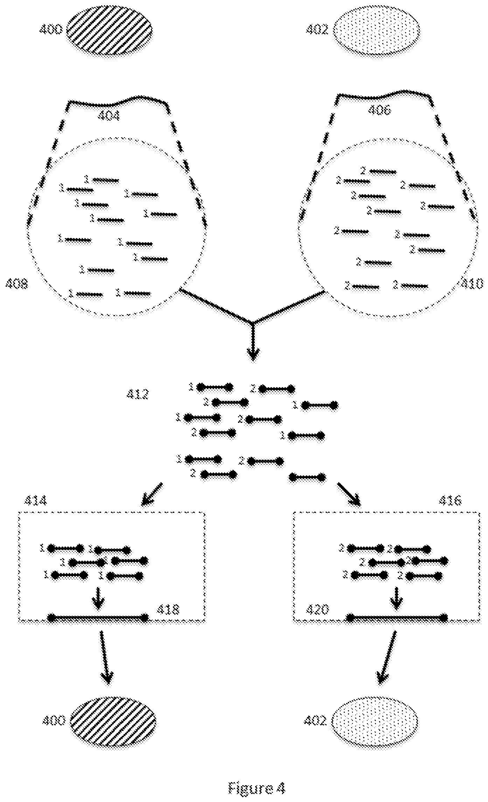

All of the fragments from multiple different partitions may then be pooled for sequencing on high throughput sequencers as described herein, where the pooled fragments comprise a large number of fragments derived from the nucleic acids of different cells or small cell populations, but where the fragments from the nucleic acids of a given cell will share the same barcode sequence. In particular, because each fragment is coded as to its partition of origin, and consequently its single cell or small population of cells, the sequence of that fragment may be attributed back to that cell or those cells based upon the presence of the barcode, which will also aid in applying the various sequence fragments from multiple partitions to assembly of individual genomes for different cells. This is schematically illustrated in Figure 4. As shown in one example, a first nucleic acid 404 from a first cell 400, and a second nucleic acid 406 from a second cell 402 are each partitioned along with their own sets of barcode oligonucleotides as described above. The nucleic acids may comprise a chromosome, entire genome or other large nucleic acid from the cells.

Within each partition, each cell's nucleic acids 404 and 406 is then processed to separately provide overlapping set of second fragments of the first fragment(s), e.g., second fragment sets 408 and 410. This processing also provides the second fragments with a barcode sequence that is the same for each of the second fragments derived from a particular first fragment. As shown, the barcode sequence for second fragment set 408 is denoted by "1" while the barcode sequence for fragment set 410 is denoted by "2". A diverse library of barcodes may be used to differentially barcode large numbers of different fragment sets. However, it is not necessary for every second fragment set from a different first fragment to be barcoded with different barcode sequences. In fact, in many cases, multiple different first fragments may be processed concurrently to include the same barcode sequence. Diverse barcode libraries are described in detail elsewhere herein.

The barcoded fragments, e.g., from fragment sets 408 and 410, may then be pooled for sequencing using, for example, sequence by synthesis technologies available from Illumina or Ion Torrent division of Thermo-Fisher, Inc. Once sequenced, the sequence reads 412 can be attributed to their respective fragment set, e.g., as shown in aggregated reads 414 and 416, at least in part based upon the included barcodes, and in some cases, in part based upon the sequence of the fragment itself. The attributed sequence reads for each fragment set are then assembled to provide the assembled sequence for each cell's nucleic acids, e.g., sequences 418 and 420, which in turn, may be attributed to individual cells, e.g., cells 400 and 402.

While described in terms of analyzing the genetic material present within cells, the methods and systems described herein may have much broader applicability, including the ability to characterize other aspects of individual cells or cell populations, by allowing for the allocation of reagents to individual cells, and providing for the attributable analysis or characterization of those cells in response to those reagents. These methods and systems are particularly valuable in being able to characterize cells for, e.g., research, diagnostic, pathogen identification, and many other purposes. By way of example, a wide range of different cell surface features, e.g., cell surface proteins like cluster of differentiation or CD proteins, have significant diagnostic relevance in characterization of diseases like cancer.

In one particularly useful application, the methods and systems described herein may be used to characterize cell features, such as cell surface features, e.g., proteins, receptors, etc. In particular, the methods described herein may be used to attach reporter molecules to these cell features, that when partitioned as described above, may be barcoded and analyzed, e.g., using DNA sequencing technologies, to ascertain the presence, and in some cases, relative abundance or quantity of such cell features within an individual cell or population of cells.

In a particular example, a library of potential cell binding ligands, e.g., antibodies, antibody fragments, cell surface receptor binding molecules, or the like, maybe provided associated with a first set of nucleic acid reporter molecules, e.g., where a different reporter oligonucleotide sequence is associated with a specific ligand, and therefore capable of binding to a specific cell surface feature. In some aspects, different members of the library may be characterized by the presence of a different oligonucleotide sequence label, e.g., an antibody to a first type of cell surface protein or receptor may have associated with it a first known reporter oligonucleotide sequence, while an antibody to a second receptor protein may have a different known reporter oligonucleotide sequence associated with it. Prior to co-partitioning, the cells may be incubated with the library of ligands, that may represent antibodies to a broad panel of different cell surface features, e.g., receptors, proteins, etc., and which include their associated reporter oligonucleotides. Unbound ligands are washed from the cells, and the cells are then co-partitioned along with the barcode oligonucleotides described above. As a result, the partitions will include the cell or cells, as well as the bound ligands and their known, associated reporter oligonucleotides.

Without the need for lysing the cells within the partitions, one may then subject the reporter oligonucleotides to the barcoding operations described above for cellular nucleic acids, to produce barcoded, reporter oligonucleotides, where the presence of the reporter oligonucleotides can be indicative of the presence of the particular cell surface feature, and the barcode sequence will allow the attribution of the range of different cell surface features to a given individual cell or population of cells based upon the barcode sequence that was co-partitioned with that cell or population of cells. As a result, one may generate a cell-by-cell profile of the cell surface features within a broader population of cells. This aspect of the methods and systems described herein, is described in greater detail below.

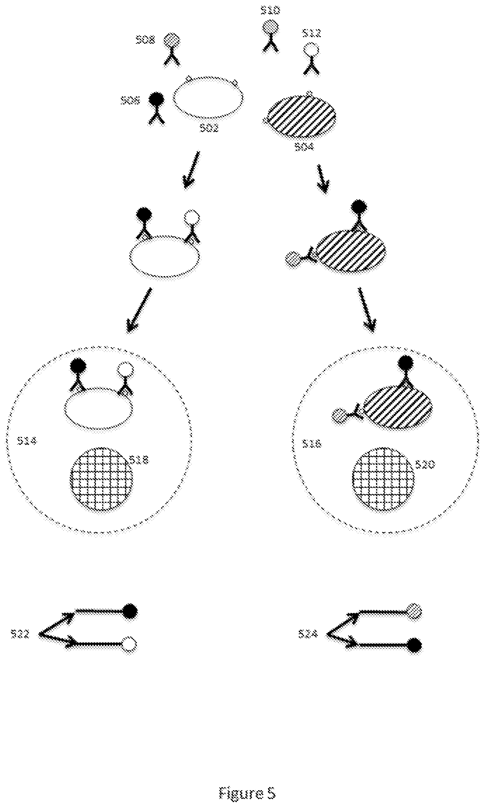

This example is schematically illustrated in Figure 5. As shown, a population of cells, represented by cells 502 and 504 are incubated with a library of cell surface associated reagents, e.g., antibodies, cell surface binding proteins, ligands or the like, where each different type of binding group includes an associated nucleic acid reporter molecule associated with it, shown as ligands and associated reporter molecules 506, 508, 510 and 512 (with the reporter molecules being indicated by the differently shaded circles). Where the cell expresses the surface features that are bound by the library, the ligands and their associated reporter molecules can become associated or coupled with the cell surface. Individual cells are then partitioned into separate partitions, e.g., droplets 514 and 516, along with their associated ligand/reporter molecules, as well as an individual barcode oligonucleotide bead as described elsewhere herein, e.g., beads 522 and 524, respectively. As with other examples described herein, the barcoded oligonucleotides are released from the beads and used to attach the barcode sequence the reporter molecules present within each partition with a barcode that is common to a given partition, but which varies widely among different partitions. For example, as shown in Figure 5, the reporter molecules that associate with cell 502 in partition 514 are barcoded with barcode sequence 518, while the reporter molecules associated with cell 504 in partition 516 are barcoded with barcode 520. As a result, one is provided with a library of oligonucleotides that reflects the surface ligands of the cell, as reflected by the reporter molecule, but which is substantially attributable to an individual cell by virtue of a common barcode sequence, allowing a single cell level profiling of the surface characteristics of the cell. As will be appreciated, this process is not limited to cell surface receptors but may be used to identify the presence of a wide variety of specific cell structures, chemistries or other characteristics.

Single cell processing and analysis methods and systems described herein can be utilized for a wide variety of applications, including analysis of specific individual cells, analysis of different cell types within populations of differing cell types, analysis and characterization of large populations of cells for environmental, human health, epidemiological forensic, or any of a wide variety of different applications.

A particularly valuable application of the single cell analysis processes described herein is in the sequencing and characterization of a diseased cell. A diseased cell can have altered metabolic properties, gene expression, and/or morphologic features. Examples of diseases include inflammatory disorders, metabolic disorders, nervous system disorders, and cancer.

Of particular interest are cancer cells. In particular, conventional analytical techniques, including the ensemble sequencing processes alluded to above, are not highly adept at picking small variations in genomic make-up of cancer cells, particularly where those exist in a sea of normal tissue cells. Further, even as between tumor cells, wide variations can exist and can be masked by the ensemble approaches to sequencing (See, e.g., Patel, et al., Single-cell RNA-seq highlights intratumoral heterogeneity in primary glioblastoma, Science DOI: 10.1126/science. 1254257 (Published online June 12, 2014). Cancer cells may be derived from solid tumors, hematological malignancies, cell lines, or obtained as circulating tumor cells, and subjected to the partitioning processes described above. Upon analysis, one can identify individual cell sequences as deriving from a single cell or small group of cells, and distinguish those over normal tissue cell sequences.

Non-limiting examples of cancer cells include cells of cancers such as Acanthoma, Acinic cell carcinoma, Acoustic neuroma, Acral lentiginous melanoma, Acrospiroma, Acute eosinophilic leukemia, Acute lymphoblastic leukemia, Acute megakaryoblastic leukemia, Acute monocytic leukemia, Acute myeloblastic leukemia with maturation, Acute myeloid dendritic cell leukemia, Acute myeloid leukemia, Acute promyelocytic leukemia, Adamantinoma, Adenocarcinoma, Adenoid cystic carcinoma, Adenoma, Adenomatoid odontogenic tumor, Adrenocortical carcinoma, Adult T-cell leukemia, Aggressive NK-cell leukemia, AIDS-Related Cancers, AIDS-related lymphoma, Alveolar soft part sarcoma, Ameloblastic fibroma, Anal cancer, Anaplastic large cell lymphoma, Anaplastic thyroid cancer, Angioimmunoblastic T-cell lymphoma, Angiomyolipoma, Angiosarcoma, Appendix cancer, Astrocytoma, Atypical teratoid rhabdoid tumor, Basal cell carcinoma, Basal-like carcinoma, B-cell leukemia, B-cell lymphoma, Bellini duct carcinoma, Biliary tract cancer, Bladder cancer, Blastoma, Bone Cancer, Bone tumor, Brain Stem Glioma, Brain Tumor, Breast Cancer, Brenner tumor, Bronchial Tumor, Bronchioloalveolar carcinoma, Brown tumor, Burkitt's lymphoma, Cancer of Unknown Primary Site, Carcinoid Tumor, Carcinoma, Carcinoma in situ, Carcinoma of the penis, Carcinoma of Unknown Primary Site, Carcinosarcoma, Castleman's Disease, Central Nervous System Embryonal Tumor, Cerebellar Astrocytoma, Cerebral Astrocytoma, Cervical Cancer, Cholangiocarcinoma, Chondroma, Chondrosarcoma, Chordoma, Choriocarcinoma, Choroid plexus papilloma, Chronic Lymphocytic Leukemia, Chronic monocytic leukemia, Chronic myelogenous leukemia, Chronic Myeloproliferative Disorder, Chronic neutrophilic leukemia, Clear-cell tumor, Colon Cancer, Colorectal cancer, Craniopharyngioma, Cutaneous T-cell lymphoma, Degos disease, Dermatofibrosarcoma protuberans, Dermoid cyst, Desmoplastic small round cell tumor, Diffuse large B cell lymphoma, Dysembryoplastic neuroepithelial tumor, Embryonal carcinoma, Endodermal sinus tumor, Endometrial cancer, Endometrial Uterine Cancer, Endometrioid tumor, Enteropathy-associated T-cell lymphoma, Ependymoblastoma, Ependymoma, Epithelioid sarcoma, Erythroleukemia,Esophageal cancer, Esthesioneuroblastoma, Ewing Family of Tumor, Ewing Family Sarcoma, Ewing's sarcoma, Extracranial Germ Cell Tumor, Extragonadal Germ Cell Tumor, Extrahepatic Bile Duct Cancer, Extramammary Paget's disease, Fallopian tube cancer, Fetus in fetu, Fibroma, Fibrosarcoma, Follicular lymphoma, Follicular thyroid cancer, Gallbladder Cancer, Gallbladder cancer, Ganglioglioma, Ganglioneuroma, Gastric Cancer, Gastric lymphoma, Gastrointestinal cancer, Gastrointestinal Carcinoid Tumor, Gastrointestinal Stromal Tumor, Gastrointestinal stromal tumor, Germ cell tumor, Germinoma, Gestational choriocarcinoma, Gestational Trophoblastic Tumor, Giant cell tumor of bone, Glioblastoma multiforme, Glioma, Gliomatosis cerebri, Glomus tumor, Glucagonoma, Gonadoblastoma, Granulosa cell tumor, Hairy Cell Leukemia, Hairy cell leukemia, Head and Neck Cancer, Head and neck cancer, Heart cancer, Hemangioblastoma, Hemangiopericytoma, Hemangiosarcoma, Hematological malignancy, Hepatocellular carcinoma, Hepatosplenic T-cell lymphoma, Hereditary breast-ovarian cancer syndrome, Hodgkin Lymphoma, Hodgkin's lymphoma, Hypopharyngeal Cancer, Hypothalamic Glioma, Inflammatory breast cancer, Intraocular Melanoma, Islet cell carcinoma, Islet Cell Tumor, Juvenile myelomonocytic leukemia, Kaposi Sarcoma, Kaposi's sarcoma, Kidney Cancer, Klatskin tumor, Krukenberg tumor, Laryngeal Cancer, Laryngeal cancer, Lentigo maligna melanoma, Leukemia, Leukemia, Lip and Oral Cavity Cancer, Liposarcoma, Lung cancer, Luteoma, Lymphangioma, Lymphangiosarcoma, Lymphoepithelioma, Lymphoid leukemia, Lymphoma, Macroglobulinemia, Malignant Fibrous Histiocytoma, Malignant fibrous histiocytoma, Malignant Fibrous Histiocytoma of Bone, Malignant Glioma, Malignant Mesothelioma, Malignant peripheral nerve sheath tumor, Malignant rhabdoid tumor, Malignant triton tumor, MALT lymphoma, Mantle cell lymphoma, Mast cell leukemia, Mediastinal germ cell tumor, Mediastinal tumor, Medullary thyroid cancer, Medulloblastoma, Medulloblastoma, Medulloepithelioma, Melanoma, Melanoma, Meningioma, Merkel Cell Carcinoma, Mesothelioma, Mesothelioma, Metastatic Squamous Neck Cancer with Occult Primary, Metastatic urothelial carcinoma, Mixed Mullerian tumor, Monocytic leukemia, Mouth Cancer, Mucinous tumor, Multiple Endocrine Neoplasia Syndrome, Multiple Myeloma, Multiple myeloma, Mycosis Fungoides, Mycosis fungoides, Myelodysplastic Disease, Myelodysplastic Syndromes, Myeloid leukemia, Myeloid sarcoma, Myeloproliferative Disease, Myxoma, Nasal Cavity Cancer, Nasopharyngeal Cancer, Nasopharyngeal carcinoma, Neoplasm, Neurinoma, Neuroblastoma, Neuroblastoma, Neurofibroma, Neuroma, Nodular melanoma, Non-Hodgkin Lymphoma, Non-Hodgkin lymphoma, Nonmelanoma Skin Cancer, Non-Small Cell Lung Cancer, Ocular oncology, Oligoastrocytoma, Oligodendroglioma, Oncocytoma, Optic nerve sheath meningioma, Oral Cancer, Oral cancer, Oropharyngeal Cancer, Osteosarcoma, Osteosarcoma, Ovarian Cancer, Ovarian cancer, Ovarian Epithelial Cancer, Ovarian Germ Cell Tumor, Ovarian Low Malignant Potential Tumor, Paget's disease of the breast, Pancoast tumor, Pancreatic Cancer, Pancreatic cancer, Papillary thyroid cancer, Papillomatosis, Paraganglioma, Paranasal Sinus Cancer, Parathyroid Cancer, Penile Cancer, Perivascular epithelioid cell tumor, Pharyngeal Cancer, Pheochromocytoma, Pineal Parenchymal Tumor of Intermediate Differentiation, Pineoblastoma, Pituicytoma, Pituitary adenoma, Pituitary tumor, Plasma Cell Neoplasm, Pleuropulmonary blastoma, Polyembryoma, Precursor T-lymphoblastic lymphoma, Primary central nervous system lymphoma, Primary effusion lymphoma, Primary Hepatocellular Cancer, Primary Liver Cancer, Primary peritoneal cancer, Primitive neuroectodermal tumor, Prostate cancer, Pseudomyxoma peritonei, Rectal Cancer, Renal cell carcinoma, Respiratory Tract Carcinoma Involving the NUT Gene on Chromosome 15, Retinoblastoma, Rhabdomyoma, Rhabdomyosarcoma, Richter's transformation, Sacrococcygeal teratoma, Salivary Gland Cancer, Sarcoma, Schwannomatosis, Sebaceous gland carcinoma, Secondary neoplasm, Seminoma, Serous tumor, Sertoli-Leydig cell tumor, Sex cord-stromal tumor, Sezary Syndrome, Signet ring cell carcinoma, Skin Cancer, Small blue round cell tumor, Small cell carcinoma, Small Cell Lung Cancer, Small cell lymphoma, Small intestine cancer, Soft tissue sarcoma, Somatostatinoma, Soot wart, Spinal Cord Tumor, Spinal tumor, Splenic marginal zone lymphoma, Squamous cell carcinoma, Stomach cancer, Superficial spreading melanoma, Supratentorial Primitive Neuroectodermal Tumor, Surface epithelial-stromal tumor, Synovial sarcoma, T-cell acute lymphoblastic leukemia, T-cell large granular lymphocyte leukemia, T-cell leukemia, T-cell lymphoma, T-cell prolymphocytic leukemia, Teratoma, Terminal lymphatic cancer, Testicular cancer, Thecoma, Throat Cancer, Thymic Carcinoma, Thymoma, Thyroid cancer, Transitional Cell Cancer of Renal Pelvis and Ureter, Transitional cell carcinoma, Urachal cancer, Urethral cancer, Urogenital neoplasm, Uterine sarcoma, Uveal melanoma, Vaginal Cancer, Verner Morrison syndrome, Verrucous carcinoma, Visual Pathway Glioma, Vulvar Cancer, Waldenstrom's macroglobulinemia, Warthin's tumor, Wilms' tumor, and combinations thereof.

Where cancer cells are to be analyzed, primer sequences useful in any of the various operations for attaching barcode sequences and/or amplification reactions may comprise gene specific sequences which target genes or regions of genes associated with or suspected of being associated with cancer. For example, this can include genes or regions of genes where the presence of mutations (e.g., insertions, deletions, polymorphisms, copy number variations, and gene fusions) associated with a cancerous condition are suspected to be present in a cell population.

As with cancer cell analysis, the analysis and diagnosis of fetal health or abnormality through the analysis of fetal cells is a difficult task using conventional techniques. In particular, in the absence of relatively invasive procedures, such as amniocentesis obtaining fetal cell samples can employ harvesting those cells from the maternal circulation. As will be appreciated, such circulating fetal cells make up an extremely small fraction of the overall cellular population of that circulation. As a result complex analyses are performed in order to characterize what of the obtained data is likely derived from fetal cells as opposed to maternal cells. By employing the single cell characterization methods and systems described herein, however, one can attribute genetic make up to individual cells, and categorize those cells as maternal or fetal based upon their respective genetic make-up. Further, the genetic sequence of fetal cells may be used to identify any of a number of genetic disorders, including, e.g., aneuploidy such as Down syndrome, Edwards syndrome, and Patau syndrome.

Also of interest are immune cells. Methods and compositions disclosed herein can be utilized for sequence analysis of the immune repertoire. Analysis of sequence information underlying the immune repertoire can provide a significant improvement in understanding the status and function of the immune system.

Non-limiting examples of immune cells which can be analyzed utilizing the methods described herein include B cells, T cells (e.g., cytotoxic T cells, natural killer T cells, regulatory T cells, and T helper cells), natural killer cells, cytokine induced killer (CIK) cells; myeloid cells, such as granulocytes (basophil granulocytes, eosinophil granulocytes, neutrophil granulocytes/hypersegmented neutrophils), monocytes/macrophages, mast cell, thrombocytes/megakaryocytes, and dendritic cells. In some embodiments, individual T cells are analyzed using the methods disclosed herein. In some embodiments, individual B cells are analyzed using the methods disclosed herein.

Immune cells express various adaptive immunological receptors relating to immune function, such as T cell receptors and B cell receptors. T cell receptors and B cells receptors play a part in the immune response by specifically recognizing and binding to antigens and aiding in their destruction.

The T cell receptor, or TCR, is a molecule found on the surface of T cells that is generally responsible for recognizing fragments of antigen as peptides bound to major histocompatibility complex (MHC) molecules. The TCR is generally a heterodimer of two chains, each of which is a member of the immunoglobulin superfamily, possessing an N-terminal variable (V) domain, and a C terminal constant domain. In humans, in 95% of T cells the TCR consists of an alpha ($\alpha$) and beta ($\beta$) chain, whereas in 5% of T cells the TCR consists of gamma and delta ($\gamma/\delta$) chains. This ratio can change during ontogeny and in diseased states as well as in different species. When the TCR engages with antigenic peptide and MHC (peptide/MHC), the T lymphocyte is activated through signal transduction.

Each of the two chains of a TCR contains multiple copies of gene segments - a variable 'V' gene segment, a diversity 'D' gene segment, and a joining 'J' gene segment. The TCR alpha chain is generated by recombination of V and J segments, while the beta chain is generated by recombination of V, D, and J segments. Similarly, generation of the TCR gamma chain involves recombination of V and J gene segments, while generation of the TCR delta chain occurs by recombination of V, D, and J gene segments. The intersection of these specific regions (V and J for the alpha or gamma chain, or V, D and J for the beta or delta chain) corresponds to the CDR3 region that is important for antigen-MHC recognition. Complementarity determining regions (e.g., CDR1, CDR2, and CDR3), or hypervariable regions, are sequences in the variable domains of antigen receptors (e.g., T cell receptor and immunoglobulin) that can complement an antigen. Most of the diversity of CDRs is found in CDR3, with the diversity being generated by somatic recombination events during the development of T lymphocytes. A unique nucleotide sequence that arises during the gene arrangement process can be referred to as a clonotype.

The B cell receptor, or BCR, is a molecule found on the surface of B cells. The antigen binding portion of a BCR is composed of a membrane-bound antibody that, like most antibodies (e.g., immunoglobulins), has a unique and randomly determined antigen-binding site. The antigen binding portion of a BCR includes membrane-bound immunoglobulin molecule of one isotype (e.g., IgD, IgM, IgA, IgG, or IgE). When a B cell is activated by its first encounter with a cognate antigen, the cell proliferates and differentiates to generate a population of antibody-secreting plasma B cells and memory B cells. The various immunoglobulin isotypes differ in their biological features, structure, target specificity and distribution. A variety of molecular mechanisms exist to generate initial diversity, including genetic recombination at multiple sites.

The BCR is composed of two genes IgH and IgK (or IgL) coding for antibody heavy and light chains. Immunoglobulins are formed by recombination among gene segments, sequence diversification at the junctions of these segments, and point mutations throughout the gene. Each heavy chain gene contains multiple copies of three different gene segments - a variable 'V' gene segment, a diversity 'D' gene segment, and a joining 'J' gene segment. Each light chain gene contains multiple copies of two different gene segments for the variable region of the protein - a variable 'V' gene segment and a joining 'J' gene segment. The recombination can generate a molecule with one of each of the V, D, and J segments. Furthermore, several bases may be deleted and others added (called N and P nucleotides) at each of the two junctions, thereby generating further diversity. After B cell activation, a process of affinity maturation through somatic hypermutation occurs. In this process progeny cells of the activated B cells accumulate distinct somatic mutations throughout the gene with higher mutation concentration in the CDR regions leading to the generation of antibodies with higher affinity to the antigens. In addition to somatic hypermutation activated B cells undergo the process of isotype switching. Antibodies with the same variable segments can have different forms (isotypes) depending on the constant segment. Whereas all naive B cells express IgM (or IgD), activated B cells mostly express IgG but also IgM, IgA and IgE. This expression switching from IgM (and/or IgD) to IgG, IgA, or IgE occurs through a recombination event causing one cell to specialize in producing a specific isotype. A unique nucleotide sequence that arises during the gene arrangement process can similarly be referred to as a clonotype.

In some embodiments, the methods, compositions and systems disclosed herein are utilized to analyze the various sequences of TCRs and BCRs from immune cells, for example various clonotypes. In some embodiments, methods, compositions and systems disclosed herein are used to analyze the sequence of a TCR alpha chain, a TCR beta chain, a TCR delta chain, a TCR gamma chain, or any fragment thereof (e.g., variable regions including VDJ or VJ regions, constant regions, transmembrane regions, fragments thereof, combinations thereof, and combinations of fragments thereof). In some embodiments, methods, compositions and systems disclosed herein are used to analyze the sequence of a B cell receptor heavy chain, B cell receptor light chain, or any fragment thereof (e.g., variable regions including VDJ or VJ regions, constant regions, transmembrane regions, fragments thereof, combinations thereof, and combinations of fragments thereof).

Where immune cells are to be analyzed, primer sequences useful in any of the various operations for attaching barcode sequences and/or amplification reactions may comprise gene specific sequences which target genes or regions of genes of immune cell proteins, for example immune receptors. Such gene sequences include, but are not limited to, sequences of various T cell receptor alpha variable genes (TRAV genes), T cell receptor alpha joining genes (TRAJ genes), T cell receptor alpha constant genes (TRAC genes), T cell receptor beta variable genes (TRBV genes), T cell receptor beta diversity genes (TRBD genes), T cell receptor beta joining genes (TRBJ genes), T cell receptor beta constant genes (TRBC genes), T cell receptor gamma variable genes (TRGV genes), T cell receptor gamma joining genes (TRGJ genes), T cell receptor gamma constant genes (TRGC genes), T cell receptor delta variable genes (TRDV genes), T cell receptor delta diversity genes (TRDD genes), T cell receptor delta joining genes (TRDJ genes), and T cell receptor delta constant genes (TRDC genes).

The ability to characterize individual cells from larger diverse populations of cells is also of significant value in both environmental testing as well as in forensic analysis, where samples may, by their nature, be made up of diverse populations of cells and other material that "contaminate" the sample, relative to the cells for which the sample is being tested, e.g., environmental indicator organisms, toxic organisms, and the like for, e.g., environmental and food safety testing, victim and/or perpetrator cells in forensic analysis for sexual assault, and other violent crimes, and the like.

Additionally the methods and compositions disclosed herein, allow the determination of not only the immune repertoire and different clonotypes, but the functional characteristics (e.g., the transcriptome) of the cells associated with a clonotype or plurality of clonotypes that bind to the same or similar antigen. These functional characteristics can comprise transcription of cytokine, chemokine, or cell-surface associated molecules, such as, costimulatory molecules, checkpoint inhibitors, cell surface maturation markers, or cell-adhesion molecules. Such analysis allows a cell or cell population expressing a particular T cell receptor or B cell receptor to be associated with certain functional characteristics. For example, for any given antigen there will be multiple clonotypes of T cell receptor or B cell receptor that specifically bind to that antigen. Multiple clonotypes that bind to the same antigen are known as the idiotype.

Additional useful applications of the above described single cell sequencing and characterization processes are in the field of neuroscience research and diagnosis. In particular, neural cells can include long interspersed nuclear elements (LINEs), or 'jumping' genes that can move around the genome, which cause each neuron to differ from its neighbor cells. Research has shown that the number of LINEs in human brain exceeds that of other tissues, e.g., heart and liver tissue, with between 80 and 300 unique insertions (See, e.g., Coufal, N. G. et al. Nature 460, 1127-1131 (2009)). These differences have been postulated as being related to a person's susceptibility to neuro-logical disorders (see, e.g., Muotri, A. R. et al. Nature 468, 443-446 (2010)), or provide the brain with a diversity with which to respond to challenges. As such, the methods described herein may be used in the sequencing and characterization of individual neural cells.

The single cell analysis methods described herein are also useful in the analysis of gene expression, as noted above, both in terms of identification of RNA transcripts and their quantitation. In particular, using the single cell level analysis methods described herein, one can isolate and analyze the RNA transcripts present in individual cells, populations of cells, or subsets of populations of cells. In particular, in some cases, the barcode oligonucleotides may be configured to prime, replicate and consequently yield barcoded fragments of RNA from individual cells. For example, in some cases, the barcode oligonucleotides may include mRNA specific priming sequences, e.g., poly-T primer segments that allow priming and replication of mRNA in a reverse transcription reaction or other targeted priming sequences. Alternatively or additionally, random RNA priming may be carried out using random N-mer primer segments of the barcode oligonucleotides.

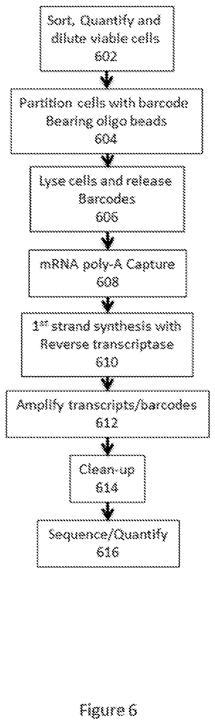

Figure 6 provides a schematic of one example method for RNA expression analysis in individual cells using the methods described herein. As shown, at operation 602 a cell containing sample is sorted for viable cells, which are quantified and diluted for subsequent partitioning. At operation 604, the individual cells separately co-partitioned with gel beads bearing the barcoding oligonucleotides as described herein. The cells are lysed and the barcoded oligonucleotides released into the partitions at operation 606, where they interact with and hybridize to the mRNA at operation 608, e.g., by virtue of a poly-T primer sequence, which is complementary to the poly-A tail of the mRNA. Using the poly-T barcode oligonucleotide as a priming sequence, a reverse transcription reaction is carried out at operation 610 to synthesize a cDNA transcript of the mRNA that includes the barcode sequence. The barcoded cDNA transcripts are then subjected to additional amplification at operation 612, e.g., using a polymerase chain reaction (PCR) process, purification at operation 614, before they are placed on a nucleic acid sequencing system for determination of the cDNA sequence and its associated barcode sequence(s). In some cases, as shown, operations 602 through 608 can occur while the reagents remain in their original droplet or partition, while operations 612 through 616 can occur in bulk (e.g., outside of the partition). In the case where a partition is a droplet in an emulsion, the emulsion can be broken and the contents of the droplet pooled in order to complete operations 612 through 616. In some cases, barcode oligonucleotides may be digested with exonucleases after the emulsion is broken. Exonuclease activity can be inhibited by ethylenediaminetetraacetic acid (EDTA) following primer digestion. In some cases, operation 610 may be performed either within the partitions based upon co-partitioning of the reverse transcription mixture, e.g., reverse transcriptase and associated reagents, or it may be performed in bulk.

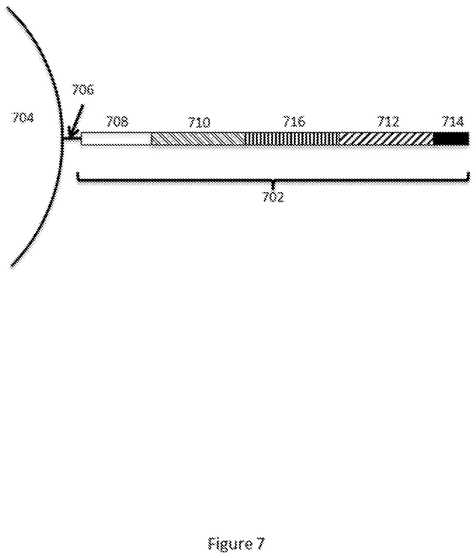

As noted elsewhere herein, the structure of the barcode oligonucleotides may include a number of sequence elements in addition to the oligonucleotide barcode sequence. One example of a barcode oligonucleotide for use in RNA analysis as described above is shown in Figure 7. As shown, the overall oligonucleotide 702 is coupled to a bead 704 by a releasable linkage 706, such as a disulfide linker. The oligonucleotide may include functional sequences that are used in subsequent processing, such as functional sequence 708, which may include one or more of a sequencer specific flow cell attachment sequence, e.g., a P5 sequence for Illumina sequencing systems, as well as sequencing primer sequences, e.g., a R1 primer for Illumina sequencing systems. A barcode sequence 710 is included within the structure for use in barcoding the sample RNA. An mRNA specific priming sequence, such as poly-T sequence 712 is also included in the oligonucleotide structure. An anchoring sequence segment 714 may be included to ensure that the poly-T sequence hybridizes at the sequence end of the mRNA. This anchoring sequence can include a random short sequence of nucleotides, e.g., 1-mer, 2-mer, 3-mer or longer sequence, which will ensure that the poly-T segment is more likely to hybridize at the sequence end of the poly-A tail of the mRNA. An additional sequence segment 716 may be provided within the oligonucleotide sequence. In some cases, this additional sequence provides a unique molecular identifier (UMI) sequence segment, e.g., as a random sequence (e.g., such as a random N-mer sequence) that varies across individual oligonucleotides coupled to a single bead, whereas barcode sequence 710 can be constant among oligonucleotides tethered to an individual bead. This unique sequence serves to provide a unique identifier of the starting mRNA molecule that was captured, in order to allow quantitation of the number of original expressed RNA. As will be appreciated, although shown as a single oligonucleotide tethered to the surface of a bead, individual bead can include tens to hundreds of thousands or even millions of individual oligonucleotide molecules, where, as noted, the barcode segment can be constant or relatively constant for a given bead, but where the variable or unique sequence segment will vary across an individual bead. This unique molecular identifier (UMI) sequence segment may include from 5 to about 8 or more nucleotides within the sequence of the oligonucleotides. In some cases, the unique molecular identifier (UMI) sequence segment can be 2, 3, 4, 5, 6, 7, 8, 9, 10, 11, 12, 13, 14, 15, 16, 17, 18, 19 or 20 nucleotides in length or longer. In some cases, the unique molecular identifier (UMI) sequence segment can be at least 2, 3, 4, 5, 6, 7, 8, 9, 10, 11, 12, 13, 14, 15, 16, 17, 18, 19 or 20 nucleotides in length or longer. In some cases, the unique molecular identifier (UMI) sequence segment can be at most 2, 3, 4, 5, 6, 7, 8, 9, 10, 11, 12, 13, 14, 15, 16, 17, 18, 19 or 20 nucleotides in length or shorter.

In operation, and with reference to Figures 6 and 7, a cell is co-partitioned along with a barcode bearing bead and lysed while the barcoded oligonucleotides are released from the bead. The poly-T portion of the released barcode oligonucleotide then hybridizes to the poly-A tail of the mRNA. The poly-T segment then primes the reverse transcription of the mRNA to produce a cDNA transcript of the mRNA, but which includes each of the sequence segments 708-716 of the barcode oligonucleotide. Again, because the oligonucleotide 702 includes an anchoring sequence 714, it will more likely hybridize to and prime reverse transcription at the sequence end of the poly-A tail of the mRNA. Within any given partition, all of the cDNA transcripts of the individual mRNA molecules will include a common barcode sequence segment 710. However, by including the unique random N-mer sequence, the transcripts made from different mRNA molecules within a given partition will vary at this unique sequence. This provides a quantitation feature that can be identifiable even following any subsequent amplification of the contents of a given partition, e.g., the number of unique segments associated with a common barcode can be indicative of the quantity of mRNA originating from a single partition, and thus, a single cell. As noted above, the transcripts are then amplified, cleaned up and sequenced to identify the sequence of the cDNA transcript of the mRNA, as well as to sequence the barcode segment and the unique sequence segment.

As noted elsewhere herein, while a poly-T primer sequence is described, other targeted or random priming sequences may also be used in priming the reverse transcription reaction. In some cases, the primer sequence can be a gene specific primer sequence which targets specific genes for reverse transcription. The target genes comprise T cell receptor genes and B cell receptor genes. Likewise, although described as releasing the barcoded oligonucleotides into the partition along with the contents of the lysed cells, it will be appreciated that in some cases, the gel bead bound oligonucleotides may be used to hybridize and capture the mRNA on the solid phase of the gel beads, in order to facilitate the separation of the RNA from other cell contents.

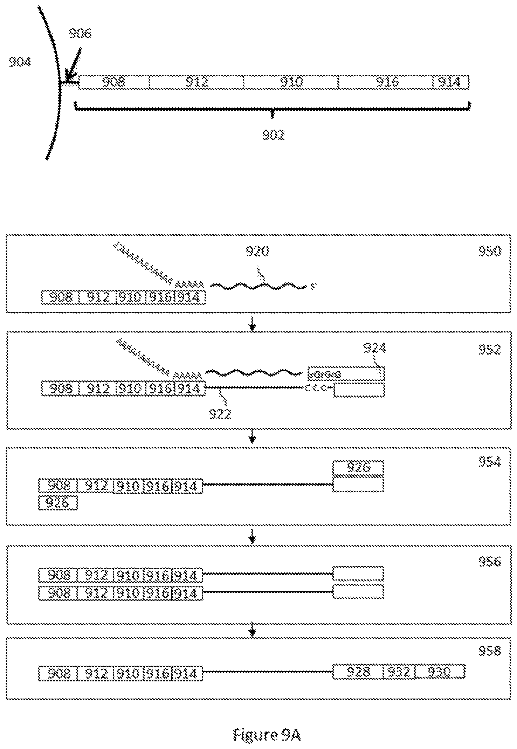

An additional example of a barcode oligonucleotide for use in RNA analysis, including messenger RNA (mRNA, including mRNA obtained from a cell) analysis, is shown in Figure 9A. As shown, the overall oligonucleotide 902 can be coupled to a bead 904 by a releasable linkage 906, such as a disulfide linker. The oligonucleotide may include functional sequences that are used in subsequent processing, such as functional sequence 908, which may include a sequencer specific flow cell attachment sequence, e.g., a P5 sequence for Illumina sequencing systems, as well as functional sequence 910, which may include sequencing primer sequences, e.g., a R1 primer binding site for Illumina sequencing systems. A barcode sequence 912 is included within the structure for use in barcoding the sample RNA. An RNA specific (e.g., mRNA specific) priming sequence, such as poly-T sequence 914 is also included in the oligonucleotide structure. An anchoring sequence segment (not shown) may be included to ensure that the poly-T sequence hybridizes at the sequence end of the mRNA. An additional sequence segment 916 may be provided within the oligonucleotide sequence. This additional sequence can provide a unique molecular identifier (UMI) sequence segment, e.g., as a random N-mer sequence that varies across individual oligonucleotides coupled to a single bead, whereas barcode sequence 912 can be constant among oligonucleotides tethered to an individual bead. As described elsewhere herein, this unique sequence can serve to provide a unique identifier of the starting mRNA molecule that was captured, in order to allow quantitation of the number of original expressed RNA, e.g., mRNA counting. As will be appreciated, although shown as a single oligonucleotide tethered to the surface of a bead, individual beads can include tens to hundreds of thousands or even millions of individual oligonucleotide molecules, where, as noted, the barcode segment can be constant or relatively constant for a given bead, but where the variable or unique sequence segment will vary across an individual bead.

In an example method of cellular RNA (e.g., mRNA) analysis and in reference to Figure 9A, a cell is co-partitioned along with a barcode bearing bead, switch oligo 924, and other reagents such as reverse transcriptase, a reducing agent and dNTPs into a partition (e.g., a droplet in an emulsion). In operation 950, the cell is lysed while the barcoded oligonucleotides 902 are released from the bead (e.g., via the action of the reducing agent) and the poly-T segment 914 of the released barcode oligonucleotide then hybridizes to the poly-A tail of mRNA 920 that is released from the cell. Next, in operation 952 the poly-T segment 914 is extended in a reverse transcription reaction using the mRNA as a template to produce a cDNA transcript 922 complementary to the mRNA and also includes each of the sequence segments 908, 912, 910, 916 and 914 of the barcode oligonucleotide. Terminal transferase activity of the reverse transcriptase can add additional bases to the cDNA transcript (e.g., polyC). The switch oligo 924 may then hybridize with the additional bases added to the cDNA transcript and facilitate template switching. A sequence complementary to the switch oligo sequence can then be incorporated into the cDNA transcript 922 via extension of the cDNA transcript 922 using the switch oligo 924 as a template. Within any given partition, all of the cDNA transcripts of the individual mRNA molecules will include a common barcode sequence segment 912. However, by including the unique random N-mer sequence 916, the transcripts made from different mRNA molecules within a given partition will vary at this unique sequence. As described elsewhere herein, this provides a quantitation feature that can be identifiable even following any subsequent amplification of the contents of a given partition, e.g., the number of unique segments associated with a common barcode can be indicative of the quantity of mRNA originating from a single partition, and thus, a single cell. Following operation 952, the cDNA transcript 922 is then amplified with primers 926 (e.g., PCR primers) in operation 954. Next, the amplified product is then purified (e.g., via solid phase reversible immobilization (SPRI)) in operation 956. At operation 958, the amplified product is then sheared, ligated to additional functional sequences, and further amplified (e.g., via PCR). The functional sequences may include a sequencer specific flow cell attachment sequence 930, e.g., a P7 sequence for Illumina sequencing systems, as well as functional sequence 928, which may include a sequencing primer binding site, e.g., for a R2 primer for Illumina sequencing systems, as well as functional sequence 932, which may include a sample index, e.g., an i7 sample index sequence for Illumina sequencing systems. In some cases, operations 950 and 952 can occur in the partition, while operations 954, 956 and 958 can occur in bulk solution (e.g., in a pooled mixture outside of the partition). In the case where a partition is a droplet in an emulsion, the emulsion can be broken and the contents of the droplet pooled in order to complete operations 954, 956 and 958. In some cases, operation 954 may be completed in the partition. In some cases, barcode oligonucleotides may be digested with exonucleases after the emulsion is broken. Exonuclease activity can be inhibited by ethylenediaminetetraacetic acid (EDTA) following primer digestion. Although described in terms of specific sequence references used for certain sequencing systems, e.g., Illumina systems, it will be understood that the reference to these sequences is for illustration purposes only, and the methods described herein may be configured for use with other sequencing systems incorporating specific priming, attachment, index, and other operational sequences used in those systems, e.g., systems available from Ion Torrent, Oxford Nanopore, Genia, Pacific Biosciences, Complete Genomics, and the like.

In an alternative example of a barcode oligonucleotide for use in RNA (e.g., cellular RNA) analysis as shown in Figure 9A, functional sequence 908 may be a P7 sequence and functional sequence 910 may be a R2 primer binding site. Moreover, the functional sequence 930 may be a P5 sequence, functional sequence 928 may be a R1 primer binding site, and functional sequence 932 may be an i5 sample index sequence for Illumina sequencing systems. The configuration of the constructs generated by such a barcode oligonucleotide can help minimize (or avoid) sequencing of the poly-T sequence during sequencing.

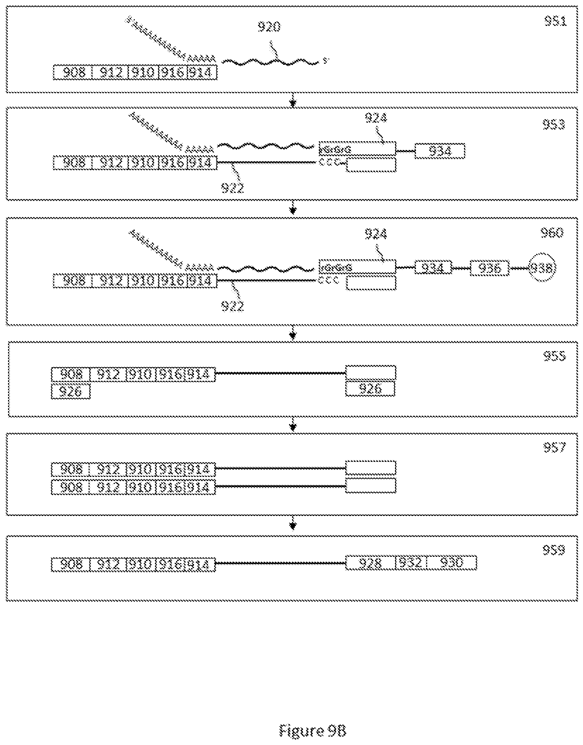

Shown in Figure 9B is another example method for RNA analysis, including cellular mRNA analysis. In this method, the switch oligo 924 is co-partitioned with the individual cell and barcoded bead along with reagents such as reverse transcriptase, a reducing agent and dNTPs into a partition (e.g., a droplet in an emulsion). The switch oligo 924 may be labeled with an additional tag 934, e.g., biotin. In operation 951, the cell is lysed while the barcoded oligonucleotides 902 (e.g., as shown in Figure 9A) are released from the bead (e.g., via the action of the reducing agent). In some cases, sequence 908 is a P7 sequence and sequence 910 is a R2 primer binding site. In other cases, sequence 908 is a P5 sequence and sequence 910 is a R1 primer binding site. Next, the poly-T segment 914 of the released barcode oligonucleotide hybridizes to the poly-A tail of mRNA 920 that is released from the cell. In operation 953, the poly-T segment 914 is then extended in a reverse transcription reaction using the mRNA as a template to produce a cDNA transcript 922 complementary to the mRNA and also includes each of the sequence segments 908, 912, 910, 916 and 914 of the barcode oligonucleotide. Terminal transferase activity of the reverse transcriptase can add additional bases to the cDNA transcript (e.g., polyC). The switch oligo 924 may then hybridize with the cDNA transcript and facilitate template switching. A sequence complementary to the switch oligo sequence can then be incorporated into the cDNA transcript 922 via extension of the cDNA transcript 922 using the switch oligo 924 as a template. Next, an isolation operation 960 can be used to isolate the cDNA transcript 922 from the reagents and oligonucleotides in the partition. The additional tag 934, e.g., biotin, can be contacted with an interacting tag 936, e.g., streptavidin, which may be attached to a magnetic bead 938. At operation 960 the cDNA can be isolated with a pull-down operation (e.g., via magnetic separation, centrifugation) before amplification (e.g., via PCR) in operation 955, followed by purification (e.g., via solid phase reversible immobilization (SPRI)) in operation 957 and further processing (shearing, ligation of sequences 928, 932 and 930 and subsequent amplification (e.g., via PCR)) in operation 959. In some cases where sequence 908 is a P7 sequence and sequence 910 is a R2 primer binding site, sequence 930 is a P5 sequence and sequence 928 is a R1 primer binding site and sequence 932 is an i5 sample index sequence. In some cases where sequence 908 is a P5 sequence and sequence 910 is a R1 primer binding site, sequence 930 is a P7 sequence and sequence 928 is a R2 primer binding site and sequence 932 is an i7 sample index sequence. In some cases, as shown, operations 951 and 953 can occur in the partition, while operations 960, 955, 957 and 959 can occur in bulk solution (e.g., in a pooled mixture outside of the partition). In the case where a partition is a droplet in an emulsion, the emulsion can be broken and the contents of the droplet pooled in order to complete operation 960. The operations 955, 957, and 959 can then be carried out following operation 960 after the transcripts are pooled for processing.

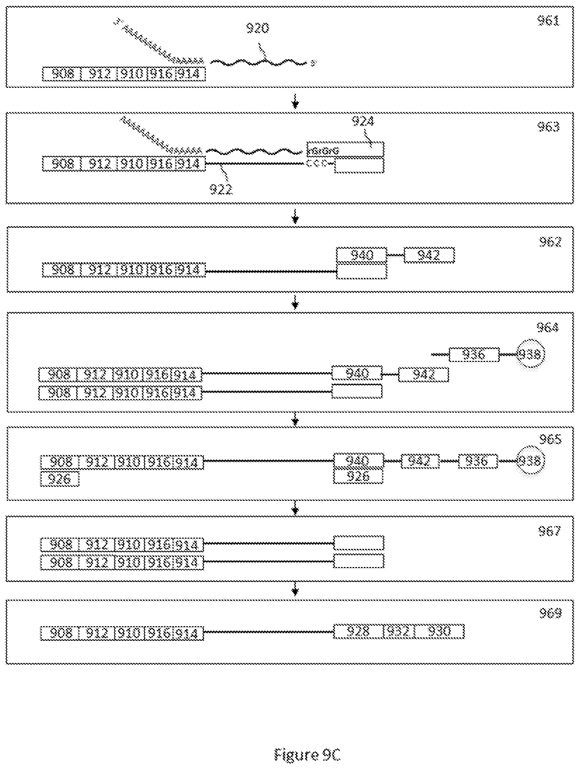

Shown in Figure 9C is another example method for RNA analysis, including cellular mRNA analysis. In this method, the switch oligo 924 is co-partitioned with the individual cell and barcoded bead along with reagents such as reverse transcriptase, a reducing agent and dNTPs in a partition (e.g., a droplet in an emulsion). In operation 961, the cell is lysed while the barcoded oligonucleotides 902 (e.g., as shown in Figure 9A) are released from the bead (e.g., via the action of the reducing agent). In some cases, sequence 908 is a P7 sequence and sequence 910 is a R2 primer binding site. In other cases, sequence 908 is a P5 sequence and sequence 910 is a R1 primer binding site. Next, the poly-T segment 914 of the released barcode oligonucleotide then hybridizes to the poly-A tail of mRNA 920 that is released from the cell. Next, in operation 963 the poly-T segment 914 is then extended in a reverse transcription reaction using the mRNA as a template to produce a cDNA transcript 922 complementary to the mRNA and also includes each of the sequence segments 908, 912, 910, 916 and 914 of the barcode oligonucleotide. Terminal transferase activity of the reverse transcriptase can add additional bases to the cDNA transcript (e.g., polyC). The switch oligo 924 may then hybridize with the cDNA transcript and facilitate template switching. A sequence complementary to the switch oligo sequence can then be incorporated into the cDNA transcript 922 via extension of the cDNA transcript 922 using the switch oligo 924 as a template. Following operation 961 and operation 963, mRNA 920 and cDNA transcript 922 are denatured in operation 962. At operation 964, a second strand is extended from a primer 940 having an additional tag 942, e.g., biotin, and hybridized to the cDNA transcript 922. Also in operation 964, the biotin labeled second strand can be contacted with an interacting tag 936, e.g., streptavidin, which may be attached to a magnetic bead 938. The cDNA can be isolated with a pull-down operation (e.g., via magnetic separation, centrifugation) before amplification (e.g., via polymerase chain reaction (PCR)) in operation 965, followed by purification (e.g., via solid phase reversible immobilization (SPRI)) in operation 967 and further processing (shearing, ligation of sequences 928, 932 and 930 and subsequent amplification (e.g., via PCR)) in operation 969. In some cases where sequence 908 is a P7 sequence and sequence 910 is a R2 primer binding site, sequence 930 is a P5 sequence and sequence 928 is a R1 primer binding site and sequence 932 is an i5 sample index sequence. In some cases where sequence 908 is a P5 sequence and sequence 910 is a R1 primer binding site, sequence 930 is a P7 sequence and sequence 928 is a R2 primer binding site and sequence 932 is an i7 sample index sequence. In some cases, operations 961 and 963 can occur in the partition, while operations 962, 964, 965, 967, and 969 can occur in bulk (e.g., outside the partition). In the case where a partition is a droplet in an emulsion, the emulsion can be broken and the contents of the droplet pooled in order to complete operations 962, 964, 965, 967 and 969.

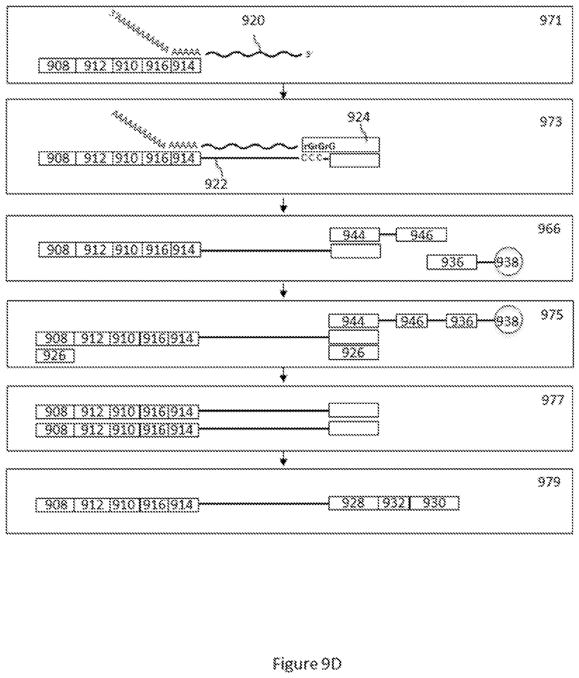

Shown in Figure 9D is another example method for RNA analysis, including cellular mRNA analysis. In this method, the switch oligo 924 is co-partitioned with the individual cell and barcoded bead along with reagents such as reverse transcriptase, a reducing agent and dNTPs. In operation 971, the cell is lysed while the barcoded oligonucleotides 902 (e.g., as shown in Figure 9A) are released from the bead (e.g., via the action of the reducing agent). In some cases, sequence 908 is a P7 sequence and sequence 910 is a R2 primer binding site. In other cases, sequence 908 is a P5 sequence and sequence 910 is a R1 primer binding site. Next the poly-T segment 914 of the released barcode oligonucleotide then hybridizes to the poly-A tail of mRNA 920 that is released from the cell. Next in operation 973, the poly-T segment 914 is then extended in a reverse transcription reaction using the mRNA as a template to produce a cDNA transcript 922 complementary to the mRNA and also includes each of the sequence segments 908, 912, 910, 916 and 914 of the barcode oligonucleotide. Terminal transferase activity of the reverse transcriptase can add additional bases to the cDNA transcript (e.g., polyC). The switch oligo 924 may then hybridize with the cDNA transcript and facilitate template switching. A sequence complementary to the switch oligo sequence can then be incorporated into the cDNA transcript 922 via extension of the cDNA transcript 922 using the switch oligo 924 as a template. In operation 966, the mRNA 920, cDNA transcript 922 and switch oligo 924 can be denatured, and the cDNA transcript 922 can be hybridized with a capture oligonucleotide 944 labeled with an additional tag 946, e.g., biotin. In this operation, the biotin-labeled capture oligonucleotide 944, which is hybridized to the cDNA transcript, can be contacted with an interacting tag 936, e.g., streptavidin, which may be attached to a magnetic bead 938. Following separation from other species (e.g., excess barcoded oligonucleotides) using a pull-down operation (e.g., via magnetic separation, centrifugation), the cDNA transcript can be amplified (e.g., via PCR) with primers 926 at operation 975, followed by purification (e.g., via solid phase reversible immobilization (SPRI)) in operation 977 and further processing (shearing, ligation of sequences 928, 932 and 930 and subsequent amplification (e.g., via PCR)) in operation 979. In some cases where sequence 908 is a P7 sequence and sequence 910 is a R2 primer binding site, sequence 930 is a P5 sequence and sequence 928 is a R1 primer binding site and sequence 932 is an i5 sample index sequence. In other cases where sequence 908 is a P5 sequence and sequence 910 is a R1 primer binding site, sequence 930 is a P7 sequence and sequence 928 is a R2 primer binding site and sequence 932 is an i7 sample index sequence. In some cases, operations 971 and 973 can occur in the partition, while operations 966, 975, 977 (purification), and 979 can occur in bulk (e.g., outside the partition). In the case where a partition is a droplet in an emulsion, the emulsion can be broken and the contents of the droplet pooled in order to complete operations 966, 975, 977 and 979.

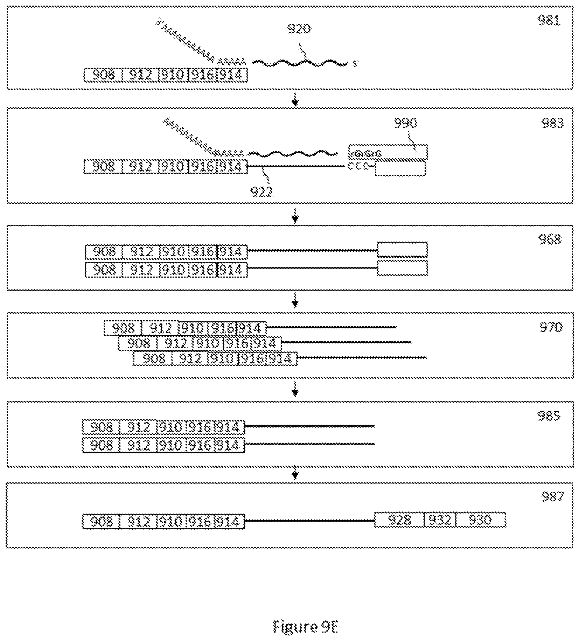

Shown in Figure 9E is another example method for RNA analysis, including cellular RNA analysis. In this method, an individual cell is co-partitioned along with a barcode bearing bead, a switch oligo 990, and other reagents such as reverse transcriptase, a reducing agent and dNTPs into a partition (e.g., a droplet in an emulsion). In operation 981, the cell is lysed while the barcoded oligonucleotides (e.g., 902 as shown in Figure 9A) are released from the bead (e.g., via the action of the reducing agent). In some cases, sequence 908 is a P7 sequence and sequence 910 is a R2 primer binding site. In other cases, sequence 908 is a P5 sequence and sequence 910 is a R1 primer binding site. Next, the poly-T segment of the released barcode oligonucleotide then hybridizes to the poly-A tail of mRNA 920 released from the cell. Next at operation 983, the poly-T segment is then extended in a reverse transcription reaction to produce a cDNA transcript 922 complementary to the mRNA and also includes each of the sequence segments 908, 912, 910, 916 and 914 of the barcode oligonucleotide. Terminal transferase activity of the reverse transcriptase can add additional bases to the cDNA transcript (e.g., polyC).The switch oligo 990 may then hybridize with the cDNA transcript and facilitate template switching. A sequence complementary to the switch oligo sequence and including a T7 promoter sequence, can be incorporated into the cDNA transcript 922. At operation 968, a second strand is synthesized and at operation 970 the T7 promoter sequence can be used by T7 polymerase to produce RNA transcripts in in vitro transcription. At operation 985 the RNA transcripts can be purified (e.g., via solid phase reversible immobilization (SPRI)), reverse transcribed to form DNA transcripts, and a second strand can be synthesized for each of the DNA transcripts. In some cases, prior to purification, the RNA transcripts can be contacted with a DNase (e.g., DNAase I) to break down residual DNA. At operation 987 the DNA transcripts are then fragmented and ligated to additional functional sequences, such as sequences 928, 932 and 930 and, in some cases, further amplified (e.g., via PCR). In some cases where sequence 908 is a P7 sequence and sequence 910 is a R2 primer binding site, sequence 930 is a P5 sequence and sequence 928 is a R1 primer binding site and sequence 932 is an i5 sample index sequence. In some cases where sequence 908 is a P5 sequence and sequence 910 is a R1 primer binding site, sequence 930 is a P7 sequence and sequence 928 is a R2 primer binding site and sequence 932 is an i7 sample index sequence. In some cases, prior to removing a portion of the DNA transcripts, the DNA transcripts can be contacted with an RNase to break down residual RNA. In some cases, operations 981 and 983 can occur in the partition, while operations 968, 970, 985 and 987 can occur in bulk (e.g., outside the partition). In the case where a partition is a droplet in an emulsion, the emulsion can be broken and the contents of the droplet pooled in order to complete operations 968, 970, 985 and 987.

The approaches of Figures 9A-9E may be employed for use with various target regions. The target regions are TCR and/or BCR regions. In such examples, oligonucleotides coupled to beads may include primers with sequences that are targeted for such target regions (e.g., constant regions). For example, polyT primer regions can instead be gene specific primer sequences.

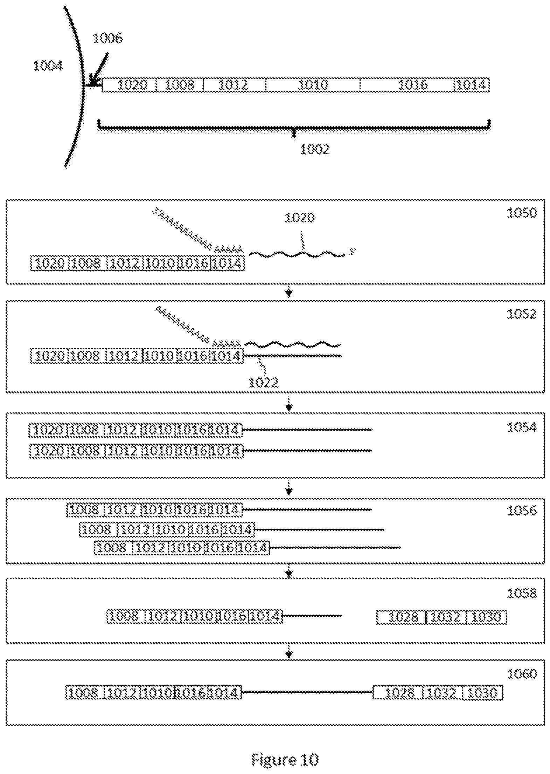

Another example of a barcode oligonucleotide for use in RNA analysis, including messenger RNA (mRNA, including mRNA obtained from a cell) analysis is shown in Figure 10. As shown, the overall oligonucleotide 1002 is coupled to a bead 1004 by a releasable linkage 1006, such as a disulfide linker. The oligonucleotide may include functional sequences that are used in subsequent processing, such as functional sequence 1008, which may include a sequencer specific flow cell attachment sequence, e.g., a P7 sequence, as well as functional sequence 1010, which may include sequencing primer sequences, e.g., a R2 primer binding site. A barcode sequence 1012 is included within the structure for use in barcoding the sample RNA. An RNA specific (e.g., mRNA specific) priming sequence, such as poly-T sequence 1014 may be included in the oligonucleotide structure. An anchoring sequence segment (not shown) may be included to ensure that the poly-T sequence hybridizes at the sequence end of the mRNA. An additional sequence segment 1016 may be provided within the oligonucleotide sequence. This additional sequence can provide a unique molecular identifier (UMI) sequence segment, as described elsewhere herein. An additional functional sequence 1020 may be included for in vitro transcription, e.g., a T7 RNA polymerase promoter sequence. As will be appreciated, although shown as a single oligonucleotide tethered to the surface of a bead, individual beads can include tens to hundreds of thousands or even millions of individual oligonucleotide molecules, where, as noted, the barcode segment can be constant or relatively constant for a given bead, but where the variable or unique sequence segment will vary across an individual bead.

In an example method of cellular RNA analysis and in reference to Figure 10, a cell is co-partitioned along with a barcode bearing bead, and other reagents such as reverse transcriptase, reducing agent and dNTPs into a partition (e.g., a droplet in an emulsion). In operation 1050, the cell is lysed while the barcoded oligonucleotides 1002 are released (e.g., via the action of the reducing agent) from the bead, and the poly-T segment 1014 of the released barcode oligonucleotide then hybridizes to the poly-A tail of mRNA 1020. Next at operation 1052, the poly-T segment is then extended in a reverse transcription reaction using the mRNA as template to produce a cDNA transcript 1022 of the mRNA and also includes each of the sequence segments 1020, 1008, 1012, 1010, 1016, and 1014 of the barcode oligonucleotide. Within any given partition, all of the cDNA transcripts of the individual mRNA molecules will include a common barcode sequence segment 1012. However, by including the unique random N-mer sequence, the transcripts made from different mRNA molecules within a given partition will vary at this unique sequence. As described elsewhere herein, this provides a quantitation feature that can be identifiable even following any subsequent amplification of the contents of a given partition, e.g., the number of unique segments associated with a common barcode can be indicative of the quantity of mRNA originating from a single partition, and thus, a single cell. At operation 1054 a second strand is synthesized and at operation 1056 the T7 promoter sequence can be used by T7 polymerase to produce RNA transcripts in in vitro transcription. At operation 1058 the transcripts are fragmented (e.g., sheared), ligated to additional functional sequences, and reverse transcribed. The functional sequences may include a sequencer specific flow cell attachment sequence 1030, e.g., a P5 sequence, as well as functional sequence 1028, which may include sequencing primers, e.g., a R1 primer binding sequence, as well as functional sequence 1032, which may include a sample index, e.g., an i5 sample index sequence. At operation 1060 the RNA transcripts can be reverse transcribed to DNA, the DNA amplified (e.g., via PCR), and sequenced to identify the sequence of the cDNA transcript of the mRNA, as well as to sequence the barcode segment and the unique sequence segment. In some cases, operations 1050 and 1052 can occur in the partition, while operations 1054, 1056, 1058 and 1060 can occur in bulk (e.g., outside the partition). In the case where a partition is a droplet in an emulsion, the emulsion can be broken and the contents of the droplet pooled in order to complete operations 1054, 1056, 1058 and 1060.

In an alternative example of a barcode oligonucleotide for use in RNA (e.g., cellular RNA) analysis as shown in Figure 10, functional sequence 1008 may be a P5 sequence and functional sequence 1010 may be a R1 primer binding site. Moreover, the functional sequence 1030 may be a P7 sequence, functional sequence 1028 may be a R2 primer binding site, and functional sequence 1032 may be an i7 sample index sequence.

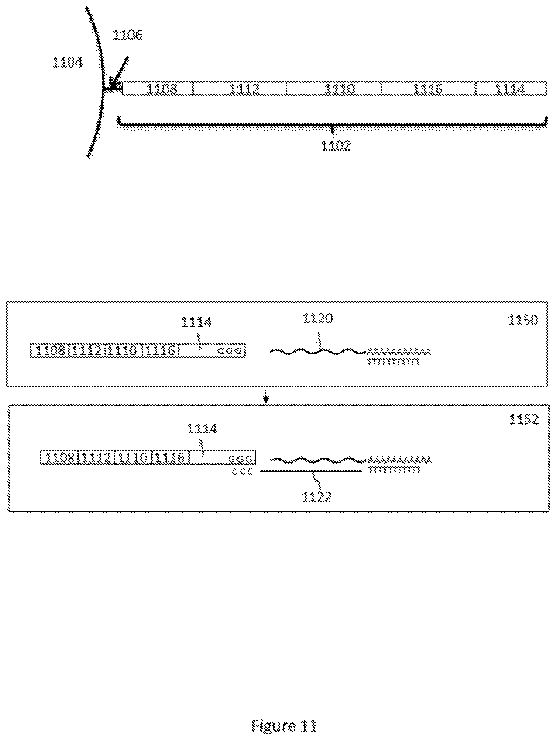

An additional example of a barcode oligonucleotide for use in RNA analysis, including messenger RNA (mRNA, including mRNA obtained from a cell) analysis is shown in Figure 11. As shown, the overall oligonucleotide 1102 is coupled to a bead 1104 by a releasable linkage 1106, such as a disulfide linker. The oligonucleotide may include functional sequences that are used in subsequent processing, such as functional sequence 1108, which may include a sequencer specific flow cell attachment sequence, e.g., a P5 sequence, as well as functional sequence 1110, which may include sequencing primer sequences, e.g., a R1 primer binding site. In some cases, sequence 1108 is a P7 sequence and sequence 1110 is a R2 primer binding site. A barcode sequence 1112 is included within the structure for use in barcoding the sample RNA. An additional sequence segment 1116 may be provided within the oligonucleotide sequence. In some cases, this additional sequence can provide a unique molecular identifier (UMI) sequence segment, as described elsewhere herein. An additional sequence 1114 may be included to facilitate template switching, e.g., polyG. As will be appreciated, although shown as a single oligonucleotide tethered to the surface of a bead, individual beads can include tens to hundreds of thousands or even millions of individual oligonucleotide molecules, where, as noted, the barcode segment can be constant or relatively constant for a given bead, but where the variable or unique sequence segment will vary across an individual bead.

In an example method of cellular mRNA analysis and in reference to Figure 11, a cell is co-partitioned along with a microcapsule (e.g., bead bearing a barcoded oligonucleotide), polyT sequence, and other reagents such as a DNA polymerase, a reverse transcriptase, oligonucleotide primers, dNTPs, and reducing agent into a partition (e.g., a droplet in an emulsion). The partition can serve as a reaction volume. As described elsewhere herein, the partition serving as the reaction volume can comprise a container or vessel such as a well, a microwell, vial, a tube, through ports in nanoarray substrates, or micro-vesicles having an outer barrier surrounding an inner fluid center or core, emulsion, or a droplet. In some embodiments, the partition comprises a droplet of aqueous fluid within a non-aqueous continuous phase, e.g., an oil phase. Within the partition, the cell can be lysed and the barcoded oligonucleotides can be released from the bead (e.g., via the action of the reducing agent or other stimulus). Cell lysis and release of the barcoded oligonucleotides from the microcapsule may occur simultaneously in the partition (e.g., a droplet in an emulsion) or the reaction volume. In some embodiments, cell lysis precedes release of the barcoded oligonucleotides from the microcapsule. In some embodiments, release of the barcoded oligonucleotides from the microcapsule precedes cell lysis.

Subsequent to cell lysis and the release of barcoded oligonucleotides from the microcapsule, the reaction volume can be subjected to an amplification reaction to generate an amplification product. In an example amplification reaction, the polyT sequence hybridizes to the polyA tail of mRNA 1120 released from the cell as illustrated in operation 1150. Next, in operation 1152, the polyT sequence is then extended in a reverse transcription reaction using the mRNA as a template to produce a cDNA transcript 1122 complementary to the mRNA. Terminal transferase activity of the reverse transcriptase can add additional bases to the cDNA transcript (e.g., polyC) in a template independent manner. The additional bases added to the cDNA transcript, e.g., polyC, can then hybridize with 1114 of the barcoded oligonucleotide. This can facilitate template switching and a sequence complementary to the barcoded oligonucleotide can be incorporated into the cDNA transcript. In various embodiments, the barcoded oligonucleotide does not hybridize to the template polynucleotide.

The barcoded oligonucleotide, upon release from the microcapsule, can be present in the reaction volume at any suitable concentration. In some embodiments, the barcoded oligonucleotide is present in the reaction volume at a concentration of about 0.2 $\mu$M, 0.3 $\mu$M, 0.4 $\mu$M, 0.5 $\mu$M, 1 $\mu$M, 5 $\mu$M, 10 $\mu$M, 15 $\mu$M, 20 $\mu$M, 25 $\mu$M, 30 $\mu$M, 35 $\mu$M, 40 $\mu$M, 50 $\mu$M, 100 $\mu$M, 150 $\mu$M, 200 $\mu$M, 250 $\mu$M, 300 $\mu$M, 400 $\mu$M, or 500 $\mu$M. In some embodiments, the barcoded oligonucleotide is present in the reaction volume at a concentration of at least about 0.2 $\mu$M, 0.3 $\mu$M, 0.4 $\mu$M, 0.5 $\mu$M, 1 $\mu$M, 5 $\mu$M, 10 $\mu$M, 15 $\mu$M, 20 $\mu$M, 25 $\mu$M, 30 $\mu$M, 35 $\mu$M, 40 $\mu$M, 50 $\mu$M, 100 $\mu$M, 150 $\mu$M, 200 $\mu$M, 250 $\mu$M, 300 $\mu$M, 400 $\mu$M, 500 $\mu$M or greater. In some embodiments, the barcoded oligonucleotide is present in the reaction volume at a concentration of at most about 0.2 $\mu$M, 0.3 $\mu$M, 0.4 $\mu$M, 0.5 $\mu$M, 1 $\mu$M, 5 $\mu$M, 10 $\mu$M, 15 $\mu$M, 20 $\mu$M, 25 $\mu$M, 30 $\mu$M, 35 $\mu$M, 40 $\mu$M, 50 $\mu$M, 100 $\mu$M, 150 $\mu$M, 200 $\mu$M, 250 $\mu$M, 300 $\mu$M, 400 $\mu$M, or 500 $\mu$M.

The transcripts can be further processed (e.g., amplified, portions removed, additional sequences added, etc.) and characterized as described elsewhere herein. In some embodiments, the transcripts are sequenced directly. In some embodiments, the transcripts are further processed (e.g., portions removed, additional sequences added, etc) and then sequenced. In some embodiments, the reaction volume is subjected to a second amplification reaction to generate an additional amplification product. The transcripts or first amplification products can be used as the template for the second amplification reaction. In some embodiments, primers for the second amplification reaction comprise the barcoded oligonucleotide and polyT sequence. In some embodiments, primers for the second amplification reaction comprise additional primers co-partitioned with the cell. In some embodiments, these additional amplification products are sequenced directly. In some embodiments, these additional amplification products are further processed (e.g., portions removed, additional sequences added, etc) and then sequenced. The configuration of the amplification products (e.g., first amplification products and second amplification products) generated by such a method can help minimize (or avoid) sequencing of the poly-T sequence during sequencing.

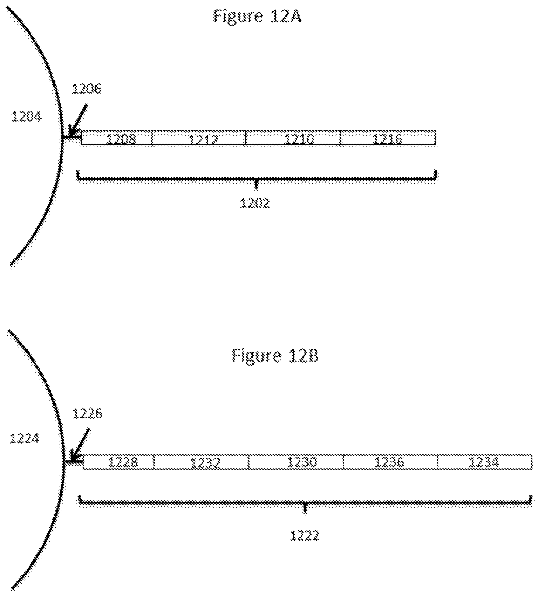

An additional example of a barcode oligonucleotide for use in RNA analysis, including cellular RNA analysis is shown in Figure 12A. As shown, the overall oligonucleotide 1202 is coupled to a bead 1204 by a releasable linkage 1206, such as a disulfide linker. The oligonucleotide may include functional sequences that are used in subsequent processing, such as functional sequence 1208, which may include a sequencer specific flow cell attachment sequence, e.g., a P5 sequence, as well as functional sequence 1210, which may include sequencing primer sequences, e.g., a R1 primer binding site. In some cases, sequence 1208 is a P7 sequence and sequence 1210 is a R2 primer binding site. A barcode sequence 1212 is included within the structure for use in barcoding the sample RNA. An additional sequence segment 1216 may be provided within the oligonucleotide sequence. In some cases, this additional sequence can provide a unique molecular identifier (UMI) sequence segment, as described elsewhere herein. As will be appreciated, although shown as a single oligonucleotide tethered to the surface of a bead, individual beads can include tens to hundreds of thousands or even millions of individual oligonucleotide molecules, where, as noted, the barcode segment can be constant or relatively constant for a given bead, but where the variable or unique sequence segment will vary across an individual bead. In an example method of cellular RNA analysis using this barcode, a cell is co-partitioned along with a barcode bearing bead and other reagents such as RNA ligase and a reducing agent into a partition (e.g., a droplet in an emulsion). The cell is lysed while the barcoded oligonucleotides are released (e.g., via the action of the reducing agent) from the bead. The barcoded oligonucleotides can then be ligated to the 5' end of mRNA transcripts while in the partitions by RNA ligase. Subsequent operations may include purification (e.g., via solid phase reversible immobilization (SPRI)) and further processing (shearing, ligation of functional sequences, and subsequent amplification (e.g., via PCR)), and these operations may occur in bulk (e.g., outside the partition). In the case where a partition is a droplet in an emulsion, the emulsion can be broken and the contents of the droplet pooled for the additional operations.

An additional example of a barcode oligonucleotide for use in RNA analysis, including cellular RNA analysis is shown in Figure 12B. As shown, the overall oligonucleotide 1222 is coupled to a bead 1224 by a releasable linkage 1226, such as a disulfide linker. The oligonucleotide may include functional sequences that are used in subsequent processing, such as functional sequence 1228, which may include a sequencer specific flow cell attachment sequence, e.g., a P5 sequence, as well as functional sequence 1230, which may include sequencing primer sequences, e.g., a R1 primer binding site. In some cases, sequence 1228 is a P7 sequence and sequence 1230 is a R2 primer binding site. A barcode sequence 1232 is included within the structure for use in barcoding the sample RNA. A priming sequence 1234 (e.g., a random priming sequence) can also be included in the oligonucleotide structure, e.g., a random hexamer. An additional sequence segment 1236 may be provided within the oligonucleotide sequence. In some cases, this additional sequence provides a unique molecular identifier (UMI) sequence segment, as described elsewhere herein. As will be appreciated, although shown as a single oligonucleotide tethered to the surface of a bead, individual beads can include tens to hundreds of thousands or even millions of individual oligonucleotide molecules, where, as noted, the barcode segment can be constant or relatively constant for a given bead, but where the variable or unique sequence segment will vary across an individual bead. In an example method of cellular mRNA analysis using the barcode oligonucleotide of Figure 12B, a cell is co-partitioned along with a barcode bearing bead and additional reagents such as reverse transcriptase, a reducing agent and dNTPs into a partition (e.g., a droplet in an emulsion). The cell is lysed while the barcoded oligonucleotides are released from the bead (e.g., via the action of the reducing agent). In some cases, sequence 1228 is a P7 sequence and sequence 1230 is a R2 primer binding site. In other cases, sequence 1228 is a P5 sequence and sequence 1230 is a R1 primer binding site. The priming sequence 1234 of random hexamers can randomly hybridize cellular mRNA. The random hexamer sequence can then be extended in a reverse transcription reaction using mRNA from the cell as a template to produce a cDNA transcript complementary to the mRNA and also includes each of the sequence segments 1228, 1232, 1230, 1236, and 1234 of the barcode oligonucleotide. Subsequent operations may include purification (e.g., via solid phase reversible immobilization (SPRI)), further processing (shearing, ligation of functional sequences, and subsequent amplification (e.g., via PCR)), and these operations may occur in bulk (e.g., outside the partition). In the case where a partition is a droplet in an emulsion, the emulsion can be broken and the contents of the droplet pooled for additional operations. Additional reagents that may be co-partitioned along with the barcode bearing bead may include oligonucleotides to block ribosomal RNA (rRNA) and nucleases to digest genomic DNA and cDNA from cells. Alternatively, rRNA removal agents may be applied during additional processing operations. The configuration of the constructs generated by such a method can help minimize (or avoid) sequencing of the poly-T sequence during sequencing.

The single cell analysis methods described herein may also be useful in the analysis of the whole transcriptome. Referring back to the barcode of Figure 12B, the priming sequence 1234 may be a random N-mer. In some cases, sequence 1228 is a P7 sequence and sequence 1230 is a R2 primer binding site. In other cases, sequence 1228 is a P5 sequence and sequence 1230 is a R1 primer binding site. In an example method of whole transcriptome analysis using this barcode, the individual cell is co-partitioned along with a barcode bearing bead, poly-T sequence, and other reagents such as reverse transcriptase, polymerase, a reducing agent and dNTPs into a partition (e.g., droplet in an emulsion). In an operation of this method, the cell is lysed while the barcoded oligonucleotides are released from the bead (e.g., via the action of the reducing agent) and the poly-T sequence hybridizes to the poly-A tail of cellular mRNA.

In a reverse transcription reaction using the mRNA as template, cDNA transcripts of cellular mRNA can be produced. The RNA can then be degraded with an RNase. The priming sequence 1234 in the barcoded oligonucleotide can then randomly hybridize to the cDNA transcripts. The oligonucleotides can be extended using polymerase enzymes and other extension reagents co-partitioned with the bead and cell similar to as shown in Figure 3 to generate amplification products (e.g., barcoded fragments), similar to the example amplification product shown in Figure 3 (panel F). The barcoded nucleic acid fragments may, in some cases subjected to further processing (e.g., amplification, addition of additional sequences, clean up processes, etc. as described elsewhere herein) characterized, e.g., through sequence analysis. In this operation, sequencing signals can come from full length RNA.

In some embodiments, the barcode sequence can be appended to the 3' end of the template polynucleotide sequence (e.g., mRNA). Such configuration may be desired, for example, if the sequence at the 3' end of the template polynucleotide is desired to be analyzed.

In some embodiments, the barcode sequence can be appended to the 5' end of a template polynucleotide sequence (e.g., mRNA). Such configuration may be desired, for example, if the sequence at the 5' end of the template polynucleotide is desired to be analyzed.

In some embodiments, a barcode sequence can be appended to the 3' end of a first subset of the template polynucleotides, and a barcode sequence can be appended to the 5' end of a second subset of the template polynucleotides. In some embodiments, the first subset of template polynucleotides and the second subset of template polynucleotides are appended to barcode sequences in the same partition. In some cases, the barcodes appended to the 3' ends of template polynucleotides are different from the barcodes appended to the 5' ends of template polynucleotides. For example, the barcodes appended to the 3' ends may have a different barcode sequence compared to the barcodes appended to the 5' end. In some cases, the barcodes appended to the 3' ends of template polynucleotides have the same barcode sequence as the barcodes appended to the 5' ends of template polynucleotides. In some cases, beads are used to deliver the barcode oligonucleotides to partitions. The different barcodes can be attached to the same or different bead.

A barcode sequence can be appended to the 5' end of a template polynucleotide sequence by any suitable method. In some cases, the template polynucleotide is a messenger RNA, mRNA, molecule. The barcode sequence can be appended to the 5' end of a template polynucleotide sequence by use of a primer comprising the barcode sequence in a primer extension reaction. For example, the barcode may be present in a primer used for a primer extension reaction in which the template polynucleotide or a derivative thereof, for example an amplification product, is used as the template for primer extension. In some cases, the barcode may be present on a template switching oligonucleotide participating in a primer extension reaction. As an alternative, the barcode sequence can be appended to the 5' end of a template polynucleotide by ligating an oligonucleotide comprising the barcode sequence directly to the template polynucleotide.

In another aspect, the present disclosure provides a method of appending a barcode sequence to the 5' end of a template polynucleotide sequence by a primer extension reaction using a primer comprising a barcode sequence and the template polynucleotide or a derivative thereof as the template for primer extension. The primer extension reaction may occur in a partition. In some embodiments, a cell, or a nucleic acid derivative thereof, is co-partitioned with a primer capable of primer extension and a template switching oligo comprising a barcode sequence. The primer capable of primer extension may hybridize to a nucleic acid of the cell or to a nucleic acid derivative. In some cases, the template switching oligo comprising the barcode sequence is releasably attached to a bead, e.g., a gel bead. In some embodiments, a cell, or a nucleic acid derivative thereof, is co-partitioned with a primer having a sequence towards a 3' end that hybridizes to the template polynucleotide, a template switching oligonucleotide having a first predefined sequence towards a 5' end, and a microcapsule, such as a bead, having barcoded oligonucleotides releasably coupled thereto. In some embodiments, the oligonucleotides coupled to the bead include barcode sequences that are identical (e.g., all oligonucleotides sharing the same barcode sequence). In some aspects, the oligonucleotides coupled to the beads additionally include unique molecular identifier (UMI) sequence segments (e.g., all oligonucleotides having different unique molecular identifier sequences).

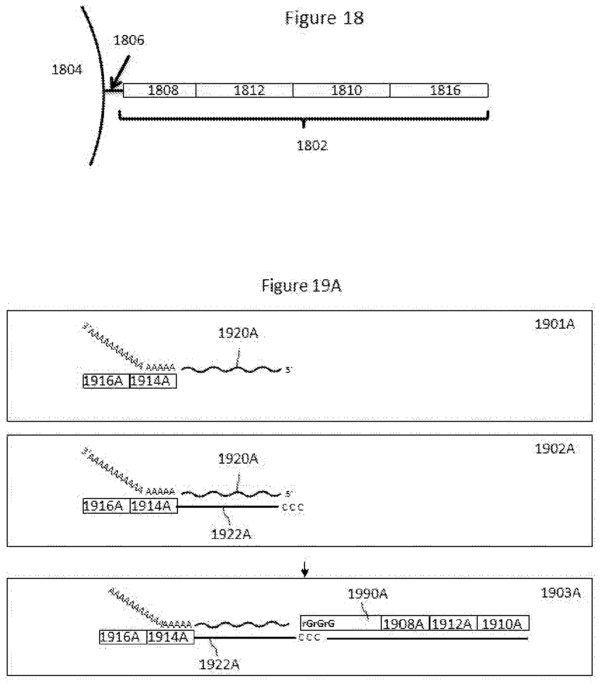

In an example, Figure 18 shows a barcoded oligonucleotide coupled to a bead. As shown, the overall oligonucleotide 1802 is coupled to a bead 1804 by a releasable linkage 1806, such as a disulfide linker. The oligonucleotide may include functional sequences that are useful for subsequent processing, such as functional sequence 1808, which may include a sequencer specific flow cell attachment sequence, e.g., a P5 sequence, as well as functional sequence 1810, which may include sequencing primer sequences, e.g., a R1 primer binding site. In some cases, sequence 1808 is a P7 sequence and sequence 1810 is a R2 primer binding site. A barcode sequence 1812 can be included within the structure for use in barcoding the template polynucleotide. The functional sequences may be selected for compatibility with a variety of different sequencing systems, e.g., 454 Sequencing, Ion Torrent Proton or PGM, Illumina X10, etc., and the requirements thereof. In many cases, the barcode sequence 1812, functional sequences 1808 (e.g., flow cell attachment sequence) and 1810 (e.g., sequencing primer sequences) may be common to all of the oligonucleotides attached to a given bead. The barcoded oligonucleotide can also comprise a sequence 1816 to facilitate template switching (e.g., a polyG sequence). In some cases, the additional sequence provides a unique molecular identifier (UMI) sequence segment, as described elsewhere herein. The one or more functional sequences that may be present in an oligonucleotide can be arranged in any suitable order.

Although shown as a single oligonucleotide tethered to the surface of a bead, individual beads can include tens to hundreds of thousands or even millions of individual oligonucleotide molecules, where, as previously noted herein, the barcode sequence can be constant or relatively constant for a given bead.

In an example method of generating labeled polynucleotides using a barcode oligonucleotide, a cell or a nucleic acid derived therefrom is co-partitioned with a bead bearing a barcoded oligonucleotide and reagents such as reverse transcriptase, poly-T primers, dNTPs, and a chemical stimulus (e.g., reducing agent) into a partition. The barcoded oligonucleotide attached to the bead can comprise a sequence to facilitate template switching (e.g., polyG or riboG). The partition can be a droplet in an emulsion. In cases where a cell is provided in the partition, the partition can further comprise a lysis reagent to lyse the cell.

Where the bead is a degradable or disruptable bead, the barcoded oligonucleotide can be released from the bead when contacted with the chemical stimulus (e.g., reducing agent). Following release from the bead, the barcoded oligonucleotide can be present in the partition at any suitable concentration. In some embodiments, the barcoded oligonucleotide is present in the partition at a concentration that is suitable for generating a sufficient yield of amplification products for downstream processing and analysis, including, but not limited to, sequencing adaptor attachment and sequencing analysis.

With reference to Figure 19A, in 1901A, an oligonucleotide with a poly-T sequence 1914A, and in some cases an additional sequence 1916A that binds to, for example, a sequencing or PCR primer, anneals to a target mRNA 1920A. In 1902A, the oligonucleotide is extended yielding an anti-sense strand 1922A which is appended by multiple cytidines on the 3' end. In 1903A, the template switching sequence 1990A (e.g., polyG or riboG) of the barcoded oligonucleotide pairs with the cytidines of the anti-sense strand 1922A and the anti-sense strand is extended using the barcoded oligonucleotide as template. In addition to the riboG sequence, the barcoded oligonucleotide can comprise additional functional sequences 1908A, 1912A, and 1910A. In some cases, the barcoded oligonucleotide comprises a unique molecular identifier (UMI, for example 1908A), a barcode sequence (for example 1912A), and a Read 1 sequence (R1, for example 1910A). Operations 1901A, 1902A, and 1903A may be performed in the partition (e.g., droplet or well). The extension in 1902A and 1903A can be facilitated by an enzyme comprising polymerase activity. For example, the extension can be facilitated by a DNA-dependent polymerase or a reverse-transcriptase (e.g., RNA dependent). In some embodiments, the extension comprises polymerase chain reaction. In some embodiments, the extension comprises reverse transcription. The enzyme can add nucleotides in a template independent manner. In some cases, at least three cytidines are appended to the 3' end of the cDNA transcript in a template independent manner.

Subsequent to 1903A, the nucleic acid product (e.g., cDNA product) may be released from the partition and subject to further processing reactions such as additional amplification. In some cases, the nucleic acid product is pooled with products from other partitions for subsequent processing in bulk. In some cases, a portion of the amplified product can be subjected to enrichment to obtain a subset of nucleic acids corresponding to genes of interest.

In some cases, enrichment to obtain a subset of nucleic acids corresponding to genes of interest comprises one or more amplification reactions. One or more gene specific primers can be used for primer extension using the cDNA molecule as a template. Any of a variety of polymerases can be used in embodiments herein for primer extension, non-limiting examples of which include exonuclease minus DNA Polymerase I large (Klenow) Fragment, Phi29 DNA polymerase, Taq DNA Polymerase, T4 DNA polymerase, T7 DNA polymerase, and the like. Further examples of polymerase enzymes that can be used in embodiments herein include thermostable polymerases, including but not limited to, Thermus thermophilus HB8; Thermus oshimai; Thermus scotoductus; Thermus thermophilus 1B21; Thermus thermophilus GK24; Thermus aquaticus polymerase AmpliTaq(R) FS or Taq (G46D; F667Y), Taq (G46D; F667Y; E6811), and Taq (G46D; F667Y; T664N; R660G); Pyrococcus furiosus polymerase; Thermococcus gorgonarius polymerase; Pyrococcus species GB-D polymerase; Thermococcus sp. (strain 9deg. N-7) polymerase; Bacillus stearo thermophilus polymerase; Tsp polymerase; Thermus flavus polymerase; Thermus litoralis polymerase; Thermus Z05 polymerase; delta Z05 polymerase (e.g. delta Z05 Gold DNA polymerase); and mutants, variants, or derivatives thereof. In some embodiments, a hot start polymerase is used. A hot start polymerase is a modified form of a DNA polymerase that can be activated by incubation at elevated temperatures.

Additional functional sequences can be added to the nucleic acid product or an amplification product thereof. The additional functional sequences may allow for amplification or sample identification. This may occur in the partition or, alternatively, in bulk. In some cases, the amplification products can be sheared, ligated to adapters and amplified to add additional functional sequences. In some cases, both the enriched and unenriched amplification products are subject to analysis.

In an example method of cellular polynucleotide analysis using the barcode oligonucleotide of Figure 18, a cell is co-partitioned along with a bead bearing a barcoded oligonucleotide and additional reagents such as reverse transcriptase, primers, oligonucleotides (e.g., template switching oligonucleotides), dNTPs, and reducing agent into a partition (e.g., a droplet in an emulsion). Within the partition, the cell can be lysed to yield a plurality of template polynucleotides (e.g., DNA such as genomic DNA, RNA such as mRNA, etc). In some cases, the cell is lysed using a lysis reagent that is co-partitioned with the cell.

Where the bead is a degradable or disruptable bead, the barcoded oligonucleotide can be released from the bead following the application of stimulus as previously described herein. Following release from the bead, the barcoded oligonucleotide can be present in the partition at any suitable concentration. In some embodiments, the barcoded oligonucleotide is present in the partition at a concentration that is suitable for generating a sufficient yield of amplification products for downstream processing and analysis, including, but not limited to, sequencing adaptor attachment and sequencing analysis. In some embodiments, the concentration of the barcoded oligonucleotide is limited by the loading capacity of the barcode bearing bead, or the amount of oligonucleotides deliverable by the bead.

The template switching oligonucleotide, which can be co-partitioned with the cell, bead bearing barcoded oligonucleotides, etc, can be present in the partition at any suitable concentration. In some embodiments, the template switching oligonucleotide is present in the partition at a concentration that is suitable for efficient template switching during an amplification reaction. The concentration of the template switching oligonucleotide can be dependent on the reagents used for droplet generation. In some embodiments, the template switching oligonucleotide is among a plurality of template switching oligonucleotides.

In some embodiments, the barcoded oligonucleotide and template switching oligonucleotide are present in the partition at similar concentrations. In some embodiments, the barcoded oligonucleotide and template switching oligonucleotides may be present in proportions reflective of the desired amount of amplification products to be generated using each oligonucleotide. In some embodiments, the template switching oligonucleotide is present in the partition at a greater concentration than the barcoded oligonucleotide. This difference in concentration can be due to limitations on the capacity of the barcode bearing bead. In some embodiments, the concentration of the template switching oligonucleotide in the reaction volume is at least 2, 5, 10, 20, 50, 100, 200 or more times that of the concentration of the barcoded oligonucleotide in the same reaction volume when the barcoded oligonucleotide is free in the partition (e.g., not attached to the bead).

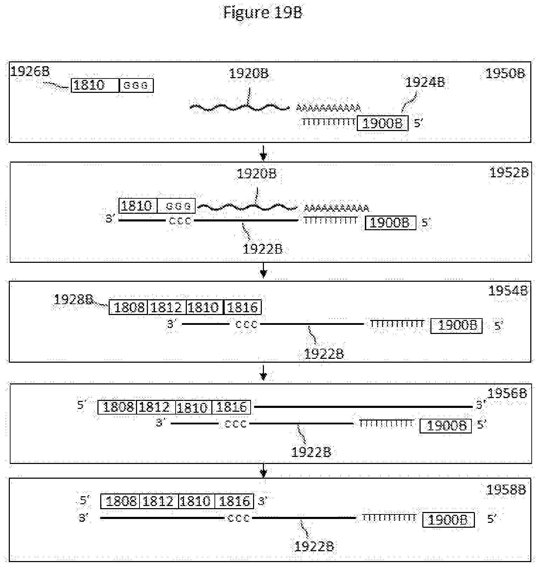

As illustrated in Figure 19B, a reaction mixture comprising a template polynucleotide from a cell 1920B and (i) the primer 1924B having a sequence towards a 3' end that hybridizes to the template polynucleotide (e.g., polyT) and an additional sequence element 1900B and (ii) a template switching oligonucleotide 1926B that comprises a first predefined sequence 1810 towards a 5' end can be subjected to an amplification reaction to yield a first amplification product. In some cases, the template polynucleotide is an mRNA with a polyA tail and the primer that hybridizes to the template polynucleotide comprises a polyT sequence towards a 3' end, which is complementary to the polyA segment. The first predefined sequence can comprise at least one of an adaptor sequence, a barcode sequence, a unique molecular identifier (UMI) sequence, a primer binding site, and a sequencing primer binding site or any combination thereof. In some cases, the first predefined sequence 1810 is a sequence that can be common to all partitions of a plurality of partitions. For example, the first predefined sequence may comprise a flow cell attachment sequence, an amplification primer binding site, or a sequencing primer binding site and the first amplification reaction facilitates the attachment the predefined sequence to the template polynucleotide from the cell. In some embodiments, the first predefined sequence comprises a primer binding site. In some embodiments, the first predefined sequence comprises a sequencing primer binding site. In some embodiments, the first predefined sequence comprises a barcode sequence. As illustrated in operation 1950B, the sequence towards a 3' end (e.g., polyT) of the primer 1924B hybridizes to the template polynucleotide 1920B. In a first amplification reaction, extension reaction reagents, e.g., reverse transcriptase, nucleoside triphosphates, co-factors (e.g., Mg2+ or Mn2+), that are also co-partitioned, can extend the primer 1924B sequence using the cell's nucleic acid as a template, to produce a transcript, e.g., cDNA transcript, 1922B having a fragment complementary to the nucleic acid to which the primer annealed. In some cases, the reverse transcriptase has terminal transferase activity and the reverse transcriptase adds additional nucleotides, e.g., polyC, to the cDNA transcript in a template independent manner. As illustrated in operation 1952B, the template switching oligonucleotide 1926B, for example a template switching oligonucleotide which includes a polyG sequence, can hybridize to the cDNA transcript 1922B and facilitate template switching in the first amplification reaction. The transcript, therefore, may comprise the sequence of the primer 1924B, a sequence complementary to the template polynucleotide from the cell, and a sequence complementary to the template switching oligonucleotide.

Among a plurality of partitions, the primer and template switching oligonucleotide may be universal to all partitions. The partitions may individually contain more than one cell, one cell, no cells, or nucleic acids derived from a cell. Where analysis of mRNA is desired, for example, the primer may comprise at least a polyT segment capable of hybridizing and priming an extension reaction from the polyA segment of an mRNA. Where analysis of a variety of polynucleotides is desired, the primer may comprise a random sequence capable of hybridizing to and priming extension reactions randomly on various polynucleotide templates. As template switching can occur with the use of an enzyme having terminal transferase activity, a template switching oligonucleotide having a sequence capable of hybridizing to the appended bases can be used for template switching in manner that is independent of the sequence of the polynucleotide templates to be analyzed. In some embodiments, the template switching oligonucleotide can comprise a first predefined sequence towards a 5' end that does not specifically hybridize to the template. In some embodiments, analysis of particular genes is desired. In such cases, the primer may comprise a gene specific sequence capable of hybridizing to and priming extension reactions from templates comprising specific genes. In some embodiments, multiple genes are to be analyzed and a primer is among a plurality of primers. Individual primers of the plurality may target different genes. Each of the plurality of primers may have a sequence for a particular gene.

Subsequent to the first amplification reaction, the first amplification product or transcript can be subjected to a second amplification reaction to generate a second amplification product. In some cases, additional sequences (e.g., functional sequences such as flow cell attachment sequence, sequencing primer binding sequences, barcode sequences, etc) are to be attached. The first and second amplification reactions can be performed in the same volume, such as for example in a droplet or well. In some cases, the first amplification product is subjected to a second amplification reaction in the presence of a barcoded oligonucleotide to generate a second amplification product having a barcode sequence. The barcode sequence can be unique to a partition, that is, each partition has a unique barcode sequence. The barcoded oligonucleotide may comprise a sequence of at least a segment of the template switching oligonucleotide and at least a second predefined sequence. The segment of the template switching oligonucleotide on the barcoded oligonucleotide can facilitate hybridization of the barcoded oligonucleotide to the transcript, e.g., cDNA transcript, to facilitate the generation of a second amplification product. In addition to a barcode sequence, the barcoded oligonucleotide may comprise a second defined sequence such as at least one of an adaptor sequence, a unique molecular identifier (UMI) sequence, a primer binding site, and a sequencing primer binding site or any combination thereof.

In some embodiments, the second amplification reaction uses the first amplification product as a template and the barcoded oligonucleotide as a primer. As illustrated in operation 1954B, the segment of the template switching oligonucleotide on the barcoded oligonucleotide 1928B can hybridize to the portion of the cDNA transcript or complementary fragment 1922B having a sequence complementary to the template switching oligonucleotide or that which was copied from the template switching oligonucleotide. In the second amplification reaction, extension reaction reagents, e.g., polymerase, nucleoside triphosphates, co-factors (e.g., Mg2+ or Mn2+), that are also co-partitioned, can extend the primer sequence using the first amplification product as template as illustrated in operation 1956B. The second amplification product can comprise a second predefined sequence (e.g., 1808, 1812, and 1810), a sequence of a segment of the template polynucleotide (e.g., mRNA), and a sequence complementary to the primer (e.g., 1924B). In cases where the template polynucleotide is an mRNA molecule, amplification products derived therefrom can comprise the corresponding DNA sequence, for example thymine instead of uracil bases.

In some embodiments, the second amplification product uses the barcoded oligonucleotide as a template and at least a portion of the first amplification product as a primer. As illustrated in operation 1954B, the segment of the first amplification product (e.g., cDNA transcript) having a sequence complementary to the template switching oligonucleotide can hybridize to the segment of the barcoded oligonucleotide comprising a sequence of at least a segment of the template switching oligonucleotide. In the second amplification reaction, extension reaction reagents, e.g., polymerase, nucleoside triphosphates, co-factors (e.g., Mg2+ or Mn2+), that are also co-partitioned, can extend the primer sequence (e.g., first amplification product) using the barcoded oligonucleotide as template as illustrated in operation 1958B. The second amplification product may comprise the sequence of the primer (e.g., 1924B), a sequence which is complementary to the sequence of the template polynucleotide (e.g., mRNA), and a sequence complementary to the second predefined sequence (e.g., 1808, 1812, and 1810).

In some embodiments, the second amplification reaction is performed subsequent to the first amplification reaction in the presence of an intervening purification step. An intervening purification step can be used, for example, to purify the template (e.g., first amplification product) from excess reagents, including excess primers such as template switching oligonucleotides. In some embodiments, the amplification reaction is performed in the absence of an intervening purification step. In certain embodiments, an intervening purification step is not performed so that all sample preparation is performed in a same reaction volume. In the absence of an intervening purification step, the template switching oligonucleotide may compete with barcoded oligonucleotide in the second amplification reaction as the barcoded oligonucleotide comprises at least a segment of the template switching oligonucleotide. Competition between the template switching oligonucleotide and barcoded oligonucleotide in the second amplification reaction to generate additional amplification product may result in a second amplification product lacking a barcode sequence. Such amplification products lacking a barcode sequence may be undesirable as they lack a barcode sequence which can provide unique identifying information of the template. In some embodiments, the template switching oligonucleotide may out-compete the barcoded oligonucleotide in the second amplification reaction if the template switching oligonucleotide is present at a higher concentration in the reaction volume than the barcoded oligonucleotide. Various approaches can be utilized to favor the use of the barcoded oligonucleotide in the second amplification reaction to generate amplification products having a barcode sequence in situations where the barcoded oligonucleotide is present at a lower concentration than the template switching oligonucleotide in the reaction volume.

In some embodiments, the template switching oligonucleotide is not available for primer extension during the second amplification reaction. In some embodiments, the template switching oligonucleotide is degraded prior to the second amplification reaction. In some embodiments, the template switching oligonucleotide is degraded during the second amplification reaction. The template switching oligonucleotide may comprise ribonucleic acids (RNA). A template switching oligonucleotide comprising RNA can be degraded, for example, by elevated temperatures or alkaline conditions. In some embodiments, the template switching oligonucleotide comprises at least 10%, 15%, 20%, 25%, 30%, 35%, 40%, 45%, 50%, 55%, 60%, 65%, 70%, 75%, 80%, 85%, 90%, or 95% RNA. In some embodiments, the template switching oligonucleotide comprises 100% RNA. In some embodiments, a first reaction rate of the second amplification reaction using the barcoded oligonucleotide is greater than a second reaction rate of the second amplification using the template switching oligonucleotide.

In some embodiments, the barcoded oligonucleotide can hybridize to the first amplification product at a higher annealing temperature as compared to the template switching oligonucleotide. For example, the first amplification product and the barcoded oligonucleotide can have a higher melting temperature as compared to a melting temperature of the first amplification product and the template switching oligonucleotide. In such cases, the second amplification reaction may be performed with an annealing temperature at which the barcoded oligonucleotide is able to hybridize to the first amplification product and initiation primer extension and at which the template switching oligonucleotide is unable to hybridize to the first amplification product and initiate primer extension. In some embodiments, the primer annealing temperature of the second amplification reaction is at least about 0.5 °C, 1 °C, 2 °C, 3 °C, 4 °C, 5 °C, 6 °C, 7 °C, 8 °C, 9 °C, 10 °C or greater than a primer annealing temperature of the first amplification reaction. The difference in melting temperatures can result from the presence of modified nucleotides in the template switching oligonucleotide. In some embodiment, the template switching oligonucleotide comprises at least 10%, 15%, 20%, 25%, 30%, 35%, 40%, 45%, 50%, 55%, 60%, 65%, 70%, 75%, 80%, 85%, 90%, or 95% modified nucleotides. In some embodiments, the template switching oligonucleotide comprises 100% modified oligonucleotides. In some embodiments, the difference in melting temperature can be the result of the presence of modified nucleotides in the barcoded oligonucleotide. In some embodiment, the barcoded oligonucleotide comprises at least 10%, 15%, 20%, 25%, 30%, 35%, 40%, 45%, 50%, 55%, 60%, 65%, 70%, 75%, 80%, 85%, 90%, or 95% modified nucleotides. In some embodiments, the barcoded oligonucleotide comprises 100% modified oligonucleotides. Modified nucleotides include, but are not limited to, 2-Aminopurine, 2,6-Diaminopurine (2-Amino-dA), inverted dT, 5-Methyl dC, 2'-deoxyInosine, Super T (5-hydroxybutynl-2'-deoxyuridine), Super G (8-aza-7-deazaguanosine), locked nucleic acids (LNAs), unlocked nucleic acids (UNAs, e.g., UNA-A, UNA-U, UNA-C, UNA-G), Iso-dG, Iso-dC, and 2' Fluoro bases (e.g., Fluoro C, Fluoro U, Fluoro A, and Fluoro G).

In various embodiments, the first amplification reaction is facilitated using an enzyme comprising polymerase activity. For example, the first amplification reaction can be facilitated by a DNA-dependent polymerase or a reverse-transcriptase (e.g., RNA dependent). In some embodiments, the first amplification reaction comprises polymerase chain reaction. In some embodiments, the first amplification reaction comprises reverse transcription. In various embodiments, the second amplification reaction is facilitated using an enzyme comprising polymerase activity. For example, the second amplification reaction can be facilitated by a DNA-dependent polymerase. In some embodiments, the second amplification reaction comprises polymerase chain reaction.

In another aspect, a template polynucleotide comprising mRNA may first be reverse transcribed to cDNA (e.g., an amplification product of the template polynucleotide). The mRNA molecule can be reverse transcribed to cDNA using a reverse transcriptase enzyme and a primer, such as a poly-T primer. Non-limiting examples of enzymes that can be used for reverse transcription in embodiments herein include HIV-1 reverse transcriptase, M-MLV reverse transcriptase, AMV reverse transcriptase, telomerase reverse transcriptase, and variants, modified products and derivatives thereof.

A gene specific primer having a barcode sequence can then be used for primer extension using the cDNA molecule (e.g., amplification product of the template polynucleotide) as a template. A primer comprising a barcode can hybridize to the cDNA molecule via sequence complementarity. Extension of the primer using the cDNA molecule as template may result in a polynucleotide product comprising the template polynucleotide sequence and the barcode sequence located at the 5' end of the template polynucleotide sequence. Any of a variety of polymerases can be used in embodiments herein for primer extension, non-limiting examples of which include exonuclease minus DNA Polymerase I large (Klenow) Fragment, Phi29 DNA polymerase, Taq DNA Polymerase, T4 DNA polymerase, T7 DNA polymerase, and the like. Further examples of polymerase enzymes that can be used in embodiments herein include thermostable polymerases, including but not limited to, Thermus thermophilus HB8; Thermus oshimai; Thermus scotoductus; Thermus thermophilus 1B21; Thermus thermophilus GK24; Thermus aquaticus polymerase AmpliTaq(R) FS or Taq (G46D; F667Y), Taq (G46D; F667Y; E6811), and Taq (G46D; F667Y; T664N; R660G); Pyrococcus furiosus polymerase; Thermococcus gorgonarius polymerase; Pyrococcus species GB-D polymerase; Thermococcus sp. (strain 9deg. N-7) polymerase; Bacillus stearo thermophilus polymerase; Tsp polymerase; Thermus flavus polymerase; Thermus litoralis polymerase; Thermus Z05 polymerase; delta Z05 polymerase (e.g. delta Z05 Gold DNA polymerase); and mutants, variants, or derivatives thereof. In some embodiments, a hot start polymerase is used. A hot start polymerase is a modified form of a DNA polymerase that can be activated by incubation at elevated temperatures. Such a polymerase can be used, for example, to further increase sensitivity, specificity, and yield; and/or to further improve low copy target amplification.

In another aspect, a barcode sequence is appended to the 5' end of a template polynucleotide sequence by ligating an oligonucleotide comprising a barcode sequence directly to the 5' end of the template polynucleotide. Ligating an oligonucleotide comprising a barcode sequence to a template polynucleotide can be implemented by various methods. In some embodiments herein, ligating an oligonucleotide comprising a barcode sequence to a template polynucleotide involves an enzyme, such as a ligase (e.g., an RNA ligase or a DNA ligase). Non-limiting examples of enzymes that can be used for ligation in embodiments herein include ATP-dependent double-stranded polynucleotide ligases, NAD+ dependent DNA or RNA ligases, and single-strand polynucleotide ligases. Non-limiting examples of ligases which can be used in embodiments herein include CircLigase I and CircLigase II (Epicentre; Madison, WI), Escherichia coli DNA ligase, Thermus filiformis DNA ligase, Tth DNA ligase, Thermus scotoductus DNA ligase (I and II), T3 DNA ligase, T4 DNA ligase, T4 RNA ligase, T7 DNA ligase, Taq ligase, Ampligase (Epicentre®Technologies Corp.), VanC-type ligase, 9° N DNA Ligase, Tsp DNA ligase, DNA ligase I, DNA ligase III, DNA ligase IV, Sso7-T3 DNA ligase, Sso7-T4 DNA ligase, Sso7-T7 DNA ligase, Sso7-Taq DNA ligase, Sso7-E. coli DNA ligase, Sso7-Ampligase DNA ligase, and thermostable ligases. Ligase enzymes may be wild- type, mutant isoforms, and genetically engineered variants.

In some embodiments where a barcode oligonucleotide is ligated to a template polynucleotide comprising mRNA, the mRNA molecule can be treated to yield a 5' monophosphate group prior to ligating. Any suitable reaction may be employed to yield a 5' monophosphate group. For example, the mRNA molecule can be treated with an enzyme such as a pyrophosphohydrolase. An example of a pyrophosphohydrolase that can be used in embodiments herein is RNA 5' phyrophosphohydrolase (RppH). In some cases, all of the phosphate groups at the 5' end of the molecule are removed and a single phosphate groups is added back to the 5' end. In some cases, two phosphate groups are removed from a triphosphate group to yield a monophosphate. In some cases, a single enzyme both removes the phosphate groups present on the mRNA molecule and adds the monophosphate group. In some cases, a first enzyme removes the phosphate groups present on the mRNA molecule and a second enzyme adds the monophosphate group. In some cases, the phosphate groups are removed from the 5' end of the mRNA molecule and the 5' end is adenylated. An enzyme which can be used for 5' adenylation in embodiments herein includes Mth RNA ligase.

In some cases, the oligonucleotide comprising the barcode sequence is ligated to the template polynucleotide within a partition (e.g., droplet or well). A partition, in some cases, comprises a polynucleotide sample comprising the template polynucleotide, an oligonucleotide having the barcode sequence, a ligase enzyme, and any other suitable reagents for ligation. The ligase can implement the attachment of the oligonucleotide comprising the barcode sequence to the template polynucleotide within the partition. In some cases, the template polynucleotide is an mRNA molecule and the oligonucleotide ligated to it is a DNA molecule. In some cases, the oligonucleotide comprising the barcode sequence is ligated to the template polynucleotide outside of a partition.

Following the attachment of an oligonucleotide comprising a barcode sequence to the 5' end of a template polynucleotide, for example an mRNA polynucleotide, the barcoded template can be subjected to further amplification. In some cases, one or more further amplification reactions are performed within the partition. In some cases, one or more further amplification reactions are performed outside of a partition. In some cases, a plurality of barcoded mRNA polynucleotides, for example from a plurality of partitions, is pooled and subjected to further processing in bulk. In some embodiments, the barcoded template polynucleotide is subjected to polymerase chain reaction. In some embodiments, the template polynucleotide comprises mRNA and the barcoded template polynucleotide is subjected to reverse transcription, yielding a cDNA transcript. In embodiments where reverse transcription is performed in a partition, the partitions can comprise primers having a poly-T region capable of hybridizing to the poly-A region of the barcoded mRNA. Within the partition, the primer having a poly-T region can hybridize to the barcoded template and initiate primer extension in reverse transcription. Non-limiting examples of enzymes that can be used for reverse transcription in embodiments herein include HIV-1 reverse transcriptase, M-MLV reverse transcriptase, AMV reverse transcriptase, telomerase reverse transcriptase, and variants, modified products and derivatives thereof. A partition can contain a reverse transcriptase enzyme capable of reverse transcribing a template polynucleotide that is attached at its 5' end to a barcoded oligonucleotide. In embodiments where reverse transcription is performed in bulk, a plurality of barcoded mRNA polynucleotides from a plurality of partitions can be pooled for bulk processing. The reaction volume for performing reverse transcription can comprise primers having a poly-T region capable of hybridizing to the poly-A region of a barcoded mRNA. In some cases, the primers for reverse transcription further comprise additional elements, such as tags, which can be used, for example, for isolating cDNA transcripts. For example, cDNA transcripts comprising biotin tags can be isolated from components of the reaction volume (e.g., excess primers, reverse transcriptase enzyme, barcoded mRNA molecules) by performing a purification reaction with streptavidin or other molecule capable of binding biotin.

Following the generation of barcoded template polynucleotides or derivatives (e.g., amplification products) thereof, subsequent operations may be performed, including purification (e.g., via solid phase reversible immobilization (SPRI)) or further processing (e.g., shearing, addition of functional sequences, and subsequent amplification (e.g., via PCR)). Functional sequences, such as flow cell sequences, may be added by ligation. These operations may occur in bulk (e.g., outside the partition). In the case where a partition is a droplet in an emulsion, the emulsion can be broken and the contents of the droplet pooled for additional operations. Additional reagents that may be co-partitioned along with the barcode bearing bead may include oligonucleotides to block ribosomal RNA (rRNA) and nucleases to digest genomic DNA from cells. Alternatively, rRNA removal agents may be applied during additional processing operations. The configuration of the constructs generated by such a method can help minimize (or avoid) sequencing of the poly-T sequence during sequencing and/or sequence the 5' end of a polynucleotide sequence. The amplification products, for example first amplification products and/or second amplification products, may be subject to sequencing for sequence analysis.

Although operations with various barcode designs have been discussed individually, individual beads can include barcode oligonucleotides of various designs for simultaneous use.

In addition to characterizing individual cells or cell sub-populations from larger populations, the processes and systems described herein may also be used to characterize individual cells as a way to provide an overall profile of a cellular, or other organismal population. A variety of applications require the evaluation of the presence and quantification of different cell or organism types within a population of cells, including, for example, microbiome analysis and characterization, environmental testing, food safety testing, epidemiological analysis, e.g., in tracing contamination or the like. In particular, the analysis processes described above may be used to individually characterize, sequence and/or identify large numbers of individual cells within a population. This characterization may then be used to assemble an overall profile of the originating population, which can provide important prognostic and diagnostic information.

For example, shifts in human microbiomes, including, e.g., gut, buccal, epidermal microbiomes, etc., have been identified as being both diagnostic and prognostic of different conditions or general states of health. Using the single cell analysis methods and systems described herein, one can again, characterize, sequence and identify individual cells in an overall population, and identify shifts within that population that may be indicative of diagnostic ally relevant factors. By way of example, sequencing of bacterial 16S ribosomal RNA genes has been used as a highly accurate method for taxonomic classification of bacteria. Using the targeted amplification and sequencing processes described above can provide identification of individual cells within a population of cells. One may further quantify the numbers of different cells within a population to identify current states or shifts in states over time. See, e.g., Morgan et al, PLoS Comput. Biol., Ch. 12, December 2012, 8(12):e1002808, and Ram et al., Syst. Biol. Reprod. Med., June 2011, 57(3):162-170. Likewise, identification and diagnosis of infection or potential infection may also benefit from the single cell analyses described herein, e.g., to identify microbial species present in large mixes of other cells or other biological material, cells and/or nucleic acids, including the environments described above, as well as any other diagnostically relevant environments, e.g., cerebrospinal fluid, blood, fecal or intestinal samples, or the like.

The foregoing analyses may also be particularly useful in the characterization of potential drug resistance of different cells or pathogens, e.g., cancer cells, bacterial pathogens, etc., through the analysis of distribution and profiling of different resistance markers/mutations across cell populations in a given sample. Additionally, characterization of shifts in these markers/mutations across populations of cells over time can provide valuable insight into the progression, alteration, prevention, and treatment of a variety of diseases characterized by such drug resistance issues.

Although described in terms of cells, it will be appreciated that any of a variety of individual biological organisms, or components of organisms are encompassed within this description, including, for example, cells, viruses, organelles, cellular inclusions, vesicles, or the like. Additionally, where referring to cells, it will be appreciated that such reference includes any type of cell, including without limitation prokaryotic cells, eukaryotic cells, bacterial, fungal, plant, mammalian, or other animal cell types, mycoplasmas, normal tissue cells, tumor cells, or any other cell type, whether derived from single cell or multicellular organisms.

Similarly, analysis of different environmental samples to profile the microbial organisms, viruses, or other biological contaminants that are present within such samples, can provide important information about disease epidemiology, and potentially aid in forecasting disease outbreaks, epidemics an pandemics.

As described above, the methods, systems and compositions described herein may also be used for analysis and characterization of other aspects of individual cells or populations of cells. In one example process, a sample is provided that contains cells that are to be analyzed and characterized as to their cell surface proteins. Also provided is a library of antibodies, antibody fragments, or other molecules having a binding affinity to the cell surface proteins or antigens (or other cell features) for which the cell is to be characterized (also referred to herein as cell surface feature binding groups). For ease of discussion, these affinity groups are referred to herein as binding groups. The binding groups can include a reporter molecule that is indicative of the cell surface feature to which the binding group binds. In particular, a binding group type that is specific to one type of cell surface feature will comprise a first reporter molecule, while a binding group type that is specific to a different cell surface feature will have a different reporter molecule associated with it. In some aspects, these reporter molecules will comprise oligonucleotide sequences. Oligonucleotide based reporter molecules provide advantages of being able to generate significant diversity in terms of sequence, while also being readily attachable to most biomolecules, e.g., antibodies, etc., as well as being readily detected, e.g., using sequencing or array technologies. In the example process, the binding groups include oligonucleotides attached to them. Thus, a first binding group type, e.g., antibodies to a first type of cell surface feature, will have associated with it a reporter oligonucleotide that has a first nucleotide sequence. Different binding group types, e.g., antibodies having binding affinity for other, different cell surface features, will have associated therewith reporter oligonucleotides that comprise different nucleotide sequences, e.g., having a partially or completely different nucleotide sequence. In some cases, for each type of cell surface feature binding group, e.g., antibody or antibody fragment, the reporter oligonucleotide sequence may be known and readily identifiable as being associated with the known cell surface feature binding group. These oligonucleotides may be directly coupled to the binding group, or they may be attached to a bead, molecular lattice, e.g., a linear, globular, cross-slinked, or other polymer, or other framework that is attached or otherwise associated with the binding group, which allows attachment of multiple reporter oligonucleotides to a single binding group.

In the case of multiple reporter molecules coupled to a single binding group, such reporter molecules can comprise the same sequence, or a particular binding group will include a known set of reporter oligonucleotide sequences. As between different binding groups, e.g., specific for different cell surface features, the reporter molecules can be different and attributable to the particular binding group.

Attachment of the reporter groups to the binding groups may be achieved through any of a variety of direct or indirect, covalent or non-covalent associations or attachments. For example, in the case of oligonucleotide reporter groups associated with antibody based binding groups, such oligonucleotides may be covalently attached to a portion of an antibody or antibody fragment using chemical conjugation techniques (e.g., Lightning-Link® antibody labeling kits available from Innova Biosciences), as well as other non-covalent attachment mechanisms, e.g., using biotinylated antibodies and oligonucleotides (or beads that include one or more biotinylated linker, coupled to oligonucleotides) with an avidin or streptavidin linker. Antibody and oligonucleotide biotinylation techniques are available (See, e.g., Fang, et al., Fluoride-Cleavable Biotinylation Phosphoramidite for 5'-end-Labeling and Affinity Purification of Synthetic Oligonucleotides, Nucleic Acids Res. Jan 15, 2003; 31(2):708-715, DNA 3' End Biotinylation Kit, available from Thermo Scientific). Likewise, protein and peptide biotinylation techniques have been developed and are readily available (See, e.g., U.S. Patent No. 6,265,552).

The reporter oligonucleotides may be provided having any of a range of different lengths, depending upon the diversity of reporter molecules desired or a given analysis, the sequence detection scheme employed, and the like. In some cases, these reporter sequences can be greater than about 5 nucleotides in length, greater than about 10 nucleotides in length, greater than about 20, 30, 40, 50, 60, 70, 80, 90, 100, 120, 150 or even 200 nucleotides in length. In some cases, these reporter nucleotides may be less than about 250 nucleotides in length, less than about 200, 180, 150, 120 100, 90, 80, 70, 60, 50, 40, or even 30 nucleotides in length. In many cases, the reporter oligonucleotides may be selected to provide barcoded products that are already sized, and otherwise configured to be analyzed on a sequencing system. For example, these sequences may be provided at a length that ideally creates sequenceable products of a desired length for particular sequencing systems. Likewise, these reporter oligonucleotides may include additional sequence elements, in addition to the reporter sequence, such as sequencer attachment sequences, sequencing primer sequences, amplification primer sequences, or the complements to any of these.

In operation, a cell-containing sample is incubated with the binding molecules and their associated reporter oligonucleotides, for any of the cell surface features desired to be analyzed. Following incubation, the cells are washed to remove unbound binding groups. Following washing, the cells are partitioned into separate partitions, e.g., droplets, along with the barcode carrying beads described above, where each partition includes a limited number of cells, e.g., in some cases, a single cell. Upon releasing the barcodes from the beads, they will prime the amplification and barcoding of the reporter oligonucleotides. As noted above, the barcoded replicates of the reporter molecules may additionally include functional sequences, such as primer sequences, attachment sequences or the like.

The barcoded reporter oligonucleotides are then subjected to sequence analysis to identify which reporter oligonucleotides bound to the cells within the partitions. Further, by also sequencing the associated barcode sequence, one can identify that a given cell surface feature likely came from the same cell as other, different cell surface features, whose reporter sequences include the same barcode sequence, i.e., they were derived from the same partition.

Based upon the reporter molecules that emanate from an individual partition based upon the presence of the barcode sequence, one may then create a cell surface profile of individual cells from a population of cells. Profiles of individual cells or populations of cells may be compared to profiles from other cells, e.g., 'normal' cells, to identify variations in cell surface features, which may provide diagnostically relevant information. In particular, these profiles may be particularly useful in the diagnosis of a variety of disorders that are characterized by variations in cell surface receptors, such as cancer and other disorders.

The present disclosure also provides methods for reducing nonspecific priming in a single-cell 5' gene expression assay. In generating an assay that allows measurement of 1) a cell barcode sequence (barcode), 2) a unique molecular identifier sequence (UMI) and 3) the 5' sequence of an mRNA transcript simultaneously, one strategy is to place these sequences on a sequence that attaches to the 5' end of an mRNA transcript -- in the present disclosure, this may be accomplished by placing the barcode and UMI on a template switching oligonucleotide (TSO). This oligonucleotide may be attached to the first strand cDNA via a template switching reaction where the reverse transcription (RT) enzyme 1) reverse transcribes a messenger RNA (mRNA) sequence into first-strand complementary DNA (cDNA) from a primer targeting the 3' end of the mRNA, 2) adds nontemplated cytidines to the 5' end of the first-strand cDNA, 3) switches template to the TSO, which may contain 3' guanidines or guanidine-derivatives that hybridize to the added cytidines. The result is a first-strand cDNA molecule that is complementary to the TSO sequence: cell-barcode, UMI, guanidines, and the 5' end of the mRNA.

In some cases, the TSO may co-exist in solution with the RT enzyme and the total RNA contents of a cell. If the TSO is a single stranded DNA (ssDNA) molecule, it can participate as an RT primer rather than as a template-switching substrate. Given, for example, that the over 90% of the total RNA contents of a cell include noncoding ribosomal RNA (rRNA), this may produce barcoded off products that do not contribute to the 5' gene expression or V(D)J sequencing assay but do consume sequencing reads, increasing the cost required to achieve the same sequencing depth. In addition, if the UMI is implemented as a randomer, the presence of this randomer at the 3' end of the TSO greatly increases its ability to serve as a primer on rRNA template.

In some cases, a TSO that is less likely to serve as an RT primer via the introduction of a particular spacer sequence between the UMI and terminal riboGs may be used. Another approach is to design and include a set of auxiliary blocking oligonucleotides that may hybridize to rRNA and prevent binding of the TSO.

The spacer sequence can be optimized by selecting a sequence that minimizes the predicted melting temperature of the (spacer-GGG):rRNA duplex against all human ribosomal RNA molecules.

The blocker sequences can be optimized by selecting sequences that maximize the predicted melting temperature of the (blocker):rRNA duplex against all human ribosomal RNA molecules.

Provided herein are TSO that are less likely to serve as an RT primer via the introduction of a particular spacer sequence between the UMI and terminal riboGs. Additionally, described herein are auxiliary blocking oligonucleotides that hybridize to rRNA and prevent binding of the TSO.

**Table 1** provides examples of spacer sequences that are optimized by selecting a sequence that minimizes the predicted melting temperature of the (spacer-GGG):rRNA duplex against all human ribosomal RNA molecules.

| Table 1. Spacer sequences | |
|---|---|
| GG_S1_6 | TTATATGGG |
| GG_S1_10 | TTTCTTATATGGG |
| GG_S1_20 | AAATCAAATCTTTCTTATATGGG |
| GG_S1_30 | ACAAACAAATAAATCAAATCTTTCTTATATGGG |
| GG_S2_6 | TTTAAAGGG |
| GG_S2_10 | GAAATTTAAAGGG |
| GG_S2_20 | CACTCTACATGAAATTTAAAGGG |
| GG_S2_30 | CCAAAGTTGTCACTCTACATGAAATTTAAAGGG |
| GL6_S3_6 | ATATAAGGG |
| GL6_S3_10 | ATATATATAAGGG |
| GL6_S3_20 | ATATATATATATATATATAAGGG |
| GL6_S3_30 | ATATATATATATATATATATATATATATAAGGG |

**Table** 2 provides examples of blocker sequences that are optimized by selecting sequences that maximize the predicted melting temperature of the (blocker):rRNA duplex against all human ribosomal RNA molecules.

| Table 2. Blocker sequences | |
|---|---|
| 28S_30_3130 | GCCGGCCGCCCCGGCGGCCGCCGCGCGGCC |
| 18S_30_254 | GCCGCCGGCGCCCGCCCCCCGGCCGGGGCC |
| 28S_30_2088 | GCGCGCGCGCGCGCCGCCCCCGCCGCTCCC |
| 28S_30_3284 | GGGGCGCGCCGCGCCGCCGCCGGGCTCCCC |
| 28S_30_834 | GCCGCCGCCACCGCCGCCGCCGCCGCCGCC |
| 28S_30_373 | GCCCCGCCCCGCCGCCCGCCGACCGCCGCC |

(continued)

| Table 2. Blocker sequences | |
|---|---|
| 28S_30_473 | GCGGCCCCTCCGCCGCCTGCCGCCGCCGCC |
| 28S_30_105 | GGAGCGGGTCGCGCCCGGCCGGGCGGGCGC |
| 28S_30_129 | GCCCCGCCCCCCGACCCGCGCGCGGCACCC |
| 28S_30_989 | GGCGGCCCGCAGGGCCGCGGACCCCGCCCC |
| 28S_30_781 | GGCGGGGCACGCGCCCTCCCGCGGCGGGGC |
| 18S_30_750 | GCCAGGGCCGTGGGCCGACCCCGGCGGGGC |
| 28S_30_611 | GTCCCCGCCGACCCCACCCCCGGCCCCGC |
| 18S_30_693 | GGCTCGCCTCGCGGCGGACCGCCCGCCCGC |
| 28S_30_232 | GACCCGGGCGCGCGCCGGCCGCTACCGGCC |
| 28S_30_919 | GCGCGCCTCGTCCAGCCGCGGCGCGCGCCC |
| 28S_30_050 | GCGCCGTGGGAGGGGTGGCCCGGCCCCCCC |
| 28S_30_725 | GGGCCCCCCGAGCCACCTTCCCCGCCGGGC |
| 28S_30_295 | GGCGGCTCCACCCGGGCCCGCGCCCTAGGC |
| 28S_30_004 | GGCGCGGGGTGGGGAGGGAGCGAGCGGCGC |
| 28S_30_547 | GCTAGGCGCCGGCCGAGGCGAGGCGCGCGC |
| 28S_30_115 | GTCCCGCGCCCCGCGGGGCGGGGATTCGGC |
| 28S_30_858 | GGGGCGGCCGCCTTTCCGGCCGCGCCCCGT |
| 28S_30_451 | ACCTCCCCGGCGCGGCGGGCGAGACGGGCC |
| 28S_30_472 | GATCCGCCGGGCCGCCGACACGGCCGGACC |
| 28S_30_246 | GCCGACCCCGTGCGCTCGCTCCGCCGTCCC |
| 28S_30_936 | GCGCGGCGACGGGTCTCGCTCCCTCGGCCC |
| 28S_30_207 | GCCCGGCTCGCGTCCAGAGTCCGCGCCGCC |
| 28S_30_578 | TCCCCGGGGCTCCCGCCGGCTTCTCCGGGA |
| 28S_30_380 | ACCTCGGCCGGCGAGCGCGCCGGCCTTCAC |
| 28S_30_791 | ACGCCCGGCTCCACGCCAGCGAGCCGGGCT |
| 28S_30_684 | GCTCACCGGACGCCGCCGGAACCGCGACGC |
| 28S_30_441 | TCGCCCGTCCCTTCGGAACGGCGCTCGCCC |
| 28S_30_671 | GGGGTGCGTCGGGTCTGCGAGAGCGCCAGC |
| 28S_30_696 | GGCCAACCGAGGCTCCGCGGCGCTGCCGTA |
| 18S_30_551 | GTTACCCGCGCCTGCCGGCGTAGGGTAGGC |
| 28S_30_634 | GCGTCAACACCCGCCGCGGGCCTTCGCGAT |
| 18S_30_827 | AGCTGCGGTATCCAGGCGGCTCGGGCCTGC |
| 28S_30_883 | GCGTCGGCATCGGGCGCCTTAACCCGGCGT |
| 18S_30_088 | GGGAATAACGCCGCCGCATCGCCGGTCGGC |
| 18S_30_923 | GCGGCGCAATACGAATGCCCCCGGCCGTCC |
| 28S_30_755 | TGCTGCGGATATGGGTACGGCCCGGCGCGA |
| 18S_30_328 | GGGCAGACGTTCGAATGGGTCGTCGCCGCC |
| 18S_30_207 | GCCGCAGGCTCCACTCCTGGTGGTGCCCTT |

(continued)

| Table 2. Blocker sequences | |
|---|---|
| 18S_30_597 | ACCGCGGCTGCTGGCACCAGACTTGCCCTC |
| 18S_30_473 | GGGTCGGGAGTGGGTAATTTGCGCGCCTGC |
| 28S_30_1 | AGCGGGTCGCCACGTCTGATCTGAGGTCGC |
| 28S_30_851 | TTCCCCGCTGATTCCGCCAAGCCCGTTCCC |
| 28S_30_556 | TGCACGTCAGGACCGCTACGGACCTCCACC |
| 28S_30_954 | AGCGGATTCCGACTTCCATGGCCACCGTCC |
| 28S_30_608 | AGCTTCGCCCCATTGGCTCCTCAGCCAAGC |
| 28S_30_971 | GATCGCAGCGAGGGAGCTGCTCTGCTACGT |
| 18S_30_311 | GAACGGCCATGCACCACCACCCACGGAATC |
| 18S_30_446 | TCTCGGGTGGCTGAACGCCACTTGTCCCTC |
| 18S_30_164 | GGGTCAGCGCCCGTCGGCATGTATTAGCTC |
| 28S_30_191 | TCCTCCCTGAGCTCGCCTTAGGACACCTGC |
| 18S_30_400 | GGAATCGAACCCTGATTCCCCGTCACCCGT |
| 18S_30_679 | ACGGGCGGTGTGTACAAAGGGCAGGGACTT |
| 28S_30_827 | TACGGATCCGGCTTGCCGACTTCCCTTACC |
| 18S_30_46 | ACCGGCCGTGCGTACTTAGACATGCATGGC |
| 28S_30_716 | GTCATAGTTACTCCCGCCGTTTACCCGCGC |
| 18S_30_837 | GATCCTTCCGCAGGTTCACCTACGGAAACC |
| 28S_30_484 | TCACGACGGTCTAAACCCAGCTCACGTTCC |
| 28S_30_273 | GGCCCCGCTTTCACGGTCTGTATTCGTACT |
| 28S_30_328 | GTACTTGTTGACTATCGGTCTCGTGCCGGT |
| 28S_30_368 | GGAACCCTTCTCCACTTCGGCCTTCAAAGT |
| 28S_30_401 | ACCCGTTTACCTCTTAACGGTTTCACGCCC |
| 18S_30_017 | GAACCTCCGACTTTCGTTCTTGATTAATGA |
| 28S_50_116 | GCCCCGCCGGCCGCCCGGCGGCCGCCGCGCGGCCCCTGCCGCCCCGAC |
| 28S_50_823 | GCCCCGCCGCCGCCGCCACCGCCGCCGCCGCCGCCGCCCCGACCCGCGC |
| 28S_50_463 | GGACCGGCCCGCGGCCCCTCCGCCGCCTGCCGCCGCCGCCGCCGCGCGCC |
| 28S_50_353 | GCCCCGCCCCGCCGCCCGCCGACCGCCGCCGCCCGACCGCTCCCGCCCCC |
| 28S_50_113 | GTCCGCCCCGCCCCCGACCCGCGCGCGGCACCCCCCCCGTCGCCGGGGC |
| 28S_50_779 | ACCCCGGTCCCGGCGCGCGGCGGGGCACGCGCCCTCCCGCGGCGGGGCGC |
| 28S_50_569 | GGCCCCGCCCGCCCACCCCCGCACCCGCCGGAGCCCGCCCCCTCCGGGGA |
| 28S_50_969 | GGCGGCCCGCAGGCCGCGGACCCCGCCCCGGGCCCCTCGCGGGGACACC |
| 28S_50_094 | GCCGCCCTCCGACGCACACCACACGCGCGCGCGCGCGCCGCCCCCGCC |
| 28S_50_904 | GGGGCGCGCGCCTCGTCCAGCCGCGGCGCGCGCCCAGCCCCGCTTCGCGC |
| 18S_50_235 | AGCCGCCGGCGCCCGCCCCCCGGCCGGGGCCGGAGAGGGGCTGACCGGGT |
| 18S_50_690 | GGGCGGGGACGGGCGGTGGCTCGCCTCGCGGCGGACCGCCCGCCCGCTCC |
| 28S_50_189 | GGGCCCGGCTCGCGTCCAGAGTCCGCGCCGCCGCCGGCCCCCCGGGTCCC |
| 28S_50_097 | GGCACTGTCCCCGGAGCGGGTCGCGCCCGGCCGGGCGGGCGCTTGGCGCC |

(continued)

| Table 2. Blocker sequences | |
|---|---|
| 28S_50_030 | GCGCCGTGGGAGGGGTGGCCCGGCCCCCCCACGAGGAGACGCCGGCGCGC |
| 28S_50_434 | TCCACCTCCCCGGCGCGGCGGGCGAGACGGGCCGGTGGTGCGCCCTCGGC |
| 28S_50_687 | TCCCCGCCGGGCCTTCCCAGCCGTCCCGGAGCCGGTCGCGGCGCACCGCC |
| 28S_50_534 | GTCGGCTGCTAGGCGCCGGCCGAGGCGAGGCGCGCGCGGAACCGCGGCCC |
| 28S_50_207 | GGGCGCGCGCCGGCCGCTACCGGCCTCACACCGTCCACGGGCTGGGCCTC |
| 28S_50_187 | GGGACGCGCGCGTGGCCCCGAGAGAACCTCCCCCGGGCCCGACGGCGCGA |
| 28S_50_461 | GGCGGGAAAGATCCGCCGGGCCGCCGACACGGCCGGACCCGCCGCCGGGT |
| 18S_50_750 | GACCGTCTTCTCAGCGCTCCGCCAGGGCCGTGGGCCGACCCCGGCGGGGC |
| 28S_50_131 | AGCGGCGCCGGGGAGCGGGTCTTCCGTACGCCACATGTCCCGCGCCCCGC |
| 28S_50_240 | GGCTCACCGCAGCGGCCCTCCTACTCGTCGCGGCGTAGCGTCCGCGGGGC |
| 28S_50_916 | GCGCGGCGACGGGTCTCGCTCCCTCGGCCCCGGGATTCGGCGAGTGCTGC |
| 28S_50_360 | ACCTCGGCCGGCGAGCGCGCCGGCCTTCACCTTCATTGCGCCACGGCGGC |
| 28S_50_442 | GGCCGAGGGCAACGGAGGCCATCGCCCGTCCCTTCGGAACGGCGCTCGCC |
| 28S_50_678 | GAGGCCAACCGAGGCTCCGCGGCGCTGCCGTATCGTTCGCCTGGGCGGGA |
| 28S_50_2665 | AGCTCACCGGACGCCGCCGGAACCGCGACGCTTTCCAAGGCACGGGCCCC |
| 28S_50_1259 | TCAAGACGGGTCGGGTGGGTAGCCGACGTCGCCGCCGACCCCGTGCGCTC |
| 28S_50_2560 | TCTCCCCGGGGCTCCCGCCGGCTTCTCCGGGATCGGTCGCGTTACCGCAC |
| 18S_50_1085 | GCTGCCCGGCGGGTCATGGGAATAACGCCGCCGCATCGCCGGTCGGCATC |
| 28S_50_1796 | GTGGCCCACTAGGCACTCGCATTCCACGCCCGGCTCCACGCCAGCGAGCC |
| 28S_50_2020 | GCGACGGCCGGGTATGGGCCCGACGCTCCAGCGCCATCCATTTTCAGGGC |
| 18S_50_1509 | GGGTAGGCACACGCTGAGCCAGTCAGTGTAGCGCGCGTGCAGCCCCGGAC |
| 28S_50_1875 | GGGGTCTGATGAGCGTCGGCATCGGGCGCCTTAACCCGGCGTTCGGTTCA |
| 28S_50_3635 | GCACTGGGCAGAAATCACATCGCGTCAACACCCGCCGCGGGCCTTCGCGA |
| 28S_50_2757 | GAGGCTGTTCACCTTGGAGACCTGCTGCGGATATGGGTACGGCCCGGCGC |
| 18S_50_469 | TCGTCACTACCTCCCCGGGTCGGGAGTGGGTAATTTGCGCGCCTGCTGCC |
| 28S_50_4889 | GAATGGTTTAGCGCCAGGTTCCCCACGAACGTGCGGTGCGTGACGGGCGA |
| 28S_50_1646 | GCGTCGGGTCTGCGAGAGCGCCAGCTATCCTGAGGGAAACTTCGGAGGGA |
| 28S_50_1557 | ACCCAGGTCGGACGACCGATTTGCACGTCAGGACCGCTACGGACCTCCAC |
| 18S_50_376 | TCGAACCCTGATTCCCCGTCACCCGTGGTCACCATGGTAGGCACGGCGAC |
| 28S_50_3831 | TTCCCCGCTGATTCCGCCAAGCCCGTTCCCTTGGCTGTGGTTTCGCTGGA |
| 18S_50_903 | GCGGCGCAATACGAATGCCCCCGGCCGTCCCTCTTAATCATGGCCTCAGT |
| 18S_50_1223 | GGGCCGGGTGAGGTTTCCCGTGTTGAGTCAAATTAAGCCGCAGGCTCCAC |
| 18S_50_827 | GGTCCTATTCCATTATTCCTAGCTGCGGTATCCAGGCGGCTCGGGCCTGC |
| 18S_50_595 | GCTATTGGAGCTGGAATTACCGCGGCTGCTGGCACCAGACTTGCCCTCCA |
| 28S_50_4172 | GTCCTCCCTGAGCTCGCCTTAGGACACCTGCGTTACCGTTTGACAGGTGT |
| 18S_50_1307 | TCGCTCCACCAACTAAGAACGGCCATGCACCACCACCCACGGAATCGAGA |
| 28S_50_4967 | GGGCTGACTTTCAATAGATCGCAGCGAGGGAGCTGCTCTGCTACGTACGA |
| 28S_50_1948 | GTTACACACTCCTTAGCGGATTCCGACTTCCATGGCCACCGTCCTGCTGT |

(continued)

| Table 2. Blocker sequences | |
|---|---|
| 28S_50_4602 | ATCCCACAGATGGTAGCTTCGCCCCATTGGCTCCTCAGCCAAGCACATAC |
| 18S_50_46 | GCCATTCGCAGTTTCACTGTACCGGCCGTGCGTACTTAGACATGCATGGC |
| 18S_50_1669 | GGTAGTAGCGACGGGCGGTGTGTACAAAGGGCAGGGACTTAATCAACGCA |
| 28S_50_390 | GCGGACCCCACCCGTTTACCTCTTAACGGTTTCACGCCCTCTTGAACTCT |
| 28S_50_2312 | TTGAATATTTGCTACTACCACCAAGATCTGCACCTGCGGCGGCTCCACCC |
| 28S_50_2832 | TTAGAGCCAATCCTTATCCCGAAGTTACGGATCCGGCTTGCCGACTTCCC |
| 28S_50_288 | TCGTGCCGGTATTTAGCCTTAGATGGAGTTTACCACCCGCTTTGGGCTGC |
| 28S_50_3718 | GGCATTTGGCTACCTTAAGAGAGTCATAGTTACTCCCGCCGTTTACCCGC |
| 28S_50_4472 | AACCTGTCTCACGACGGTCTAAACCCAGCTCACGTTCCCTATTAGTGGGT |
| 18S_50_144 | GGGTCAGCGCCCGTCGGCATGTATTAGCTCTAGAATTACCACAGTTATCC |
| 28S_50_4252 | GCCCCGCTTTCACGGTCTGTATTCGTACTGAAAATCAAGATCAAGCGAGC |
| 28S_50_9 | TCCTCCGCTGACTAATATGCTTAAATTCAGCGGGTCGCCACGTCTGATCT |
| 18S_50_1438 | GTTATTGCTCAATCTCGGGTGGCTGAACGCCACTTGTCCCTCTAAGAAGT |
| 28S_50_1723 | TGAGAATAGGTTGAGATCGTTTCGGCCCCAAGACCTCTAATCATTCGCTT |
| 18S_50_1003 | GTCTTCGAACCTCCGACTTTCGTTCTTGATTAATGAAAACATTCTTGGCA |

**Table 3** provides examples of full construct barcodes.

| Table 3 Full construct barcodes | |
|---|---|
| P7 no UMI | CAAGCAGAAGACGGCATACGAGATXXXXXXGTXXXXXXGTGACTGGAGTTCAGACGTGTGCTCTTCCGATCTrGrGrG |
| P7 early UMI | CAAGCAGAAGACGGCATACGAGATNNNNNNNNNNXXXXXXGTXXXXXXGTGACTGGAGTTCAGACGTGTGCTCTTCCGATCTrGrGrG |
| P5 no UMI | AATGATACGGCGACCACCGAGATCTACACXXXXXXGTXXXXXXACACTCTTTCCCTACACGACGCTCTTCCGATCTrGrGrG |
| P5 early UMI | AATGATACGGCGACCACCGAGATCTACACNNNNNNNNNNXXXXXXGTXXXXXXACACTCTTTCCCTACACGACGCTCTTCCGATCTrGrGrG |
| R1 inline no UMI | CTACACGACGCTCTTCCGATCTXXXXXXGTXXXXXXrGrGrG |
| R1 inline late UMI | CTACACGACGCTCTTCCGATCTXXXXXXGTXXXXXXNNNNNNNNNNrGrGrG |
| R1 late UMI AT rich | CTACACGACGCTCTTCCGATCTXXXXXXGTXXXXXX(N1:25252525)N1N1N1N1(N2:40101040)N2N2WWrGrGrG |
| R1 inline early UMI | CTACACGACGCTCTTCCGATCTNNNNNNNNNNXXXXXXGTXXXXXXrGrGrG |
| R1 inline late UMI Spacer 2 | CTACACGACGCTCTTCCGATCTXXXXXXGTXXXXXXNNNNNNNNNNATrGrGrG |

(continued)

| Table 3 Full construct barcodes | |
|---|---|
| R1 inline late UMI Spacer 4 | CTACACGACGCTCTTCCGATCTXXXXXXGTXXXXXXNNNNNNNNNNACATrGrGrG |
| R1 inline late UMI Spacer 6 | CTACACGACGCTCTTCCGATCTXXXXXXGTXXXXXXNNNNNNNNNTAACATrGrGrG |
| R1 inline late UMI Spacer 8 | CTACACGACGCTCTTCCGATCTXXXXXXGTXXXXXXNNNNNNNNNCGTAACATrGrGrG |
| R1 inline late UMI Spacer 10 | CTACACGACGCTCTTCCGATCTXXXXXXGTXXXXXXNNNNNNNNNACCGTAACATrGrGrG |
| R1 inline late UMI Full Spacer | CTACACGACGCTCTTCCGATCTXXXXXXGTXXXXXXNNNNNNNNNACACAAGAGGCACGCGTAACATrGrGrG |

X represents nucleotides that make up the barcode sequence. All oligos on one bead can have the same barcode sequence, oligos on different beads may have different barcode sequences.

N and W represent any of {A,C,G,T} and any of {A,T} respectively that make up the UMI sequence. UMIs may be different across different oligos on the same bead.

N1 is any one of {A,C,G,T}; N1 positions with ratios of 25%, 25%, 25% and 25% for the four nucleotides.

N2 is any one of {A,C,G,T}; N2 positions with ratios of 40%, 10%, 10% and 40% for the four nucleotides.

In some examples, a cell barcode may be a 16 base sequence that is a random choice from about 737,000 sequences. The length of the barcode (16) can be altered. The diversity of potential barcode sequences (737k) can be alterable. The defined nature of the barcode can be altered, for example, it may also be completely random (16 Ns) or semi-random (16 bases that come from a biased distribution of nucleotides).

The canonical UMI sequence may be a 10 nucleotide randomer. The length of the UMI can be altered. The random nature of the UMI can be altered, for example, it may be semi-random (bases that come from a biased distribution of nucleotides.) In a certain case, the distribution of UMI nucleotide(s) may be biased; for example, UMI sequences that do not contain Gs or Cs may be less likely to serve as primers.

The spacer may alterable within given or predetermined parameters. For example one method may give an optimal sequence of TTTCTTATAT, but using a slightly different optimization strategy results in a sequence that is likely just as or nearly as good.

The selected template switching region can comprise 3 consecutive riboGs or more. The selected template switching region can comprise 4, 5, 6, 7, 8, 9, 10, 11, 12,13, 14, 15, 16, 17, 18, 19, 20 consecutive riboGs or more. Alternative nucleotide may be used such as deoxyribo Gs, LNA G's, and potentially any combination thereof.

The present invention comprises the step of enriching cDNA sequences. Enrichment may be useful for TCR and BCR gene analysis since these genes may possess similar yet polymorphic variable region sequences. These sequences can be responsible for antigen binding and peptide-MHC interactions. For example, due to gene recombination events in individual developing T cells, a single human or mouse will naturally express many thousands of different TCR genes. This T cell repertoire can exceed 100,000 or more different TCR rearrangements occurring during T cell development, yielding a total T cell population that is highly polymorphic with respect to its TCR gene sequences especially for the variable region. As previously noted, each distinct sequence may correspond to a clonotype. In certain embodiments, enrichment increases accuracy and sensitivity of methods for sequencing TCR and BCR genes at a single cell level. In certain embodiments, enrichment increases the number of sequencing reads that map to a TCR or BCR gene. In some embodiments, enrichment leads to greater than or equal to 25%, 30%, 35%, 40%, 45%, 50%, 55%, 60%, 65%, 70%, 75%, 80%, 85%, 90%, 95% or more of total sequencing reads mapping to a TCR or BCR gene. In some embodiments, enrichment leads to greater than or equal to 25%, 30%, 35%, 40%, 45%, 50%, 55%, 60%, 65%, 70%, 75%, 80%, 85%, 90%, 95% or more of total sequencing reads mapping to a variable region of a TCR or BCR gene.

In order to aide in sequencing, detection, and analysis of sequences of interest, an enrichment step is employed. Enrichment may be useful for the sequencing and analysis of genes that may be related yet highly polymorphic. An enriched gene comprises a TCR sequence or a BCR sequence. In some embodiments, an enriched gene comprises a mitochondrial gene or a cytochrome family gene. In some embodiments, enrichment is employed after an initial round of reverse transcription (e.g., cDNA production). In some embodiments, enrichment is employed after an initial round of reverse transcription and cDNA amplification for at least 5, 10, 15, 20, 25, 30, 40 or more cycles. In some embodiments, enrichment is employed after a cDNA amplification. In some embodiments, the amplified cDNA can be subjected to a clean up step before the enrichment step using a column, gel extraction, or beads in order to remove unincorporated primers, unincorporated nucleotides, very short or very long nucleic acid fragments and enzymes. In some embodiments, enrichment is followed by a clean-up step before sequencing library preparation.

Enrichment of gene or cDNA sequences can be facilitated by a primer that anneals within a known sequence of the target gene. For enrichment of a TCR or BCR gene, a primer that anneals to a constant region of the gene or cDNA can be paired with a sequencing primer that anneals to a TSO functional sequence. In some embodiments, the enriched cDNA falls into a length range that approximately corresponds to that genes variable region. In some embodiments, greater than about 50%, 60%, 70%, 80%, 85%, 90%, 95 % or more cDNA or cDNA fragments fall within a range of about 300 base pairs to about 900 base pairs, of about 400 base pairs to about 800 base pairs, of about 500 base pairs to about 700 base pairs, or of about 500 base pairs to about 600 base pairs.

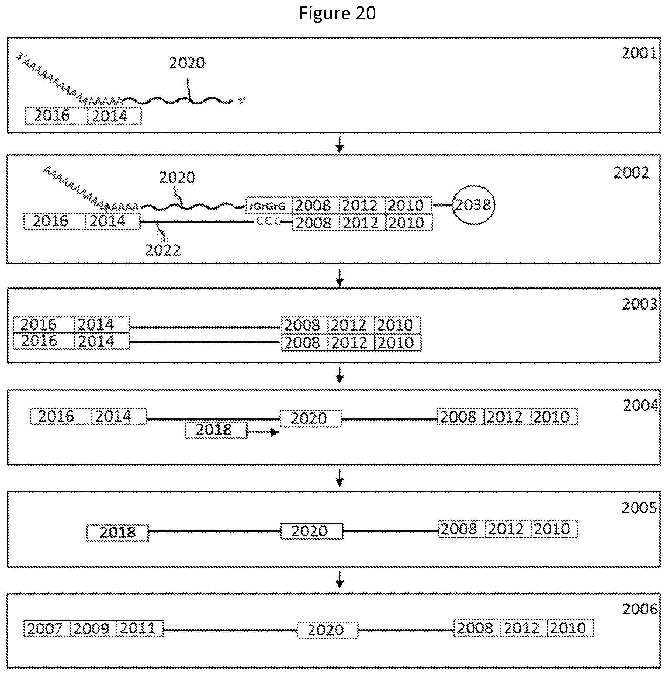

Figure 20 shows an example enrichment scheme. In operation 2001, an oligonucleotide with a poly-T sequence 2014, and in some cases an additional sequence 2016 that binds to, for example, a sequencing or PCR primer, anneals to a target RNA 2020. In operation 2002 the oligonucleotide is extended yielding an anti-sense strand 2022 which is appended by multiple cytidines on the 3' end. A template switching oligonucleotide attached to a gel bead 2038 is provided and a riboG of the TSO pairs with the cytidines of the sense strand and is extended to create a sense and an antisense strand. In some cases, the template switching oligonucleotide is released from the gel bead during extension. In some cases, the template switching oligonucleotide is released from the gel bead prior to extension. In some cases, the template switching oligonucleotide is released from the gel bead after extension. In addition to the riboG sequence, the TSO comprises a barcode 2012 and one or two additional functional sequences 2008 and 2012. The additional functional sequences can comprise a P7 or R2 sequence for attachment to an Illumina sequencing flow cell, for example. Operations 2001 and 2002 may be performed in a partition (e.g., droplet or well). Subsequent to operation 2002, the nucleic acid product from operations 2001 and 2002 may be removed from the partition and in some cases pooled with other products from other partitions for subsequent processing.

Next, additional functional sequences can be added that allow for amplification or sample identification. This may occur in a partition or in bulk. This reaction yields amplified cDNA molecules as in 2003 which are mixed templates comprising a barcode and sequencing primers. In some cases, not all of these cDNA molecules will comprise a target variable region sequence. In one enrichment scheme, shown in operation 2004, a primer 2018 that anneals to a sequence 3' of a TCR or BCR variable region 2020 specifically amplifies the variable region comprising cDNAs yielding products as shown in operation 2005. Such enrichment may be performed for various approaches described herein, such as, e.g., the approaches described above in the context of Figures 19A and 19B.

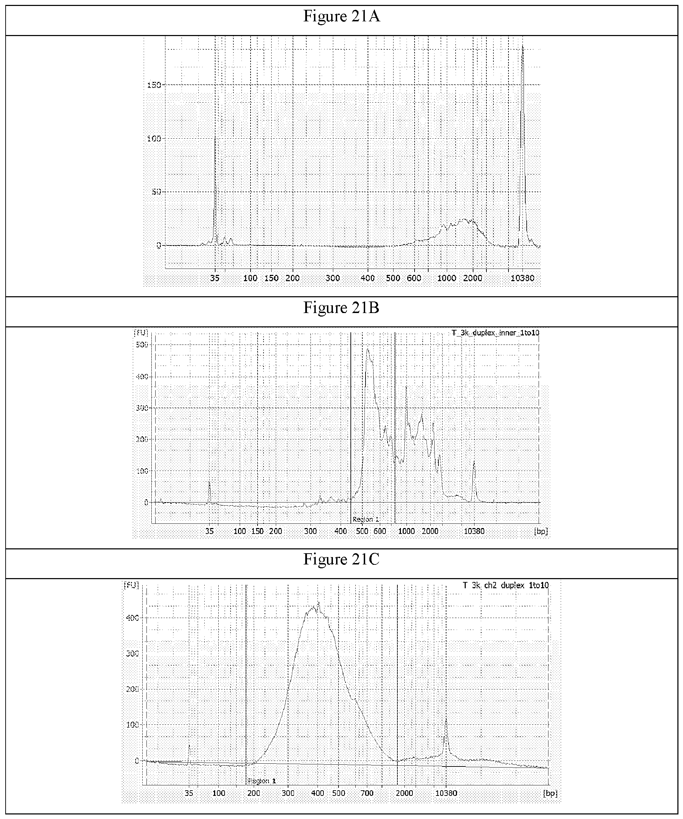

In certain aspects, primer 2018 anneals in a constant region of a TCR (e.g., TCR-alpha or TCR-beta) or BCR gene. After amplification the products are sheared, adaptors ligated and amplified a second time to add additional functional sequences 2007 and 2011 and a sample index 2009 as shown in operation 2006. The additional functional sequences can functionally complement the first pair 2008 and 2010 and comprise for example a P5 or R1 sequence. Figure 21 shows example size distributions after cDNA amplification but before enrichment (A), after enrichment but before sequencing library prep (B), and after sequencing library preparation (C). In some embodiments, the initial poly-T primer, comprising sequences 2016 and 2014 can be attached to a gel bead as opposed to the TSO. In some embodiments, the poly-T comprising primer comprises functional sequences and barcode sequences 2008, 2010, 2012, and the TSO comprises sequence 2016. Operations 2003-2006 may be performed in bulk.

In some embodiments, clonotype information derived from next-generation sequencing data of cDNA prepped from cellular RNA is combined with other targeted on non targeted cDNA enrichment to illuminate functional and ontological aspects of B-cell and T cells that express a given TCR or BCR. In some embodiments, clonotype information is combined with analysis of expression of an immunologically relevant cDNA. In some embodiments, the cDNA encodes a cell lineage marker, a cell surface functional marker, a cytokine and/or chemokine, an intracellular signaling polypeptide, a cell metabolism polypeptide, a cell-cycle polypeptide, an apoptosis polypeptide, a transcriptional activator/inhibitor, an miRNA or lncRNA.

Also disclosed herein are methods and systems for reference-free clonotype identification. Such methods may be implemented by way of software executing algorithms. Tools for assembling T-cell Receptor (TCR) sequences may use known sequences of V and C regions to "anchor" assemblies. This may make such tools only applicable to organisms with well characterized references (human and mouse). However, most mammalian T cell receptors have similar amino acid motifs and similar structure. In the absence of a reference, a method can scan assembled transcripts for regions that are diverse or semi-diverse, find the junction region which should be highly diverse, then scan for known amino acid motifs. In some cases, it may not be critical that the complementary CDRs, such as the CDR1, CDR2, or CDR3, region be accurately delimited, only that a diverse sequence is found that can uniquely identify the clonotype. One advantage of this method is that the software may not require a set of reference sequences and can operate fully *de novo,* thus this method can enable immune research in eukaryotes with poorly characterized genomes/transcriptomes.

The methods described herein allow simultaneously obtaining single-cell gene expression information with single-cell immune receptor sequences (TCRs/BCRs). This can be achieved using the methods described herein, such as by amplifying genes relevant to lymphocyte function and state (either in a targeted or unbiased way) while simultaneously amplifying the TCR/BCR sequences for clonotyping. This can allow such applications as 1) interrogating changes in lymphocyte activation/response to an antigen, at the single clonotype or single cell level; or 2) classifying lymphocytes into subtypes based on gene expression while simultaneously sequencing their TCR/BCRs. UMIs are typically ignored during TCR (or generally transcriptome) assembly.

Key analytical operations involved in clonotype sequencing according to the methods described herein include: 1) Assemble each UMI separately, then merge highly similar assembled sequences. High depth per molecule in TCR sequencing makes this feasible. This may result in a reduced chance of "chimeric" assemblies; 2) Assemble all UMIs from each cell together but use UMI information to choose paths in the assembly graph. This is analogous to using barcode and read-pair information to resolve "bubbles" in WGS assemblies; 3) Base quality estimation. UMI information and alignment of short reads may be used to assemble contigs to compute per-base quality scores. Base quality scoring may be important as a few base differences in a CDR sequence may differentiate one clonotype from another. This may be in contrast to other methods that rely on using long-read sequencing.

Thus, base quality estimates for assembled contigs can inform clonotype inference. Errors can make cells with the same (real) clonotype have mismatching assembled sequences. Further, combining base-quality estimates and clonotype abundances to correct clonotype assignments. For example, if 10 cells have clonotype X and one cell has a clonotype that differs by X in only a few bases and these bases have low quality, then this cell may be assigned to clonotype X. In some embodiments, clonotypes that differ by a single amino acid or nucleic acid may be discriminated. In some embodiments, clonotypes that differ by less than 50, 40, 30, 20, 15, 10, 9, 8, 7, 6, 5, 4, 3, or 2 amino acids or nucleic acids may be discriminated. An example, non limiting, base error calculation scheme is shown below in Example VII.

Also provided herein are the microfluidic devices used for partitioning the cells as described above. Such microfluidic devices can comprise channel networks for carrying out the partitioning process like those set forth in Figures 1 and 2. Briefly, these microfluidic devices can comprise channel networks, such as those described herein, for partitioning cells into separate partitions, and co-partitioning such cells with oligonucleotide barcode library members, e.g., disposed on beads. These channel networks can be disposed within a solid body, e.g., a glass, semiconductor or polymer body structure in which the channels are defined, where those channels communicate at their termini with reservoirs for receiving the various input fluids, and for the ultimate deposition of the partitioned cells, etc., from the output of the channel networks. By way of example, and with reference to Figure 2, a reservoir fluidly coupled to channel 202 may be provided with an aqueous suspension of cells 214, while a reservoir coupled to channel 204 may be provided with an aqueous suspension of beads 216 carrying the oligonucleotides. Channel segments 206 and 208 may be provided with a non-aqueous solution, e.g., an oil, into which the aqueous fluids are partitioned as droplets at the channel junction 212. Finally, an outlet reservoir may be fluidly coupled to channel 210 into which the partitioned cells and beads can be delivered and from which they may be harvested. As will be appreciated, while described as reservoirs, it will be appreciated that the channel segments may be coupled to any of a variety of different fluid sources or receiving components, including tubing, manifolds, or fluidic components of other systems.

Also provided are systems that control flow of these fluids through the channel networks e.g., through applied pressure differentials, centrifugal force, electrokinetic pumping, capillary or gravity flow, or the like.

Also provided herein are kits for analyzing individual cells or small populations of cells. The kits may include one, two, three, four, five or more, up to all of partitioning fluids, including both aqueous buffers and non-aqueous partitioning fluids or oils, nucleic acid barcode libraries that are releasably associated with beads, as described herein, microfluidic devices, reagents for disrupting cells amplifying nucleic acids, and providing additional functional sequences on fragments of cellular nucleic acids or replicates thereof, as well as instructions for using any of the foregoing in the methods described herein.

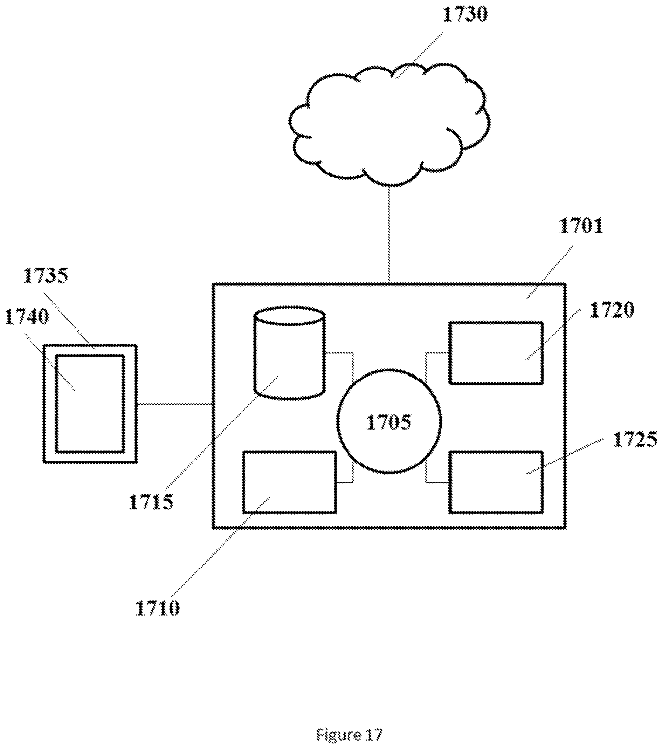

The present disclosure provides computer control systems that are programmed to implement methods of the disclosure. Figure 17 shows a computer system 1701 that is programmed or otherwise configured to implement methods of the disclosure including nucleic acid sequencing methods, interpretation of nucleic acid sequencing data and analysis of cellular nucleic acids, such as RNA (e.g., mRNA), and characterization of cells from sequencing data. The computer system 1701 can be an electronic device of a user or a computer system that is remotely located with respect to the electronic device. The electronic device can be a mobile electronic device.

The computer system 1701 includes a central processing unit (CPU, also "processor" and "computer processor" herein) 1705, which can be a single core or multi core processor, or a plurality of processors for parallel processing. The computer system 1701 also includes memory or memory location 1710 (e.g., random-access memory, read-only memory, flash memory), electronic storage unit 1715 (e.g., hard disk), communication interface 1720 (e.g., network adapter) for communicating with one or more other systems, and peripheral devices 1725, such as cache, other memory, data storage and/or electronic display adapters. The memory 1710, storage unit 1715, interface 1720 and peripheral devices 1725 are in communication with the CPU 1705 through a communication bus (solid lines), such as a motherboard. The storage unit 1715 can be a data storage unit (or data repository) for storing data. The computer system 1701 can be operatively coupled to a computer network ("network") 1730 with the aid of the communication interface 1720. The network 1730 can be the Internet, an internet and/or extranet, or an intranet and/or extranet that is in communication with the Internet. The network 1730 in some cases is a telecommunication and/or data network. The network 1730 can include one or more computer servers, which can enable distributed computing, such as cloud computing. The network 1730, in some cases with the aid of the computer system 1701, can implement a peer-to-peer network, which may enable devices coupled to the computer system 1701 to behave as a client or a server.

The CPU 1705 can execute a sequence of machine-readable instructions, which can be embodied in a program or software. The instructions may be stored in a memory location, such as the memory 1710. The instructions can be directed to the CPU 1705, which can subsequently program or otherwise configure the CPU 1705 to implement methods of the present disclosure. Examples of operations performed by the CPU 1705 can include fetch, decode, execute, and writeback.

The CPU 1705 can be part of a circuit, such as an integrated circuit. One or more other components of the system 1701 can be included in the circuit. In some cases, the circuit is an application specific integrated circuit (ASIC).

The storage unit 1715 can store files, such as drivers, libraries and saved programs. The storage unit 1715 can store user data, e.g., user preferences and user programs. The computer system 1701 in some cases can include one or more additional data storage units that are external to the computer system 1701, such as located on a remote server that is in communication with the computer system 1701 through an intranet or the Internet.

The computer system 1701 can communicate with one or more remote computer systems through the network 1730. For instance, the computer system 1701 can communicate with a remote computer system of a user. Examples of remote computer systems include personal computers (e.g., portable PC), slate or tablet PC's (e.g., Apple® iPad, Samsung® Galaxy Tab), telephones, Smart phones (e.g., Apple® iPhone, Android-enabled device, Blackberry®), or personal digital assistants. The user can access the computer system 1701 via the network 1730.

Methods as described herein can be implemented by way of machine (e.g., computer processor) executable code stored on an electronic storage location of the computer system 1701, such as, for example, on the memory 1710 or electronic storage unit 1715. The machine executable or machine readable code can be provided in the form of software. During use, the code can be executed by the processor 1705. In some cases, the code can be retrieved from the storage unit 1715 and stored on the memory 1710 for ready access by the processor 1705. In some situations, the electronic storage unit 1715 can be precluded, and machine-executable instructions are stored on memory 1710.

The code can be pre-compiled and configured for use with a machine having a processer adapted to execute the code, or can be compiled during runtime. The code can be supplied in a programming language that can be selected to enable the code to execute in a pre-compiled or as-compiled fashion.

Aspects of the systems and methods provided herein, such as the computer system 1701, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 1701 can include or be in communication with an electronic display 1735 that comprises a user interface (UI) 1740 for providing, for example, results of nucleic acid sequencing, analysis of nucleic acid sequencing data, characterization of nucleic acid sequencing samples, cell characterizations, etc. Examples of UI's include, without limitation, a graphical user interface (GUI) and web-based user interface.

Methods and systems of the present disclosure can be implemented by way of one or more algorithms. An algorithm can be implemented by way of software upon execution by the central processing unit 1705. The algorithm can, for example, initiate nucleic acid sequencing, process nucleic acid sequencing data, interpret nucleic acid sequencing results, characterize nucleic acid samples, characterize cells, etc.

## EXAMPLES

The following non-limiting examples are given for the purpose of illustrating various embodiments of present disclosure.

Example I: Cellular RNA analysis using emulsions

In an example, reverse transcription with template switching and cDNA amplification (via PCR) is performed in emulsion droplets with operations as shown in Figure 9A. The reaction mixture that is partitioned for reverse transcription and cDNA amplification (via PCR) includes 1,000 cells or 10,000 cells or 10 ng of RNA, beads bearing barcoded oligonucleotides/0.2% Tx-100/5x Kapa buffer, 2x Kapa HS HiFi Ready Mix, 4 μM switch oligo, and Smartscribe. Where cells are present, the mixture is partitioned such that a majority or all of the droplets comprise a single cell and single bead. The cells are lysed while the barcoded oligonucleotides are released from the bead, and the poly-T segment of the barcoded oligonucleotide hybridizes to the poly-A tail of mRNA that is released from the cell as in operation 950. The poly-T segment is extended in a reverse transcription reaction as in operation 952 and the cDNA transcript is amplified as in operation 954. The thermal cycling conditions are 42 °C for 130 minutes; 98 °C for 2 min; and 35 cycles of the following 98 °C for 15 sec, 60 °C for 20 sec, and 72 °C for 6 min. Following thermal cycling, the emulsion is broken and the transcripts are purified with Dynabeads and 0.6x SPRI as in operation 956.

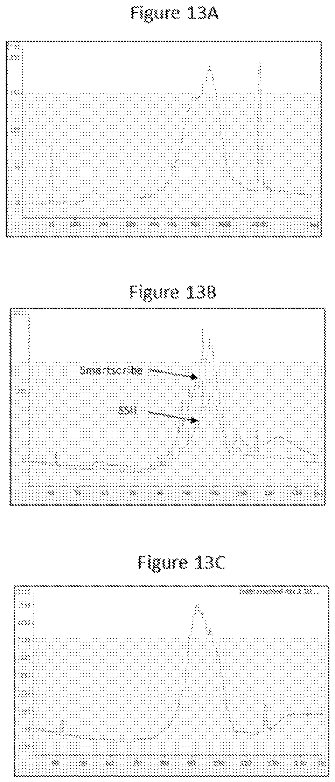

The yield from template switch reverse transcription and PCR in emulsions is shown for 1,000 cells in Figure 13A and 10,000 cells in Figure 13C and 10 ng of RNA in Figure 13B (Smartscribe line). The cDNA transcripts from RT and PCR performed in emulsions for 10 ng RNA is sheared and ligated to functional sequences, cleaned up with 0.8x SPRI, and is further amplified by PCR as in operation 958. The amplification product is cleaned up with 0.8x SPRI. The yield from this processing is shown in Figure 13B (SSII line).

Example II: Cellular RNA analysis using emulsions

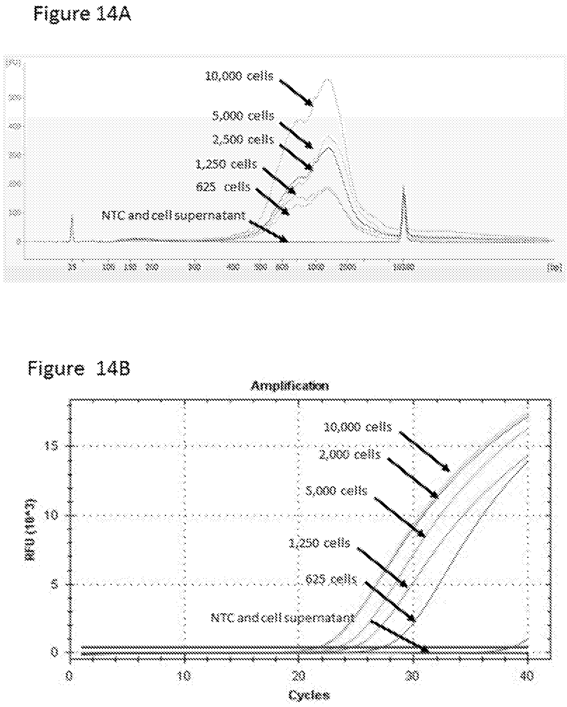

In another example, reverse transcription with template switching and cDNA amplification (via PCR) is performed in emulsion droplets with operations as shown in Figure 9A. The reaction mixture that is partitioned for reverse transcription and cDNA amplification (via PCR) includes Jurkat cells, beads bearing barcoded oligonucleotides/0.2% TritonX-100/5x Kapa buffer, 2x Kapa HS HiFi Ready Mix, 4 μM switch oligo, and Smartscribe. The mixture is partitioned such that a majority or all of the droplets comprise a single cell and single bead. The cells are lysed while the barcoded oligonucleotides are released from the bead, and the poly-T segment of the barcoded oligonucleotide hybridizes to the poly-A tail of mRNA that is released from the cell as in operation 950. The poly-T segment is extended in a reverse transcription reaction as in operation 952 and the cDNA transcript is amplified as in operation 954. The thermal cycling conditions are 42 °C for 130 minutes; 98 °C for 2 min; and 35 cycles of the following 98 °C for 15 sec, 60 °C for 20 sec, and 72 °C for 6 min. Following thermal cycling, the emulsion is broken and the transcripts are cleaned-up with Dynabeads and 0.6x SPRI as in operation 956. The yield from reactions with various cell numbers (625 cells, 1,250 cells, 2,500 cells, 5,000 cells, and 10,000 cells) is shown in Figure 14A. These yields are confirmed with GADPH qPCR assay results shown in Figure 14B.

Example III: RNA analysis using emulsions

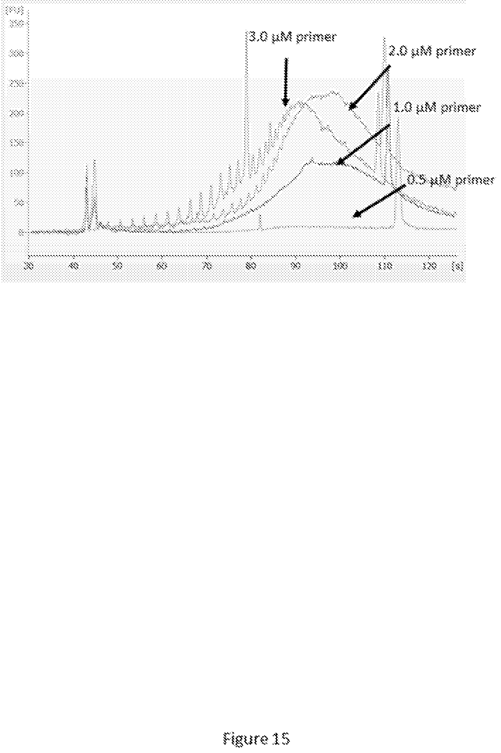

In another example, reverse transcription is performed in emulsion droplets and cDNA amplification is performed in bulk in a manner similar to that as shown in Figure 9C. The reaction mixture that is partitioned for reverse transcription includes beads bearing barcoded oligonucleotides, 10 ng Jurkat RNA (e.g., Jurkat mRNA), 5x First-Strand buffer, and Smartscribe. The barcoded oligonucleotides are released from the bead, and the poly-T segment of the barcoded oligonucleotide hybridizes to the poly-A tail of the RNA as in operation 961. The poly-T segment is extended in a reverse transcription reaction as in operation 963. The thermal cycling conditions for reverse transcription are one cycle at 42 °C for 2 hours and one cycle at 70 °C for 10 min. Following thermal cycling, the emulsion is broken and RNA and cDNA transcripts are denatured as in operation 962. A second strand is then synthesized by primer extension with a primer having a biotin tag as in operation 964. The reaction conditions for this primer extension include cDNA transcript as the first strand and biotinylated extension primer ranging in concentration from 0.5 - 3.0 μM. The thermal cycling conditions are one cycle at 98 °C for 3 min and one cycle of 98 °C for 15 sec, 60 °C for 20 sec, and 72 °C for 30min. Following primer extension, the second strand is pulled down with Dynabeads MyOne Streptavidin C1 and T1, and cleaned-up with Agilent SureSelect XT buffers. The second strand is pre-amplified via PCR as in operation 965 with the following cycling conditions - one cycle at 98 °C for 3 min and one cycle of 98 °C for 15 sec, 60 °C for 20 sec, and 72 °C for 30 min. The yield for various concentrations of biotinylated primer (0.5 μM, 1.0 μM, 2.0 μM, and 3.0 μM) is shown in Figure 15.

Example IV: RNA analysis using emulsions

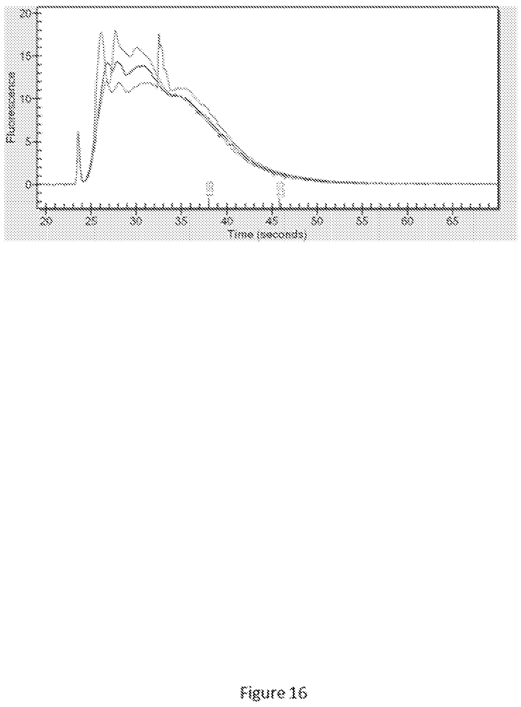

In another example, in vitro transcription by T7 polymerase is used to produce RNA transcripts as shown in Figure 10. The mixture that is partitioned for reverse transcription includes beads bearing barcoded oligonucleotides which also include a T7 RNA polymerase promoter sequence, 10 ng human RNA (e.g., human mRNA), 5x First-Strand buffer, and Smartscribe. The mixture is partitioned such that a majority or all of the droplets comprise a single bead. The barcoded oligonucleotides are released from the bead, and the poly-T segment of the barcoded oligonucleotide hybridizes to the poly-A tail of the RNA as in operation 1050. The poly-T segment is extended in a reverse transcription reaction as in operation 1052. The thermal cycling conditions are one cycle at 42 °C for 2 hours and one cycle at 70 °C for 10 min. Following thermal cycling, the emulsion is broken and the remaining operations are performed in bulk. A second strand is then synthesized by primer extension as in operation 1054. The reaction conditions for this primer extension include cDNA transcript as template and extension primer. The thermal cycling conditions are one cycle at 98 °C for 3 min and one cycle of 98 °C for 15 sec, 60 °C for 20 sec, and 72 °C for 30min. Following this primer extension, the second strand is purified with 0.6x SPRI. As in operation 1056, in vitro transcription is then performed to produce RNA transcripts. In vitro transcription is performed overnight, and the transcripts are purified with 0.6x SPRI. The RNA yields from in vitro transcription are shown in Figure 16.

Example V: Analysis of T-cell Receptors (TCRs)

In this example, methods disclosed herein are used to assay T-cell receptors. To generate labeled polynucleotides comprising T-cell receptor gene sequences, T-cells are co-partitioned with gel beads comprising barcoded template switching oligonucleotides. Prior to partitioning, T-cells are optionally enriched from a cell sample, for example by fluorescence activated cell sorting (FACS) or other sorting technique. Additional reagents for generating labeled polynucleotides including, but not limited to, reverse transcriptase enzyme, poly(dT) primer, and dNTPs, are delivered to partitions as part of a master mix. Within partitions, cells are lysed, thereby yielding template polynucleotides comprising nucleic acids from T-cells. As illustrated schematically in Figure 19, a T-cell derived template polynucleotide comprising mRNA (e.g., 1920), poly(dT) primer (e.g., 1924), and a template switching oligonucleotide (e.g., 1926) are subjected to an amplification reaction to yield a first amplification product. The poly(dT) primer hybridizes to the polyA tail of the mRNA template polynucleotide and acts as a primer for reverse transcription by the reverse transcriptase enzyme that is co-partitioned with the T-cell (e.g., 1950). The reverse transcriptase enzyme has terminal transferase activity and adds additional nucleotides, e.g., polyC, to the cDNA transcript in a template independent manner. The template switching oligonucleotide (e.g., 1926) hybridizes to the cDNA transcript and facilitates template switching in the first amplification reaction (e.g., 1952).

Using methods disclosed herein, reverse transcription performed within partitions generates unbiased cDNA comprising a sequencing adapter, a cell barcode and a unique molecular identifier (UMI) on the 5' end of the transcript. To enrich for transcripts comprising TCR gene sequences, the first amplification reaction product or cDNA transcript is subjected to a second amplification reaction to generate a second amplification product. Polymerase chain reaction (PCR) is performed with one primer for the 5' end of the transcript and one or more primers for the desired TCR/Ig constant region(s) (e.g., primers targeting TCR alpha (α) and/or beta (β) chain, and in some cases gamma and/or delta (γ/δ) chains). The contents of multiple partitions can be combined such that the second amplification reaction is performed in bulk.

Next, amplification products are subjected to enzymatic fragmentation and further processed to attach sequencing adaptors to generate a sequencing library. Additional sequences include functional sequences such as flow cell attachment sequences and sequencing primer binding sequences. The labeled polynucleotides are sequenced to yield sequencing reads, and sequencing reads are used to assemble full or partial TCR receptor gene sequences. Additional analysis includes transcript counting for which an analysis pipeline may include, for example, (i) barcode processing, (ii) read filtering, (iii) cell-by-cell consensus assembly, (iv) V(D)J annotation, and (v) clonotype inference and clustering.

Other receptors (e.g., B-cell receptors (BCRs) and Ig receptors) can be similarly analyzed using the methods described herein by partitioning the appropriate immune cell type for generating labeled polynucleotides and using receptor specific primers to generate amplification products.

Example VI: Enrichment of T-cell Receptor (TCR) transcripts

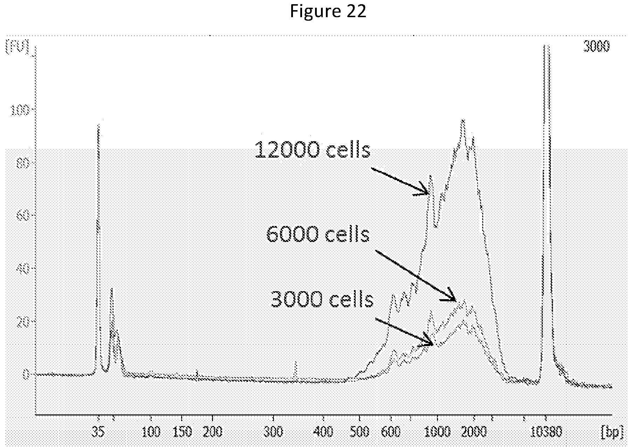

In this example, cellular suspensions of 3,000; 6,000; or 12,000 primary human T cells were loaded on a GemCode Single Cell Instrument (10x Genomics, Pleasanton, CA) to generate single cell-gel bead emulsions (SC-GEMs). The gel beads were modified to carry a template switching oligonucleotide (TSO) as shown in Figure 18 or Figure 20 at 8 μM, yielding a final concentration of 0.32 μM in GEM. After creation of SC-GEMs, reverse transcription was performed on the cells in emulsion using a poly-T primer and reverse transcriptase for 5 minutes at 55°C, followed by 1 hour and 55 minutes at 52°C. After RT, GEMs were broken and the single-stranded cDNA was cleaned up with DynaBeads® MyOne™ Silane Beads and SPRIselect Reagent Kit (0.6X SPRI). cDNA was amplified for 15 cycles with 1 minute extensions and amplified cDNA product was cleaned up with the SPRIselect Reagent Kit (0.6X SPRI). Figure 22 shows the cDNA yields from all three cellular suspensions. cDNA yields from 12,000 cells was greater than either 6,000 or 3,000 cells, which yielded similar amounts.

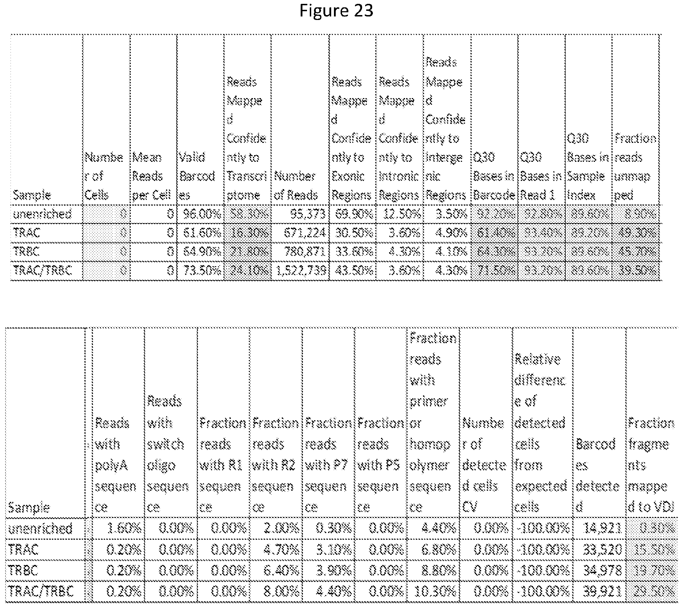

Indexed sequencing libraries were constructed using the GemCode Single Cell 3' Library Kit, following these steps: 1) end repair and A-tailing; 2) adapter ligation; 3) post-ligation cleanup with SPRIselect; 4) sample index PCR and cleanup. These sequencing libraries were sequenced using an Illumina MiSeq sequencer. Sequencing performance of poly-T primed libraries was compared to libraries constructed from enriched cDNA libraries created by using an enriched priming method which substituted the poly-T primer with primers that bound the constant region of TCRα, TCRβ, or both. Figure 23 shows that enrichment led to reduced mapping of sequencing reads to the transcriptome (8.9% unmappable reads with poly-T priming versus 49.3% for TCRα priming, 45.7% for TCRβ priming, or 39% for both). However, more reads mapped to the VDJ regions of TCR transcripts, indicating that enrichment is important for targeted VDJ sequencing (0.3% VDJ mappable fragments with poly T priming versus 15.5% for TCRα priming, 19.7% for TCRβ priming, or 29.50% for both). *See* Figure 23, Fraction fragments mapped to VDJ column.

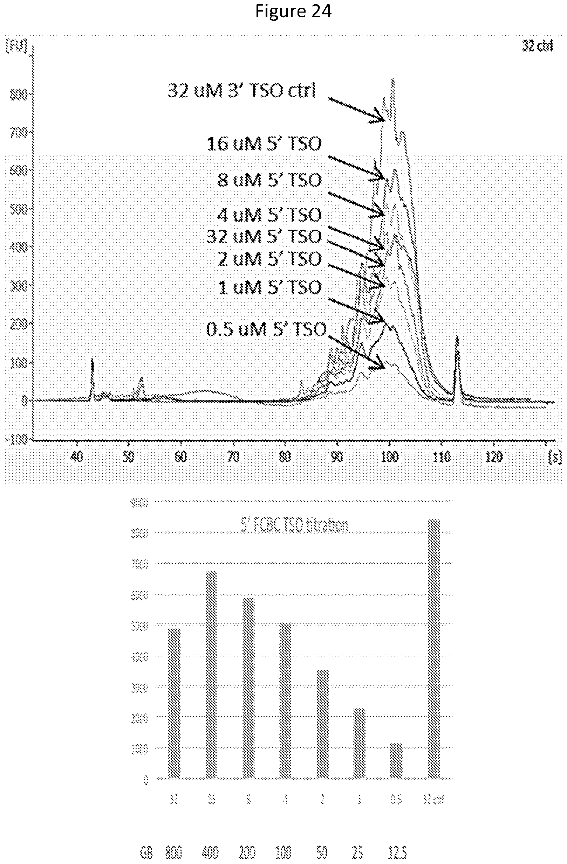

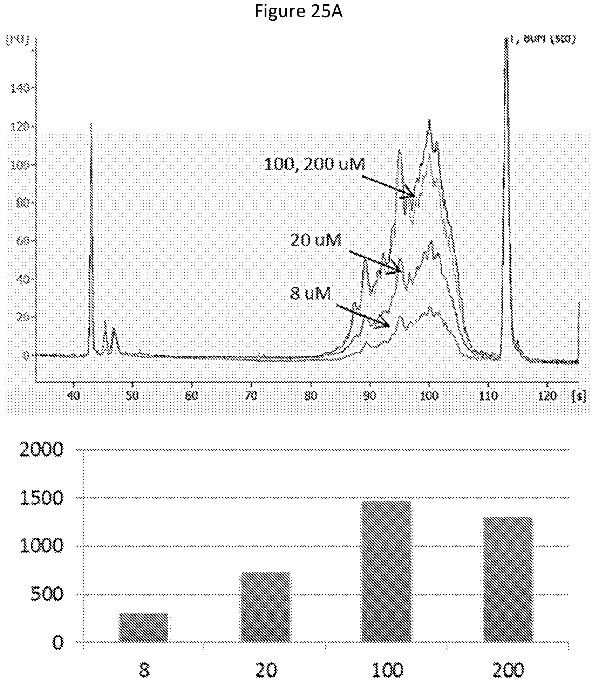

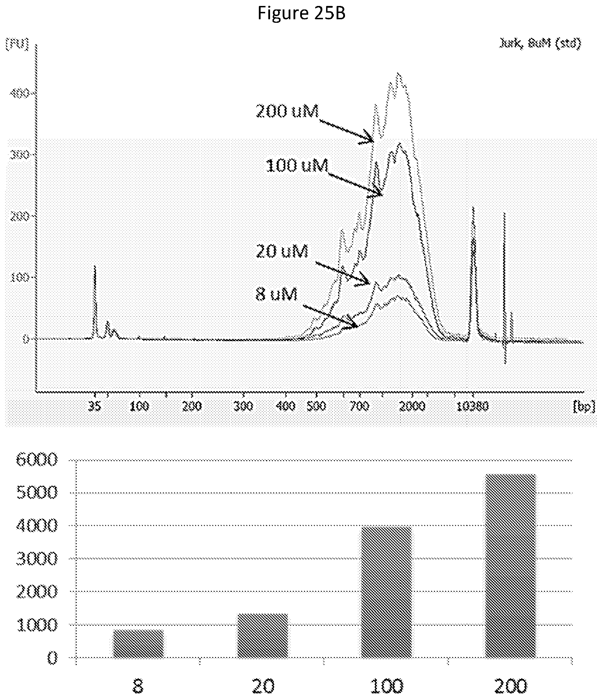

In order to increase cDNA yields prior to sequencing library preparation, differing concentrations of TSO were tested. TSO were tested at concentrations of 32, 16, 8, 4, 2, 1, and 0.5 μM (which may correspond to 800, 400, 200, 100, 50, 25, and 12.5 μM immobilized to gel beads). Jurkat T cells were used for this experiment, and results are shown in Figure 24 with cDNA yields directly correlating with TSO concentration and plateauing at about a concentration of 16 μM. These experiments were repeated with TSO immobilized to gel beads (GB-TSTO) using either 6,000 primary T cells as shown in Figure 25A, or 2,200 Jurkat cells as shown in figure 25B. GB-TSO concentrations of 8, 20, 100, and 200 μM were tested and concentrations of 100 and 200 μM showed a significant increase over the lower concentrations of 8 and 20 μM.

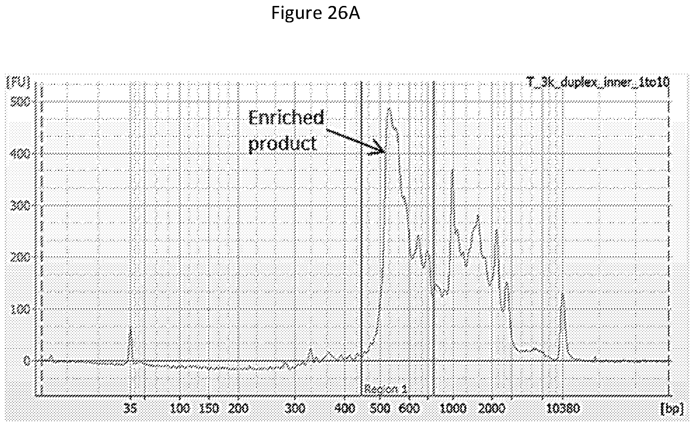

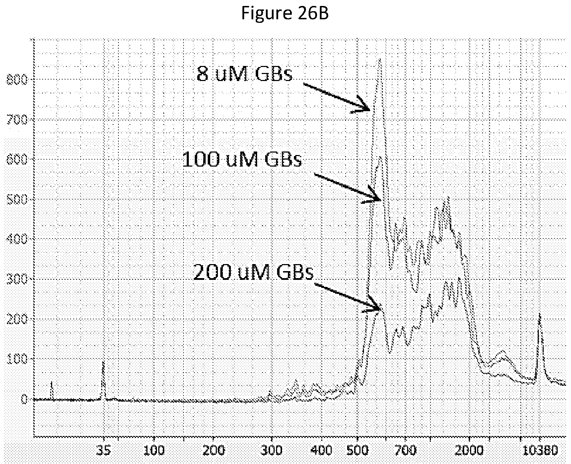

Differing enrichment schemes were tested to determine optimal enrichment methods. Using a non-GEM protocol, cDNA from 3,000 primary T cells was prepared using poly-T priming followed by enrichment using primers that anneal to TCR constant regions, yielding 38.5% VDJ mappable reads, quantitation of this enrichment is shown in Figure 26A. Using a gel-beads in emulsion-reverse transcription reaction (GEM-RT) protocol, cDNA from 6,000 primary T cells was prepared using Poly-T priming and gel beads with a TSO at 8, 100 or 200 μM concentration followed by enrichment using a two stage nested approach. This nested enrichment comprised PCR using outer primers annealing to the TCR alpha and beta paired with a P7 primer for 10 cycles using 60° C extensions, followed by PCR using inner primers annealing to the TCR alpha and beta paired with a P7 primer for 10 cycles using 60° C extensions. Results of this are shown in Figure 26B with the largest amount of enrichment exhibited using a lower concentration of gel beads (8 μM).

To further optimize enrichment, a comparison was conducted between using P7 primers and variable region specific primers in combination with the constant region primers for cDNA amplification. Primer sequences used are shown in Table 4.

| Table 4 Enrichment primer sequences and predicted products | | |
|---|---|---|
| | Primer | Sequence |
| V region primers | TRA-V1 | ACTTGTCCAGCCTAACCTGC |
| | TRA-V2 | TTACCCTGGGAGGAACCAGA |
| | TRB-V1 | TTTCAGGCCACAACTCCCTT |
| | TRB-V2 | CAGACAGACCATGATGCGGG |
| | TRB-V3 | GCCACAACTCCCTTTTCTGG |
| Constant region primers | TRAC-inner | AGTCTCTCAGCTGGTACACG |
| | TRBC-inner | TCTGATGGCTCAAACACAGC |
| Predicted amplicon size | TRA-V1/TRAC-inner | 554 |
| | TRA-V2/TRAC-inner | 413 |
| | TRB-V1/TRBC-inner | 324 |
| | TRB-V2/TRBC-inner | 296 |
| | TRB-V3/TRBC-inner | 318 |

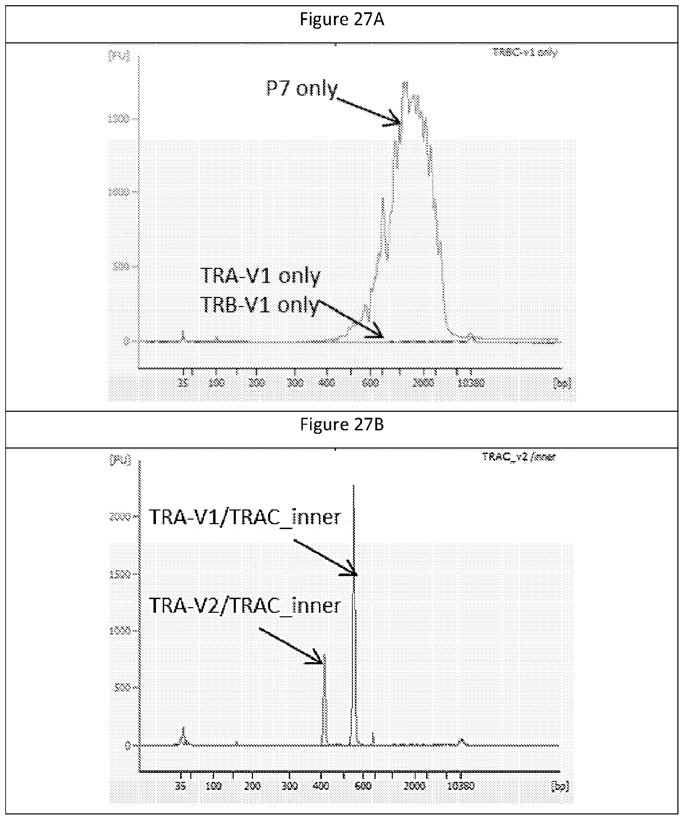

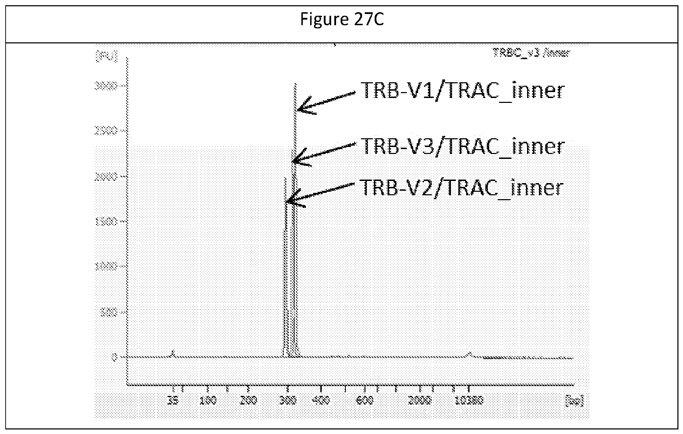

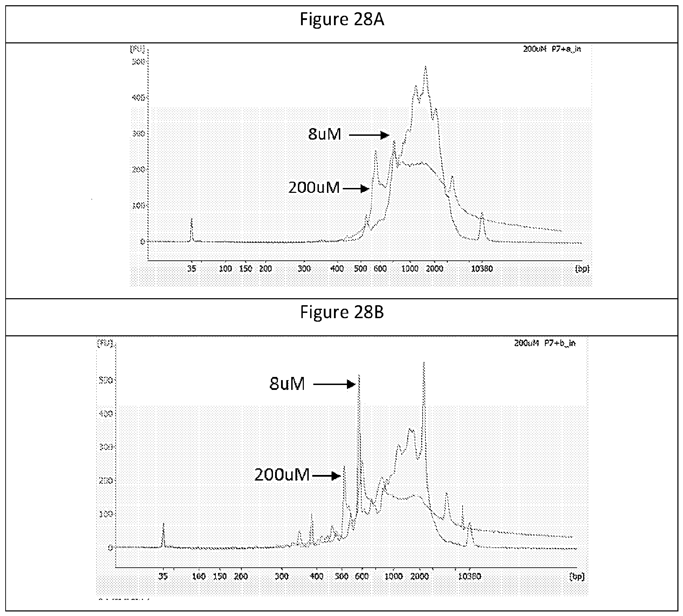

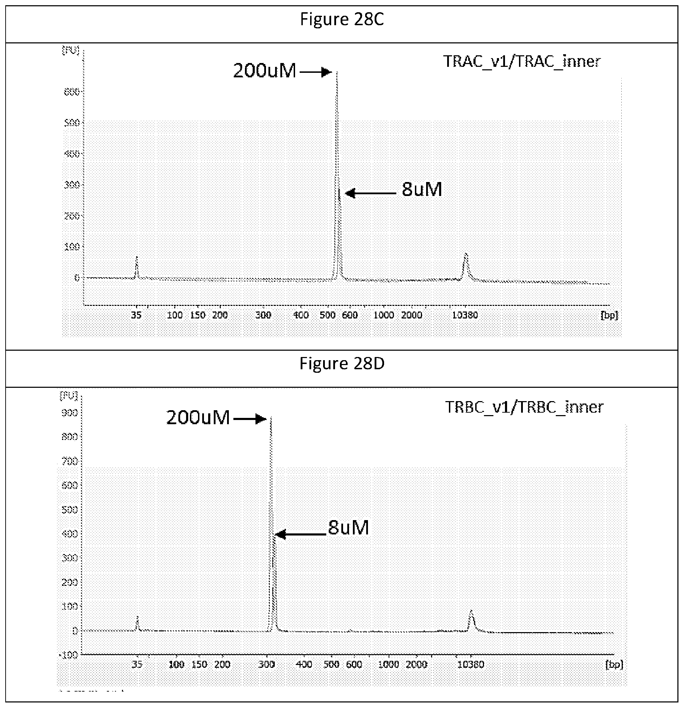

GEM-RT was carried out using poly-T priming followed by a template switch using 8μM TSO-GB, followed by clean up, 15 cycles of cDNA amplification and enrichment for 20 cycles. Results shown in Figures 27A -27C show that using V region primers in conjunction with constant region primers specifically enrich TCR alpha (Figure 27B) and TCR beta sequences (Figure 27C) compared to P7 primers paired with constant region primers. Figure 28 further shows that by using specific enrichment (28C and D; V region + C region primers) compared to general enrichment (28A and B; P7 primer + C region primer) yields of specifically enriched product were increased by increasing the amount of TSO-GB (from 8μM to 200μm). This is in contrast to what was observed using P7-constant region primer enrichment which required using less TSO-GB (8μM) in the GEM-RT reaction to produce more enriched product. Overall using the P7-constant region enrichment allows preservation of barcode information in the subsequent sequencing reaction. This configuration yields at least 30% reads mappable to VDJ genes.

Example VII: Generating labeled polynucleotides

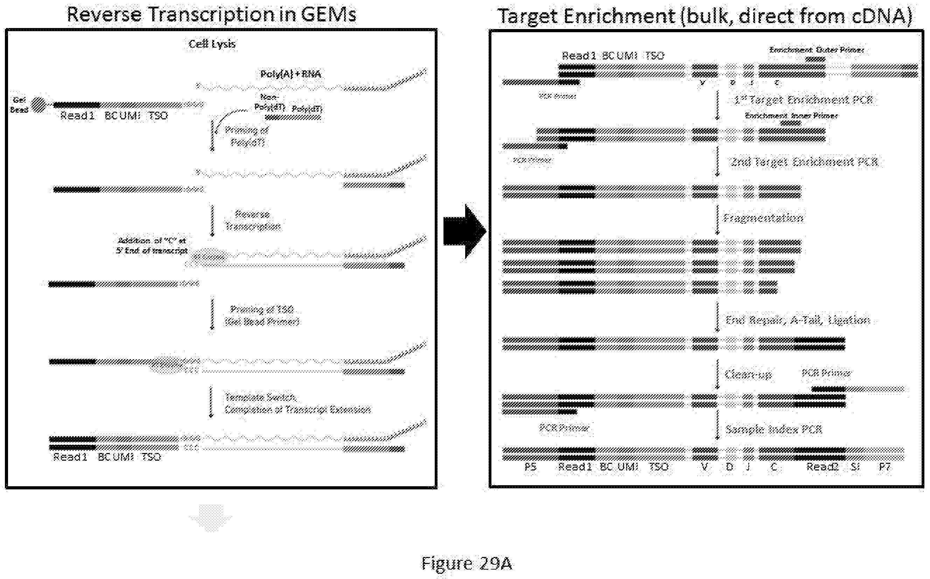

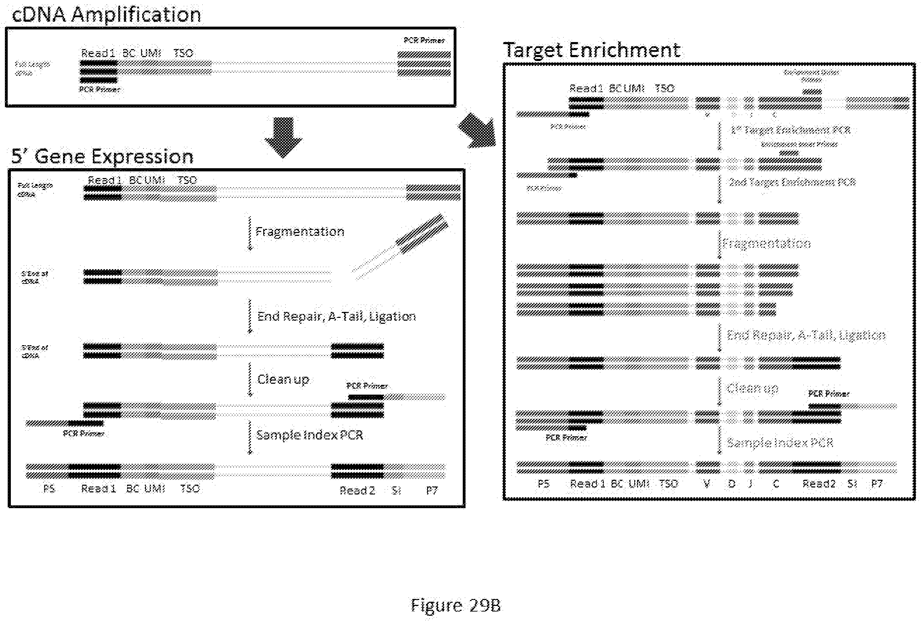

In this example, and with reference to Figures 29A and 29B, individual cells are lysed in partitions comprising gel bead emulsions (GEMs). GEMs, for example, can be aqueous droplets comprising gel beads. Within GEMs, a template polynucleotide comprising an mRNA molecule can be reverse transcribed by a reverse transcriptase and a primer comprising a poly(dT) region. A template switching oligo (TSO) present in the GEM, for example a TSO delivered by the gel bead, can facilitate template switching so that a resulting polynucleotide product or cDNA transcript from reverse transcription comprises the primer sequence, a reverse complement of the mRNA molecule sequence, and a sequence complementary to the template switching oligo. The template switching oligo can comprise additional sequence elements, such as a unique molecular identifier (UMI), a barcode sequence (BC), and a Read1 sequence. See Figure 29A. In some cases, a plurality of mRNA molecules from the cell is reverse transcribed within the GEM, yielding a plurality of polynucleotide products having various nucleic acid sequences. Following reverse transcription, the polynucleotide product can be subjected to target enrichment in bulk. Prior to target enrichment, the polynucleotide product can be optionally subjected to additional reaction(s) to yield double-stranded polynucleotides. The target may comprise VDJ sequences of a T cell and/or B cell receptor gene sequence. As shown at the top of the right panel of Figure 29A, the polynucleotide product (shown as a double-stranded molecule, but can optionally be a single-stranded transcript) can be subjected to a first target enrichment polymerase chain reaction (PCR) using a primer that hybridizes to the Read 1 region and a second primer that hybridizes to a first region of the constant region (C) of the receptor sequence (e.g., TCR or BCR). The product of the first target enrichment PCR can be subjected to a second, optional target enrichment PCR. In the second target enrichment PCR, a second primer that hybridizes to a second region of the constant region (C) of the receptor can be used. This second primer can, in some cases, hybridize to a region of the constant region that is closer to the VDJ region that the primer used in the first target enrichment PCR. Following the first and second (optional) target enrichment PCR, the resulting polynucleotide product can be further processed to add additional sequences useful for downstream analysis, for example sequencing. The polynucleotide products can be subjected to fragmentation, end repair, A-tailing, adapter ligation, and one or more clean-up/purification operations.

In some cases, a first subset of the polynucleotide products from cDNA amplification can be subjected to target enrichment (Figure 29B, right panel) and a second subset of the polynucleotide products from cDNA amplification is not subjected to target enrichment (Figure 29B, bottom left panel). The second subset can be subjected to further processing without enrichment to yield an unenriched, sequencing ready population of polynucleotides. For example, the second subset can be subjected to fragmentation, end repair, A-tailing, adapter ligation, and one or more clean-up/purification operations.

The labeled polynucleotides can then be subjected to sequencing analysis. Sequencing reads of the enriched polynucleotides can yield sequence information about a particular population of the mRNA molecules in the cell whereas the unenriched polynucleotides can yield sequence information about various mRNA molecules in the cell.

Example VIII: Base error calculations

All calculations of this example are for a single base. The terms transcript and UMI will be used inter-changeably, assuming that there is a 1-1 relationship between transcripts and UMIs. Let D be all observed data (reads, qualities, UMIs) at a given base and $D_u$, $u$ = 1, ..., $m$ be the data from UMI $u$. Let $R$ be the real template base at the given position and $T_u$ be the (unobserved) base at the given position on transcipt/UMI $u$. Let $R_{ui}$ and $_{Rui}$ be the real (pre-sequencing errors) and observed (post-sequencing errors) bases on the $i$th read of UMI $u$ and $_{Qui}$ be the corresponding base quality. Let $_{prt}$ be probability of an RT error, and $p_{pcr}$ be the probability of a PCR error. Let also $p_s(Q)$ = 10-Q/10 be the probability of a sequencing error given a base quality of Q. Finally, let $L$ = {A, C, G, T}. Transcripts are conditionally independent given the real template base and reads from a transcript are conditionally independent given the base of the transcript (i.e. errors occur completely independently of one another). Below, Equation I can be derived by summing over the unobserved value c of transcript $u$ at the given position and the (also unobserved) real value $d$ of each read at this position.

## Equation I

$$P(D|R) =$$
$$\prod P(D_u | R)$$
$$\prod_u \sum_{c \in L} \left[ \left(\frac{p_{rt}}{3}\right)^{R \neq c} (1 - p_{rt})^{R=c} \prod_i \left(\frac{p_s(Q_{u_i})}{3}\right)^{\bar{R}_{u_i} \neq c} (1 - p_s(Q_{u_i}))^{\bar{R}_{u_i}=c} \right]$$

Let X be the called base at that position (i.e. the base in the assembled sequence). The probability of an error is:

$$P(R \neq X|D) = 1 - P(R = X|D) = 1 - \frac{P(D|R = X)P(R = X)}{P(D)} =$$
$$1 - \frac{0.25 * P(D|c)}{\sum_{c \in L} 0.25 * P(D|c)}$$

If it is assumed that $p_{pcr}$ is negligible (compared to sequencing and RT errors), that is the sequenced base $R_{ui}$ always equals the transcript base $T_u$, the simplified form of Equation II can be derived below.

## Equation II

$$\prod_u \sum_{c \in L} \left[ \left(\frac{p_{rt}}{3}\right)^{R \neq c} (1 - p_{rt})^{R=c} \prod_i \left(\frac{p_s(Q_{u_i})}{3}\right)^{\bar{R}_{u_i} \neq c} (1 - p_s(Q_{u_i}))^{\bar{R}_{u_i}=c} \right]$$

Let X be the called base at that position (i.e. the base in the assembled sequence). The probability of an error is:

$$P(R \neq X|D) = 1 - P(R = X|D) = 1 - \frac{P(D|R = X)P(R = X)}{P(D)} =$$

$$1 - \frac{0.25 * P(D|c)}{\sum_{c \in L} 0.25 * P(D|c)}$$

Devices, systems, compositions and methods of the present disclosure may be used for various applications, such as, for example, processing a single analyte (e.g., RNA, DNA, or protein) or multiple analytes (e.g., DNA and RNA, DNA and protein, RNA and protein, or RNA, DNA and protein) form a single cell. For example, a biological particle (e.g., a cell or cell bead) is partitioned in a partition (e.g., droplet), and multiple analytes from the biological particle are processed for subsequent processing. The multiple analytes may be from the single cell. This may enable, for example, simultaneous proteomic, transcriptomic and genomic analysis of the cell.

While some embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention.

## Claims

1. A method for nucleic acid sequencing, comprising:

   (a) providing a plurality of partitions, wherein a partition of said plurality of partitions comprises (i) a ribonucleic acid (RNA) molecule from an immune cell, wherein the RNA molecule comprises a nucleic acid sequence of a T cell receptor or a B cell receptor, and (ii) a bead comprising a nucleic acid barcode molecule coupled thereto, wherein said nucleic acid barcode molecule comprises a barcode sequence and a primer sequence;
   (b) using said RNA molecule and said nucleic acid barcode molecule to generate a barcoded nucleic acid molecule comprising, from a 5' end to a 3' end, said barcode sequence and a sequence corresponding to said nucleic acid sequence of said RNA molecule, wherein the generating comprises (i) hybridizing said nucleic acid barcode molecule to said RNA molecule, (ii) extending said nucleic acid barcode molecule using a reverse transcriptase having terminal transferase activity to generate a nucleic acid product comprising a reverse complement of a sequence of said RNA molecule and a sequence at its 3' end that is complementary to a portion of a template switching oligonucleotide, (iii) hybridizing said nucleic acid product to said template switching oligonucleotide, and (iv) extending said nucleic acid product using said template switching oligonucleotide as a template;
   (c) using said barcoded nucleic acid molecules, or a derivative thereof, and a pair of primers to generate a subset of nucleic acids having a TCR or BCR target nucleic acid sequence to provide an enriched subset of barcoded nucleic acid molecules or derivatives thereof; and
   (d) sequencing said enriched subset of barcoded nucleic acid molecules, or derivatives thereof, and an unenriched subset of barcoded nucleic acid molecules, or derivatives thereof.

2. A method according to claim 1, wherein said nucleic acid barcode molecule is releasably coupled to the bead.

3. A method according to claim 1 or claim 2, wherein said bead is a gel bead or a magnetic bead.

4. A method according to claim 2 or claim 3, wherein said bead comprises a plurality of nucleic acid barcode molecules releasably coupled thereto, wherein each of said plurality of nucleic acid barcode molecules comprises said barcode sequence, and wherein each of said plurality of nucleic acid barcode molecules optionally comprises a unique molecular identifier sequence segment.

5. A method according to claim 2, wherein said nucleic acid barcode molecule is released from said bead upon application of a stimulus; wherein said stimulus optionally includes one or more members selected from the group consisting of a biological stimulus, a chemical stimulus, a thermal stimulus, an electrical stimulus, a magnetic stimulus, and a photo stimulus; and wherein said chemical stimulus is optionally a reducing agent.

6. A method according to any one of claims 1 to 5, wherein said partition comprises a single cell and the method comprises lysing the single cell in said partition to obtain said RNA molecule.

7. A method according to claim 6, wherein said nucleic acid barcode molecule is releasably coupled to a bead and the method comprises releasing the nucleic acid barcode molecule from the bead.

8. A method according to any one of claims 1 to 7, wherein said partition is a droplet or a well.

9. A method according to any one of claims 1 to 8, wherein:

   (i) said primer sequence comprises a gene specific primer sequence, wherein the target regions comprise TCR and/or BCR regions, e.g. a constant region of said TCR and/or BCR region;
   (ii) said primer sequence comprises a polyT sequence; and/or
   (iii) nucleic acid barcode molecules included on an individual bead include multiple types of primer sequences in addition to common barcode sequences.

10. A method according to any one of claims 1 to 9, wherein the nucleic acid barcode molecule further comprises a functional sequence (e.g. a sequencing primer sequence) and/or an additional sequence (e.g. a unique molecular identifier sequence).

11. A method according to any one of claims 1 to 10, further comprising, prior to (d), subjecting said barcoded nucleic acid molecule or derivative thereof to nucleic acid amplification, wherein said nucleic acid amplification is performed subsequent to releasing said barcoded nucleic acid molecule or derivative thereof from said partition.

12. A method according to any one of claims 1 to 11, wherein the nucleic acid sequence of the RNA comprises the sequence of:

   (a) a TCR alpha chain, a TCR beta chain, a TCR delta chain, a TCR gamma chain, or any fragment thereof, for example variable regions including VDJ or VJ regions, constant regions, transmembrane regions, fragments thereof, combinations thereof, and combinations of fragments thereof; and/or
   (b) a B cell receptor heavy chain, a B cell receptor light chain, or any fragment thereof, for example variable regions including VDJ or VJ regions, constant regions, transmembrane regions, fragments thereof, combinations thereof, and combinations of fragments thereof.

13. A method according to any one of claim 1 to 12, wherein said primer sequence comprises a polyT sequence and wherein said target nucleic acid sequence comprises a T cell receptor variable region sequence or a B cell receptor variable region sequence.

14. A method according to any one of claims 1 to 13, wherein at least one of said pair of primers hybridizes to a constant region of a T cell receptor nucleic acid sequence, a constant region of a B cell receptor nucleic acid sequence, or a constant region of an immunoglobulin nucleic acid sequence.

15. A method according to any one of claims 1 to 14, further comprising adding a further functional sequence to said enriched subset of barcoded nucleic acid molecules or derivatives thereof.

16. A method according to any one of claims 1 to 15, wherein the sequence at the 3' end that is complementary to a portion of a template switching oligonucleotide is a polyC sequence

**Patentansprüche**

1. Verfahren zur Nukleinsäuresequenzierung, umfassend:

   (a) Bereitstellen einer Mehrzahl von Partitionen, wobei eine Partition der Mehrzahl von Partitionen (i) ein Ribonukleinsäuremolekül (RNA-Molekül) aus einer Immunzelle, wobei das RNA-Molekül eine Nukleinsäuresequenz eines T-Zell-Rezeptors oder eines B-Zell-Rezeptors umfasst, und (ii) ein Kügelchen umfasst, das ein daran gekoppeltes Nukleinsäure-Barcode-Molekül umfasst, wobei das Nukleinsäure-Barcode-Molekül eine Barcode-Sequenz und eine Primer-Sequenz umfasst;

(b) Verwenden des RNA-Moleküls und des Nukleinsäure-Barcode-Moleküls, um ein barcodiertes Nukleinsäuremolekül zu erzeugen, das von einem 5'-Ende zu einem 3'-Ende die Barcode-Sequenz und eine Sequenz umfasst, die der Nukleinsäuresequenz des RNA-Moleküls entspricht, wobei das Erzeugen (i) das Hybridisieren des Nukleinsäure-Barcode-Moleküls mit dem RNA-Molekül, (ii) das Verlängern des Nukleinsäure-Barcode-Moleküls unter Verwendung einer reversen Transkriptase mit terminaler Transferaseaktivität, um ein Nukleinsäureprodukt zu erzeugen, das ein reverses Komplement einer Sequenz des RNA-Moleküls und eine Sequenz an seinem 3'-Ende umfasst, die komplementär zu einem Abschnitt eines Matrizenwechsel-Oligonukleotids ist, (iii) das Hybridisieren des Nukleinsäureprodukts mit dem Matrizenwechsel-Oligonukleotid und (iv) das Verlängern des Nukleinsäureprodukts unter Verwendung des Matrizenwechsel-Oligonukleotids als Matrize umfasst;

(c) Verwenden der barcodierten Nukleinsäuremoleküle oder eines Derivats davon und eines Paars von Primern, um eine Teilmenge von Nukleinsäuren mit einer TCR- oder BCR-Zielnukleinsäuresequenz zu erzeugen, um eine angereicherte Teilmenge von barcodierten Nukleinsäuremolekülen oder Derivaten davon bereitzustellen; und

(d) Sequenzieren der angereicherten Teilmenge von barcodierten Nukleinsäuremolekülen oder Derivaten davon und einer nicht angereicherten Teilmenge von barcodierten Nukleinsäuremolekülen oder Derivaten davon.

2. Verfahren nach Anspruch 1, wobei das Nukleinsäure-Barcode-Molekül lösbar an das Kügelchen gekoppelt ist.

3. Verfahren nach Anspruch 1 oder Anspruch 2, wobei das Kügelchen ein Gelkügelchen oder ein magnetisches Kügelchen ist.

4. Verfahren nach Anspruch 2 oder Anspruch 3, wobei das Kügelchen eine Mehrzahl von Nukleinsäure-Barcode-Molekülen umfasst, die lösbar daran gekoppelt sind, wobei jedes der Mehrzahl von Nukleinsäure-Barcode-Molekülen die Barcode-Sequenz umfasst und wobei jedes der Mehrzahl von Nukleinsäure-Barcode-Molekülen optional ein eindeutiges molekulares Kennungssequenzsegment umfasst.

5. Verfahren nach Anspruch 2, wobei das Nukleinsäure-Barcode-Molekül bei Anwendung eines Stimulus von dem Kügelchen gelöst wird; wobei der Stimulus optional ein oder mehrere Elemente beinhaltet, die aus der Gruppe ausgewählt sind, die aus einem biologischen Stimulus, einem chemischen Stimulus, einem thermischen Stimulus, einem elektrischen Stimulus, einem magnetischen Stimulus und einem Photostimulus besteht; und wobei der chemische Stimulus optional ein Reduktionsmittel ist.

6. Verfahren nach einem der Ansprüche 1 bis 5, wobei die Partition eine einzelne Zelle umfasst und das Verfahren das Lysieren der einzelnen Zelle in der Partition umfasst, um das RNA-Molekül zu erhalten.

7. Verfahren nach Anspruch 6, wobei das Nukleinsäure-Barcode-Molekül lösbar an ein Kügelchen gekoppelt wird und das Verfahren das Lösen des Nukleinsäure-Barcode-Moleküls von dem Kügelchen umfasst.

8. Verfahren nach einem der Ansprüche 1 bis 7, wobei die Partition ein Tröpfchen oder ein Well ist.

9. Verfahren nach einem der Ansprüche 1 bis 8, wobei:

(i) die Primer-Sequenz eine genspezifische Primer-Sequenz umfasst, wobei die Zielregionen TCR- und/oder BCR-Regionen umfassen, z. B. eine konstante Region der TCR- und/oder BCR-Region;
(ii) die Primer-Sequenz eine polyT-Sequenz umfasst; und/oder
(iii) Nukleinsäure-Barcode-Moleküle, die auf einem einzelnen Kügelchen enthalten sind, zusätzlich zu den üblichen Barcode-Sequenzen mehrere Arten von Primer-Sequenzen beihalten.

10. Verfahren nach einem der Ansprüche 1 bis 9, wobei das Nukleinsäure-Barcode-Molekül ferner eine funktionelle Sequenz (z. B. eine sequenzierende Primer-Sequenz) und/oder eine zusätzliche Sequenz (z. B. eine eindeutige molekulare Kennungssequenz) umfasst.

11. Verfahren nach einem der Ansprüche 1 bis 10, ferner umfassend vor (d) das Unterziehen des barcodierten Nukleinsäuremoleküls oder Derivats davon einer Nukleinsäureamplifikation, wobei die Nukleinsäureamplifikation nach dem Lösen des barcodierten Nukleinsäuremoleküls oder Derivats davon von der Partition durchgeführt wird.

12. Verfahren nach einem der Ansprüche 1 bis 11, wobei die Nukleinsäuresequenz der RNA die Sequenz umfasst von:

(a) einer TCR-Alpha-Kette, einer TCR-Beta-Kette, einer TCR-Delta-Kette, einer TCR-Gamma-Kette oder einem beliebigen Fragment davon, beispielsweise variablen Regionen einschließlich VDJ- oder VJ-Regionen, konstanten Regionen, Transmembranregionen, Fragmenten davon, Kombinationen davon, und Kombinationen von Fragmenten davon; und/oder
(b) einer schweren Kette des B-Zell-Rezeptors, einer leichten Kette des B-Zell-Rezeptors oder einem beliebigen Fragment davon, beispielsweise variablen Regionen einschließlich VDJ- oder VJ-Regionen, konstanten Regionen, Transmembranregionen, Fragmenten davon, Kombinationen davon und Kombinationen von Fragmenten davon.

13. Verfahren nach einem der Ansprüche 1 bis 12, wobei die Primer-Sequenz eine polyT-Sequenz umfasst und wobei die Ziel-Nukleinsäuresequenz eine variable T-Zell-Rezeptor-Region-Sequenz oder eine variable B-Zell-Rezeptor-Region-Sequenz umfasst.

14. Verfahren nach einem der Ansprüche 1 bis 13, wobei wenigstens eines der Paare von Primern an eine konstante Region einer T-Zell-Rezeptor-Nukleinsäuresequenz, eine konstante Region einer B-Zell-Rezeptor-Nukleinsäuresequenz oder eine konstante Region einer Immunglobulin-Nukleinsäuresequenz hybridisiert.

15. Verfahren nach einem der Ansprüche 1 bis 14, ferner umfassend das Addieren einer weiteren funktionellen Sequenz an die angereicherte Teilmenge von barcodierten Nukleinsäuremolekülen oder Derivaten davon.

16. Verfahren nach einem der Ansprüche 1 bis 15, wobei die Sequenz an dem 3'-Ende, die zu einem Abschnitt eines Matrizenwechsel-Oligonukleotids komplementär ist, eine polyC-Sequenz ist

## Revendications

1. Procédé de séquençage d'acide nucléique, comprenant :

(a) la fourniture d'une pluralité de compartiments, dans lequel un compartiment de ladite pluralité de compartiments comprend (i) une molécule d'acide ribonucléique (ARN) provenant d'une cellule immunitaire, dans lequel la molécule d'ARN comprend une séquence d'acide nucléique d'un récepteur des lymphocytes 1 ou d'un récepteur des lymphocytes B, et (ii) une bille comprenant une molécule de code-barres d'acide nucléique couplée à celle-ci, dans lequel ladite molécule de code-barres d'acide nucléique comprend une séquence de code-barres et une séquence d'amorce ;
(b) l'utilisation de ladite molécule d'ARN et de ladite molécule de code-barres d'acide nucléique pour générer une molécule d'acide nucléique à code-barres comprenant, d'une extrémité 5' à une extrémité 3', ladite séquence de code-barres et une séquence correspondant à ladite séquence d'acide nucléique de ladite molécule d'ARN, dans lequel la génération comprend (i) l'hybridation de ladite molécule de code-barres d'acide nucléique à ladite molécule d'ARN, (ii) l'extension de ladite molécule de code-barres d'acide nucléique en utilisant une transcriptase inverse ayant une activité de transférase terminale pour générer un produit d'acide nucléique comprenant un complément inverse d'une séquence de ladite molécule d'ARN et une séquence au niveau de son extrémité 3' qui est complémentaire d'une portion d'un oligonucléotide de commutation de matrice, (iii) l'hybridation dudit produit d'acide nucléique audit oligonucléotide de commutation de matrice, et (iv) l'extension dudit produit d'acide nucléique en utilisant ledit oligonucléotide de commutation de matrice en tant que matrice ;
(c) l'utilisation desdites molécules d'acide nucléique à code-barres, ou d'un dérivé de celles-ci, et d'une paire d'amorces pour générer un sous-ensemble d'acides nucléiques ayant une séquence cible d'acide nucléique de TCR ou BCR pour fournir un sous-ensemble enrichi de molécules d'acide nucléique à code-barres ou de dérivés de celles-ci ; et
(d) le séquençage dudit sous-ensemble enrichi de molécules d'acide nucléique à code-barres, ou de dérivés de celles-ci, et d'un sous-ensemble non enrichi de molécules d'acide nucléique à code-barres, ou de dérivés de celles-ci.

2. Procédé selon la revendication 1, dans lequel ladite molécule de code-barres d'acide nucléique est couplée de manière libérable à la bille.

3. Procédé selon la revendication 1 ou la revendication 2, dans lequel ladite bille est une bille de gel ou une bille magnétique.

4. Procédé selon la revendication 2 ou la revendication 3, dans lequel ladite bille comprend une pluralité de molécules de code-barres d'acide nucléique couplées de manière libérable à celle-ci, dans lequel chacune de ladite pluralité de molécules de code-barres d'acide nucléique comprend ladite séquence de code-barres, et dans lequel chacune de ladite pluralité de molécules de code-barres d'acide nucléique comprend facultativement un segment de séquence d'identifiant moléculaire unique.

5. Procédé selon la revendication 2, dans lequel ladite molécule de code-barres d'acide nucléique est libérée de ladite bille lors de l'application d'un stimulus ; dans lequel ledit stimulus comporte facultativement un ou plusieurs éléments choisis dans le groupe constitué par un stimulus biologique, un stimulus chimique, un stimulus thermique, un stimulus électrique, un stimulus magnétique et un photostimulus ; et dans lequel ledit stimulus chimique est facultativement un agent réducteur.

6. Procédé selon l'une quelconque des revendications 1 à 5, dans lequel ledit compartiment comprend une cellule unique et le procédé comprend la lyse de la cellule unique dans ledit compartiment pour obtenir ladite molécule d'ARN.

7. Procédé selon la revendication 6, dans lequel ladite molécule de code-barres d'acide nucléique est couplée de manière libérable à une bille et le procédé comprend la libération de la molécule de code-barres d'acide nucléique de la bille.

8. Procédé selon l'une quelconque des revendications 1 à 7, dans lequel ledit compartiment est une gouttelette ou un puits.

9. Procédé selon l'une quelconque des revendications 1 à 8, dans lequel :

   (i) ladite séquence d'amorce comprend une séquence d'amorce spécifique d'un gène, dans lequel les régions cibles comprennent des régions TCR et/ou BCR, par exemple, une région constante de ladite région TCR et/ou BCR ;
   (ii) ladite séquence d'amorce comprend une séquence polyT ; et/ou
   (iii) des molécules de code-barres d'acide nucléique incluses sur une bille individuelle comportent de multiples types de séquences d'amorces en supplément de séquences de code-barres communes.

10. Procédé selon l'une quelconque des revendications 1 à 9, dans lequel la molécule de code-barres d'acide nucléique comprend en outre une séquence fonctionnelle (par exemple, une séquence d'amorce de séquençage) et/ou une séquence supplémentaire (par exemple, une séquence d'identifiant moléculaire unique).

11. Procédé selon l'une quelconque des revendications 1 à 10, comprenant en outre, avant (d), l'étape consistant à soumettre ladite molécule d'acide nucléique à code-barres ou un dérivé de celle-ci à une amplification d'acide nucléique, dans lequel ladite amplification d'acide nucléique est réalisée après la libération de ladite molécule d'acide nucléique à code-barres ou d'un dérivé de celle-ci dudit compartiment.

12. Procédé selon l'une quelconque des revendications 1 à 11, dans lequel la séquence d'acide nucléique de l'ARN comprend la séquence :

   (a) d'une chaîne alpha de TCR, d'une chaîne bêta de TCR, d'une chaîne delta de TCR, d'une chaîne gamma de TCR, ou de tout fragment de celles-ci, par exemple des régions variables comportant des régions VDJ ou VJ, des régions constantes, des régions transmembranaires, des fragments de celles-ci, des combinaisons de celles-ci, et des combinaisons de fragments de celles-ci ; et/ou
   (b) d'une chaîne lourde de récepteur de lymphocytes B, d'une chaîne légère de récepteur de lymphocytes B, ou de tout fragment de celles-ci, par exemple des régions variables comportant des régions VDJ ou VJ, des régions constantes, des régions transmembranaires, des fragments de celles-ci, des combinaisons de celles-ci, et des combinaisons de fragments de celles-ci.

13. Procédé selon l'une quelconque des revendications 1 à 12, dans lequel ladite séquence d'amorce comprend une séquence polyT et dans lequel ladite séquence cible d'acide nucléique comprend une séquence de région variable de récepteur de lymphocytes 1 ou une séquence de région variable de récepteur de lymphocytes B.

**14.** Procédé selon l'une quelconque des revendications 1 à 13, dans lequel au moins l'une de ladite paire d'amorces s'hybride à une région constante d'une séquence d'acide nucléique de récepteur de lymphocytes T, à une région constante d'une séquence d'acide nucléique de récepteur de lymphocytes B, ou à une région constante d'une séquence d'acide nucléique d'immunoglobuline.

**15.** Procédé selon l'une quelconque des revendications 1 à 14, comprenant en outre l'ajout d'une autre séquence fonctionnelle audit sous-ensemble enrichi de molécules d'acide nucléique à code-barres ou de dérivés de celles-ci.

**16.** Procédé selon l'une quelconque des revendications 1 à 15, dans lequel la séquence au niveau de l'extrémité 3' qui est complémentaire d'une portion d'un oligonucléotide de commutation de matrice est une séquence polyC

Figure 1

Figure 2

Figure 3

Figure 4

Figure 5

Sort, Quantify and
dilute viable cells
602

↓

Partition cells with barcode
Bearing oligo beads
604

↓

Lyse cells and release
Barcodes
606

↓

mRNA poly-A Capture
608

↓

1st strand synthesis with
Reverse transcriptase
610

↓

Amplify transcripts/barcodes
612

↓

Clean-up
614

↓

Sequence/Quantify
616

Figure 6

Figure 7

Individual Jurkat cell

Barcode oligo bead

Aqueous:oil partition border

Individual Jurkat cells

Figure 8

Figure 9A

Figure 9B

Figure 9C

Figure 9D

Figure 9E

Figure 10

Figure 11

Figure 12A

1206

1204

| 1208 | 1212 | 1210 | 1216 |

1202

Figure 12B

1226

1224

| 1228 | 1232 | 1230 | 1236 | 1234 |

1222

Figure 13A

Figure 13B

Figure 13C

## Figure 14A

## Figure 14B

Figure 15

Figure 16

Figure 17

## Figure 18

## Figure 19A

Figure 19B

Figure 20

## Figure 21A

## Figure 21B

## Figure 21C

Figure 22

# Figure 23

| Sample | Number of Cells | Mean Reads per Cell | Valid Barcodes | Reads Mapped Confidently to Transcriptome | Number of Reads | Reads Mapped Confidently to Exonic Regions | Reads Mapped Confidently to Intronic Regions | Reads Mapped Confidently to Intergenic Regions | Q30 Bases in Barcode | Q30 Bases in Read 1 | Q30 Bases in Sample Index | Fraction reads unmapped |
|---|---|---|---|---|---|---|---|---|---|---|---|---|
| unenriched | 0 | 0 | 96.00% | 59.30% | 95,373 | 69.90% | 12.50% | 3.50% | 92.20% | 92.80% | 89.60% | 8.90% |
| TRAC | 0 | 0 | 61.60% | 16.30% | 671,224 | 30.50% | 3.60% | 4.90% | 61.40% | 93.40% | 89.20% | 49.30% |
| TRBC | 0 | 0 | 64.90% | 21.80% | 780,871 | 33.60% | 4.30% | 4.10% | 64.30% | 93.20% | 85.60% | 45.70% |
| TRAC/TRBC | 0 | 0 | 73.50% | 24.10% | 1,522,739 | 43.50% | 3.60% | 4.30% | 71.50% | 93.20% | 89.60% | 39.50% |

| Sample | Reads with polyA sequence | Reads with switch oligo sequence | Fraction reads with R1 sequence | Fraction reads with R2 sequence | Fraction reads with P7 sequence | Fraction reads with P5 sequence | Fraction reads with primer or homopolymer sequence | Number of detected cells CV | Relative difference of detected cells from expected cells | Barcodes detected | Fraction fragments mapped to VDJ |
|---|---|---|---|---|---|---|---|---|---|---|---|
| unenriched | 1.60% | 0.00% | 0.00% | 2.00% | 0.30% | 0.00% | 4.40% | 0.00% | -100.00% | 14,921 | 0.30% |
| TRAC | 0.20% | 0.00% | 0.00% | 4.70% | 3.10% | 0.00% | 6.80% | 0.00% | -100.00% | 33,520 | 15.50% |
| TRBC | 0.20% | 0.00% | 0.00% | 6.40% | 3.90% | 0.00% | 8.80% | 0.00% | -100.00% | 34,978 | 19.70% |
| TRAC/TRBC | 0.20% | 0.00% | 0.00% | 8.00% | 4.40% | 0.00% | 10.30% | 0.00% | -100.00% | 39,921 | 29.50% |

# Figure 24

Figure 25A

## Figure 25B

Figure 26A

## Figure 26B

## Figure 27A

## Figure 27B

## Figure 27C

## Figure 28A

## Figure 28B

## Figure 28C

## Figure 28D

Figure 29A

Figure 29B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015103339 A
- WO 2014144495 A
- US 2016244825 A
- US 2015376609 A
- US 20140378350 A

- US 20140155295
- US 20100105112
- US 20140378345
- US 6265552 B

**Non-patent literature cited in the description**

- **COUFAL, N. G. et al.** *Nature,* 2009, vol. 460, 1127-1131
- **MUOTRI, A. R. et al.** *Nature,* 2010, vol. 468, 443-446
- **MORGAN et al.** *PLoS Comput. Biol.,* December 2012, vol. 8 (12), e1002808

- **RAM et al.** *Syst. Biol. Reprod. Med.,* June 2011, vol. 57 (3), 162-170
- **FANG et al.** Fluoride-Cleavable Biotinylation Phosphoramidite for 5'-end-Labeling and Affinity Purification of Synthetic Oligonucleotides. *Nucleic Acids Res.,* 15 January 2003, vol. 31 (2), 708-715